# EUROPEAN PATENT APPLICATION

(11) **EP 3 293 230 A1**
(43) Date of publication of application: **14.03.2018**
(21) Application number: 16001981.6
(22) Date of filing: 12.09.2016
(51) Int. Cl.: C09B 11/24, C09B 11/28, C09B 69/00, C07F 7/30, C09K 11/06, G01N 33/58

(54) **CELL-PENETRATING FLUORESCENT DYES WITH SECONDARY ALCOHOL FUNCTIONALITIES**

(71) Applicant: Max-Planck-Gesellschaft zur Förderung der Wissenschaften e.V., 80539 München (DE)
(72) Inventor: BUTKEVICH, Alexey, 37077 Göttingen (DE); BELOV, Vladimir N., 37083 Göttingen (DE); HELL, Stefan W., 37085 Göttingen (DE); KAMIN, Dirk, 37083 Göttingen (DE); SIDENSTEIN, Sven, 37079 Göttingen (DE); SHOJAEI, Heydar, 37073 Göttingen (DE); KOLMAKOV, Kirill, 39106 Magdeburg (DE); SOKOLOV, Viktor V., 194017 St. Petersburg (RU)
(74) Representative: v. Bezold & Partner Patentanwälte - PartG mbB

(57) **Abstract**

The invention relates to novel cell-penetrating fluorescent dyes with with secondary alcohol functionalities having one of the following general formulae I-III and 4:

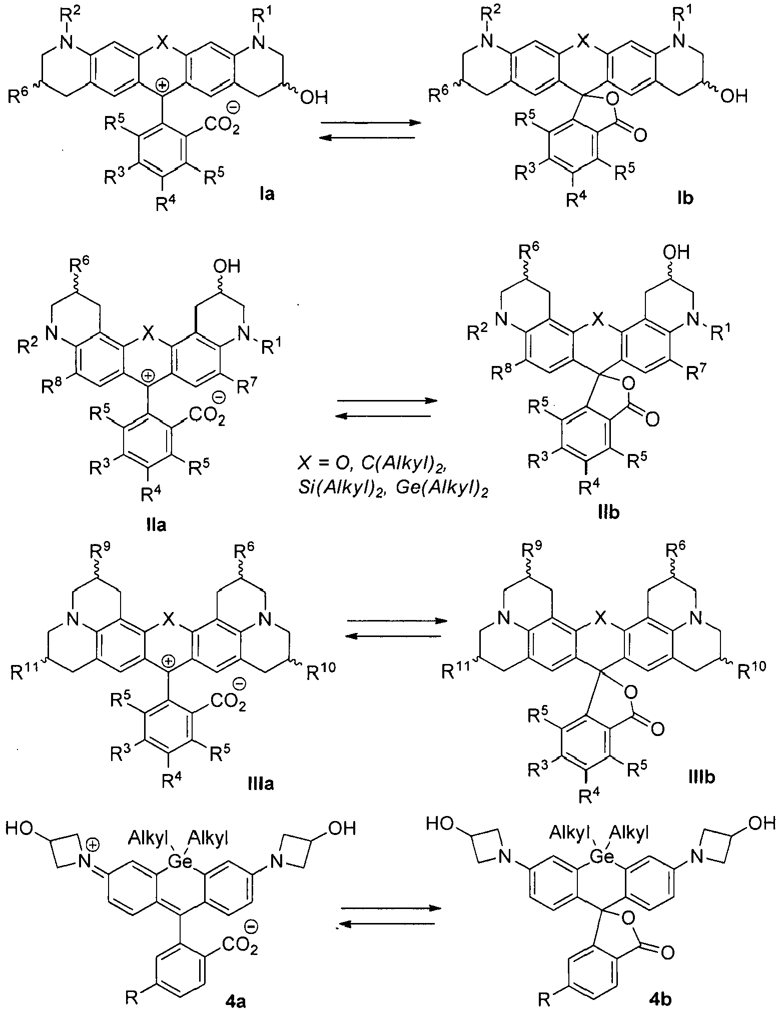

The invention also relates to the use of these compounds for optical microscopy and imaging techniques.

## Description

### Background of the invention

Fluorescent dyes are widely used as indispensable markers in biology, optical microscopy, and analytical chemistry.

In particular, the sensitive and stable imaging of cellular components depends on the favorable combination of chemical, biological and physical factors. The availability and proper choice of fluorescent dyes is a key factor to success of the entire labeling and imaging procedure. Due to their superior brightness and photostability, synthetic dyes represent an attractive alternative to fluorescent proteins.

The variety of fluorescent probes applicable for intracellular labeling of living cells is restricted due to cell permeability requirements (see, e.g., Butkevich et al., Angew. Chem. Int. Ed. 2016, 55, 3290-3294). Some carbopyronines (see, e.g., Kolmakov et al., Eur. J. Org. Chem. 2010, 3593-3610), silicon-rhodamines (*SiR*) (see, e.g., Kushida et al., Analyst 2015, 140, 685-695) and photoactivatable rhodamine dyes (see, e.g., Belov et al., Angew. Chem. 2010, 122, 3598-3602) are known to penetrate outer plasma membranes of intact cells, and methods for improving their brightness have recently been proposed (Grimm et al., Nat. Meth. 2015, 12, 244-250).

Unfortunately, the spectral variety of photostable fluorescent dyes suitable for intracellular targeting and super-resolution imaging in living cells is quite limited, and only few of them are commercially available (Scheme 1).

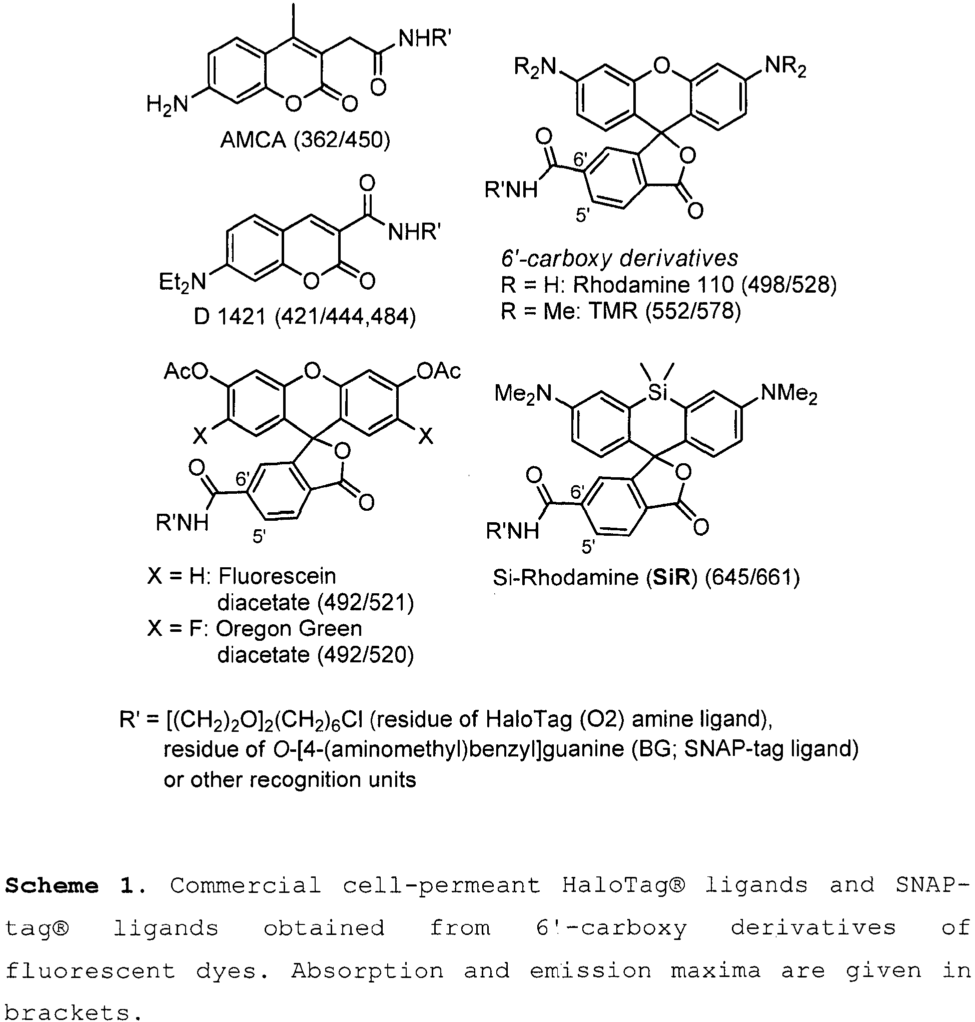

However, the solubility of these dyes in aqueous media is less than desirable. Moreover, in many cases lipophilic dyes bind with the target structures not fully specifically and, as a result, produce fluorescent background which complicates detection, reduces signal-to-noise ratio (contrast) and even makes the imaging impossible. On the other hand, modified fluorescent dyes with additional ionic groups and increased polarity often suffer from other drawbacks, such as lower or even negligible cell membrane permeation (for anionic dyes), toxicity or unspecific binding (for cationic dyes). Additionally, the presence of the polar ionic groups restricts the synthetic flexibility, requires special protecting groups and poses severe limitations on the post-synthetic modifications of these dyes.

In view of the drawbacks of the fluorescent dyes of the prior art, the main object of the present invention was to provide novel fluorescent dyes with superior properties. In particular, the solubility in aqueous media had to be improved, while maintaining cell membrane permeation capability and specificity of binding with target structures. Additionally, higher brightness (product of the fluorescence quantum yield and extinction coefficient) had to be maintained (or improved), as compared to cell-permeant probes and cellular component markers of the prior art.

This objective has been achieved by providing the novel cell-penetrating fluorescent dyes with secondary alcohol functionalities according to claims 1-8, a method for preparing germa- and silaxanthones according to claim 9, conjugates comprising said dyes according to claim 13, as well as the applications of the disclosed novel fluorescent dyes according to claims 11 and 15 to 18. Further aspects and more specific embodiments of the invention are the subject of further claims.

### Description of the invention

The structures of the novel fluorescent dyes of the invention are selected from the group consisting of compounds of the following general formulae **I-III and** 4:

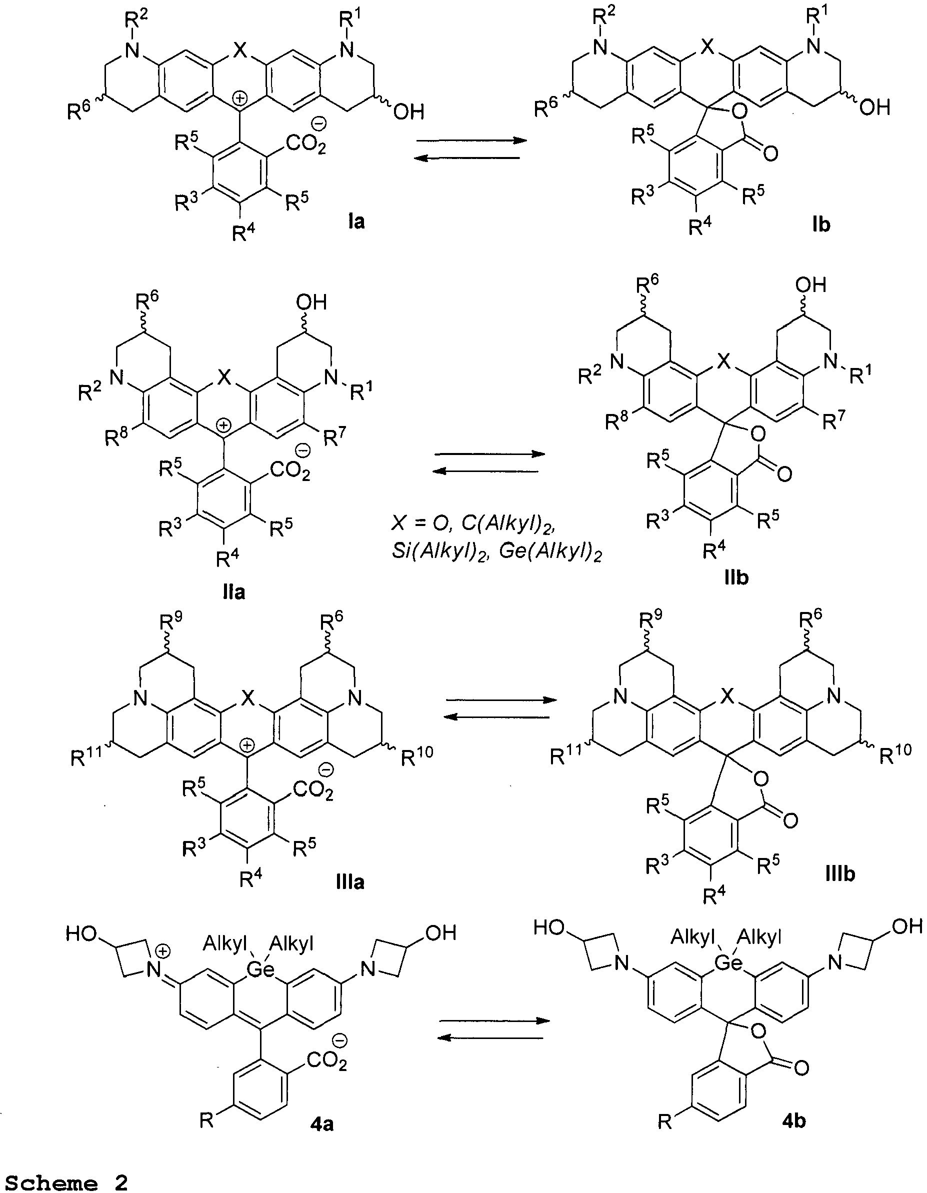

These compounds exist in equilibrium between the open zwitterionic form (**a**), which is typically colored and fluorescent, and the closed uncharged form **(b),** which is typically colorless and non-fluorescent.

In the above general structural formulae **I-III**
*Alkyl* is a C₁-C₄ alkyl group, such as a methyl, ethyl, propyl, isopropyl, n-butyl, isobutyl or sec-butyl group;
R¹ and R² in structures **I** and **II** are independently CF₃CH₂, a C₁-C₆ alkyl group, or a substituted C₁-C₆ alkyl group;
R³ and R⁴ in structures **I-III** are independently
hydrogen; a C₁-C₆ alkyl group or (functionally) substituted C₁-C₆ alkyl group; a carboxyl group or a derivative of the carboxyl group, e.g. a C₁-C₆ alkyl, aryl or hetaryl ester, in which the alkyl, aryl or hetaryl part is optionally substituted with any functional group; a primary or secondary alkyl, aryl or hetaryl carboxamide (CONHR', CONR'R"), in which the alkyl, aryl or hetaryl part(s) (R', R") is (are) optionally substituted with any functional group(s); a *N-*hydroxysuccinimidyl ester or another reactive ester of the type CO₂X, where X is a suitable good leaving group (nucleofuge) including but not limited to F, N₃, SR', OR' (R' = aryl or hetaryl); hydrazide CONHNH₂ or substituted hydrazide CONR'NR" R"', with R', R", R'" being independently H, lower alkyl (C₁-C₆), aryl or hetaryl, in which the alkyl, aryl or hetaryl part is optionally substituted with any functional group; hydroxamic acid CONHOH or substituted hydroxamic acid CONR'OR ", with R', R" being independently H, lower alkyl, aryl or hetaryl, in which the alkyl, aryl or hetaryl part is optionally substituted with any functional group; diazoketone COCHN₂ or substituted diazoketone COCR'N₂ with R' = lower alkyl, aryl or hetaryl, in which the alkyl, aryl or hetaryl part is optionally substituted with any functional group; an amino group, alkylamino or dialkylamino (with alkyl = C₁-C₁₀) group, the alkyl part(s) of which may be optionally substituted with any functional group, in particular a carboxyl group or a derivative of the carboxyl group, e.g. a lower alkyl (e.g. C₁-C₆), aryl or hetaryl ester, in which the alkyl, aryl or hetaryl part is optionally substituted with any functional group; azido group; *N*-maleimidoalkyl group, iodoacetamide or any other reactive group suitable for conjugation to biomolecules; an arylthio or alkylthio group, the aryl or alkyl part of which may be optionally substituted with any uncharged group, including an azide group -N₃, carboxyl group, a derivative of the carboxyl group, e.g. a lower alkyl, aryl or hetaryl ester, in which the alkyl, aryl or hetaryl part is optionally substituted with any functional group; an alkyl (C₁-C₁₀), aryl or hetaryl amide (NHCOR', NHCOR'R"), in which the alkyl, aryl or hetaryl part(s) (R', R") is(are) optionally substituted with any functional group; optionally, the carbon chains in R¹, R², R³ and R⁴ may contain double or triple bonds and/or cycles, and/or uncharged groups, e.g. hydroxyl, as well as one, two, three or four heteroatoms; R⁵ = H, F or Cl; R⁶, R⁹, R¹⁰, R¹¹ = H or OH (see provisions 1 and 2 below) and the wavy bonds (∼∼∼) shown in the structures **I-III** denote all possible isomers if one or more substituents from the set R⁶, R⁹, R¹⁰, R¹¹ is/are OH;
R⁷, R⁸ are independently H, a C₁-C₄ alkyl group, F, Cl, Br or I; provided (1) that all dyes represented by the formulae **I, II** and **III** contain at least one substituted 3-hydroxy-1,2,3,4-tetrahydroquinoline fragment (with an element -CH₂CH(OH)CH2-) incorporated into the structure of the fluorophore, as shown in Scheme 2 and in the structural formula below; 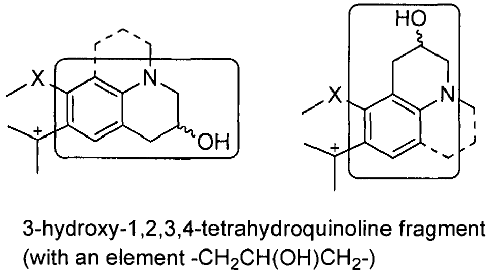 with an additional provision (2) for structure **III** that at least one of R⁶, R⁹, R¹⁰ or R¹¹ is a hydroxyl group.

In the structural formula **4** above
*Alkyl* is a C₁-C₆ alkyl group, such as methyl, ethyl, propyl, isopropyl, *n*-butyl, isobutyl, sec-butyl, pentyl, isopentyl, 2-methylbutyl, 2,2-dimethylpropyl, hexyl, isohexyl, 2-methylpentyl, 3-methylpentyl, 2,2-dimethylbutyl;
R in structure **4** may be hydrogen, a C₁-C₆ alkyl group or (functionally) substituted C₁-C₆ alkyl group, a carboxyl group or a derivative of the carboxyl group, e.g. a lower alkyl (C₁-C₆), aryl or hetaryl ester, in which the alkyl, aryl or hetaryl part is optionally substituted with any functional group; a primary or secondary alkyl, aryl or hetaryl carboxamide (CONHR', CONR'R"), in which the alkyl, aryl or hetaryl part(s) (R', R") is (are) optionally substituted with any functional group(s); a *N*-hydroxysuccinimidyl ester or another reactive ester of the type CO₂X, where X is a suitable good leaving group (nucleofuge) including but not limited to F, N₃, SR', OR' (R' = aryl or hetaryl); CONHNH₂ or CONR'NR"R"' with R', R", R"' being independently H, lower alkyl (C₁-C₆), aryl or hetaryl, in which the alkyl, aryl or hetaryl part(s) is (are) optionally substituted with any functional group(s); CONHOH or CONR'OR" with R', R" being independently H, lower alkyl, aryl or hetaryl, in which the alkyl, aryl or hetaryl part(s) is (are) optionally substituted with any functional group(s)); COCHN₂ or COCR'N₂ with R' = lower alkyl, aryl or hetaryl, in which the alkyl, aryl or hetaryl part is optionally substituted with any functional group; an amino group, alkylamino (with alkyl = C₁-C₁₀) group or dialkylamino group, the alkyl part(s) of which may be optionally substituted with any functional group, in particular with a carboxyl group or a derivative of the carboxyl group, e.g., lower alkyl (preferably C₁-C₆), aryl or hetaryl ester, in which the alkyl, aryl or hetaryl part is optionally substituted with any functional group; a primary or secondary alkyl, aryl or hetaryl amide (NHCOR', NHCOR'R"), in which the alkyl, aryl or hetaryl part(s) (R', R") is (are) optionally substituted with any functional group(s); azido group; *N*-maleimidoalkyl group; iodoacetamide or any other reactive group suitable for conjugation to biomolecules; optionally, the carbon chains in the group R may contain double or triple bonds and/or cycles, and/or uncharged groups as well as one, two, three or four heteroatoms.

Another preferred embodiment of the present invention is a compound of structure **III** above which exhibits two hydroxyl groups and wherein either R⁶ = R⁹ = OH and R¹⁰ = R¹¹ = H, or R⁶ = R⁹ = H and R¹⁰ = R¹¹ = OH.

A further preferred compound of structure **III** exhibits one hydroxyl group and either R⁶ = OH and R⁹ = R¹⁰ = R¹¹ = H, or R¹⁰ = OH and R⁶ = R⁹ = R¹¹ = H.

Another specific embodiment of the invention is a fluorescent dye representing one of the compounds **5**-R³, wherein R¹ = R² = CH₂CF₃, R⁶ = OH and R³ = CO₂H, its reactive ester (preferably *N-*succinimidyloxycarbonyl, 2,3,5,6-tetrafluorophenoxycarbonyl or pentafluorophenoxycarbonyl group), or amide with a linker bearing one terminal amino or carboxylic acid group as indicated in the structural formulae below

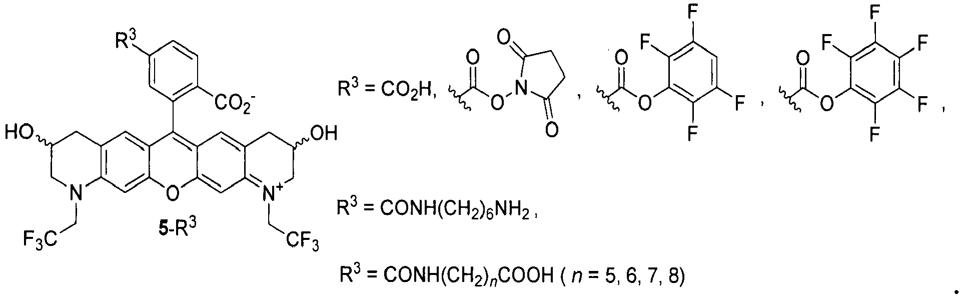

A further specific embodiment of the invention is a fluorescent dye representing one of the compounds **14a,b-**R³, wherein either R¹ = R² = OH, or R¹ = OH and R² = H, and wherein R³ = CO₂H its reactive ester, preferably *N*-succinimidyloxycarbonyl, 2,3,5,6-tetrafluorophenoxycarbonyl or pentafluorophenoxycarbonyl, or amide with a linker bearing one terminal amino or carboxylic acid group.

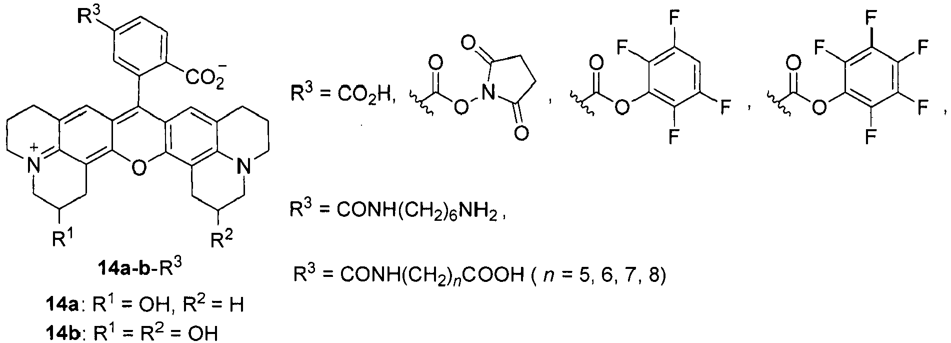

Another preferred embodiment represents a fluorescent dye of structure **4** where *Alkyl* is a straight or branched C₁-C₆ alkyl chain, preferably C₁-C₄ alkyl, most preferably methyl; R is CO₂H or a reactive ester, preferably N-succinimidyloxycarbonyl, 2,3,5,6-tetrafluorophenoxycarbonyl or pentafluorophenoxycarbonyl, or a functional group capable of participating in an *in vitro* or *in vivo* chemical ligation reaction such as, for example, an azide, alkyne, strained alkene (such as (*E*)-cyclooctene, cyclopropene or norbornene) or tetrazine reactive group. Typically, the functional group is connected with the (trisubstituted) phenyl group through a linker, preferably an ester, an amide, a polymethylene (C₂-C₁₀) or poly(ethylene glycol) (PEG, [OCH₂CH₂]ₙ, *n =* 2-6) linker. Alternatively, R is any ligand forming a covalent bond with a biological target of interest, or a noncovalent ligand binding to a biological target of interest, such as a docetaxel or jasplakinolide derivative. Typically, the ligand is connected with the (trisubstituted) phenyl group through a linker, preferably an ester, an amide, a polymethylene (C₂-C₁₀) or poly(ethylene glycol) (PEG, [OCH₂CH₂]*ₙ*, *n* = 2-6) linker.

More specifically, this fluorescent dye is represented by a compound **4**-R, wherein R = CO₂H or its reactive ester (preferably *N*-succinimidyloxycarbonyl, 2,3,5,6-tetrafluorophenoxycarbonyl or pentafluorophenoxycarbonyl group), as indicated in the structural formulae below

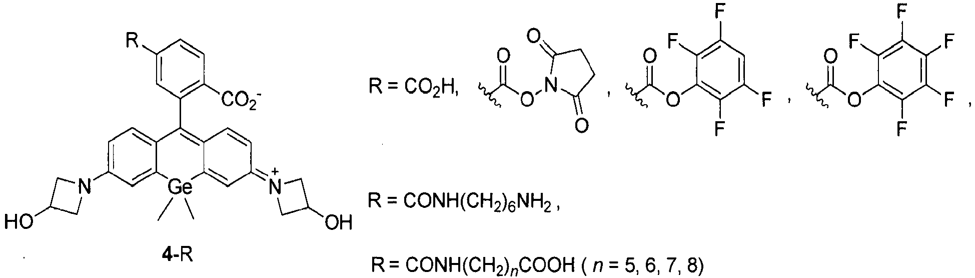

In another specific embodiment, this fluorescent dye is represented by one of the compounds **33, 33**-NHS, **33**-Halo, **33-**tubulin and **34** as indicated in the structural formulae below

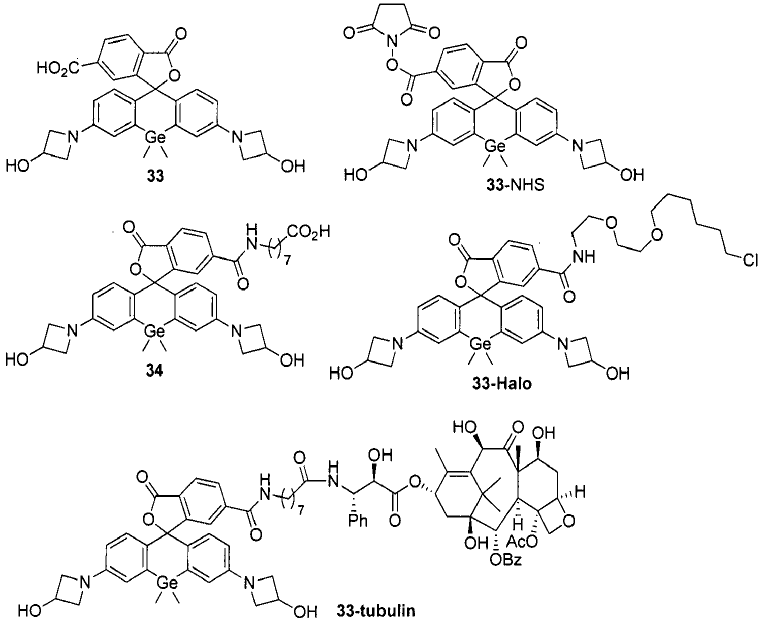

The term "substituted" as used herein, generally refers to the presence of one or more substituents, in particular substituents selected from the group comprising straight or branched alkyl, in particular C₁-C₄ alkyl, e.g. methyl, ethyl, propyl, butyl; isoalkyl, e.g. isopropyl, isobutyl (2-methylpropyl); secondary alkyl group, e.g. sec-butyl (but-2-yl); tert-alkyl group, e.g. tert-butyl (2-methylpropyl).

Additionally, the term "substituted" encompasses the more specific term "functionally substituted" as defined below. In particular, the substituents may be alkyl groups having fluoro-, chloro- or bromo substituents instead of hydrogen atoms, or methoxy, ethoxy, 2-(alkyloxy)ethyloxy groups (AlkOCH₂CH₂O), and, in a more general case, oligo(ethylene glycol) residues of the type Alk(OCH₂CH₂)ₙOCH₂CH₂-, where Alk = CH₃, C₂H₅, C₃H₇, C₄H₁₀, and *n* = 1-23. Other suitable or preferred substituents in specific structures have been indicated above but should not be contemplated as the only possible embodiments.

The term "functionally substituted" as used herein, generally refers to the presence of one or more functional groups, in particular including cycloalkenyl, alkenyl, e.g. ethenyl (vinyl), allyl, propen-1-yl, propen-2-yl, 1,3-butadien-1-yl, 1,3-budadien-2-yl, 1,2-propadien-1-yl (allenyl), alkynyl, e.g. ethynyl, propynyl, propargyl, aryl, e.g. phenyl, 1-naphthyl, 2-naphthyl, 9-anthracenyl-, pyren-1-yl, pyren-2-yl; one or more halogen atoms (F, Cl, Br, I), azido groups (-N₃), nitroso groups (N=O), diazo groups [=N(+)=N(-)], diazocarbonyl groups [>C=N(+)=N(-)], hydroxy groups (OH), protected hydroxy groups (OR, R = acyl, e. g. formyl, acetyl, tetrahydropyranyl, t-butyl, allyl, propargyl, phenyl, p-chlorophenyl, p-methoxyphenyl, p-nitrophenyl, perfluorophenyl, perchlorophenyl, benzyl, 2-picolyl, 4-picolyl, diphenylmethyl, triphenylmethyl, trialkylsilyl, triphenylsilyl, diphenylalkylsilyl), thiol groups (SH), protected thiol groups (SR, R was defined above), primary and secondary amino groups (NH₂ and NHR, R was defined above), hydrazo groups (-NHNH₂), hydroxylamino groups (-NHOH and H₂NO-), carbonyl groups in aldehydes and ketones (-CHO and >C=O), protected carbonyl groups (>C(OCH₃)₂, >C (OCH₂CH₂O), and other acetals and ketals), hydrazones (>C=NNR'R"), oximes (>C=N-OR), thioketones (>C=S), sulfoxides (>S=O), sulfones (-SO₂-), sulfonic acid residues and their salts [-SO₃H and - SO₃(-)], carboxylic acid groups (COOH), and ester groups (COOR, R = alkyl, e. g. CH₃, C₂H₅, t-butyl; allyl, propargyl, phenyl, p-chlorophenyl, p-methoxyphenyl, p-nitrophenyl, perfluorophenyl, perchlorophenyl, benzyl, 2-picolyl, 4-picolyl, diphenylmethyl, triphenylmethyl, trialkylsilyl, triphenylsilyl, diphenylalkylsilyl, in particular, N-hydroxysuccinimidyl); primary, secondary and tertiary amido groups, phosphorodiamidite group [-OP(NR₂)₂, R = CH₃, C₂H₅, isopropyl, allyl, tert-butyl, phenyl], phosphoroamidite group [-OP(NR'₂) (OR"), R' = CH₃, C₂H₅, isopropyl, allyl, tert-butyl, phenyl, R" = H, alkyl, aryl, hetaryl], phosphorodiamidite group [-OP(NR₂), R = CH₃, C₂H₅, isopropyl, allyl, tert-butyl, phenyl], phosphoric acid residues [-OP(O) (OR') (OR"), R' (R") = H, CH₃, C₂H₅, allyl, tert-butyl, phenyl, NH₂], phosphonic acid residues [-P(O)(OR')(OR"), R'(R") = H, CH₃, C₂H₅, allyl, tert-butyl, phenyl, NH₂], *N*-phthalimido group.

The term "functionally substituted" as used herein, also refers to the optional presence of one or more heterocyclic residues, in particular including oxirane, aziridine, thiirane, azetidine, pyrrolidine, tetrahydropyrane, tetrahydrothiophene, 1,3-oxazoline, 1,3-oxazolidine, thiophene, furane, pyrrole, pyridine, quinoline, pyrimidine, pyrimidin-4-one, pyrimidin-2-one, pyridazine, pyrazine, 1,3,5-triazine, 1,2,4-triazine, 1,3-imidazol, thiazole, 1,2,4-triazole, 1,2,3-triazole, 1,3-oxazole. The said heterocycles may be attached to the carbon framework of the substituents via various positions (e.g. 1-aziridinyl, 2-aziridinyl, pyrrolidin-1-yl, pyrrolidine-2-yl, pyrrolidin-3-yl, thiophen-2-yl, thiophen-3-yl, pyridin-2-yl, pyridin-3-yl, pyridin-4-yl, etc.).

The term "functionally substituted", as used herein, also refers to the optional presence of any combination of the said functional groups with each other (e.g., 2-diazoketone groups as represented by: -COCN₂-). The term "functionally substituted", as used herein, also refers to the optional presence of any combination of the said functional groups with any heterocyclic residues named above.

The terms "alkyl" or "cycloalkyl", as used herein, generally comprise any unsubstituted or substituted (cyclo)alkyl groups, typically with 1-30 or more C atoms, in particular also a lower (cyclo)alkyl group. If not defined more specifically, the term "lower alkyl group" refers to C₁-C₁₀ alkyl groups, more preferred C₁-C₆ or C₁-C₄ alkyl groups. Some non-limiting examples thereof are methyl, ethyl, propyl, isopropyl, n-butyl, isobutyl, sec-butyl, pentyl, isopentyl, 2-methylbutyl, 2,2-dimethylpropyl, hexyl, isohexyl, 2-methylpentyl, 3-methylpentyl, 2,2-dimethylbutyl etc.

The term "aryl", as used herein, refers to an unsubstituted or substituted mono-, bi- or tricyclic carbocyclic ring system having one, two or three aromatic rings including but not limited to phenyl, naphthyl, anthryl, azulyl, tetrahydronaphthyl, indanyl and indenyl.

The terms "hetaryl" or "heterocycle", as used herein, generally refer to an unsubstituted or substituted cyclic aromatic radical (residue) having from 5 to 10 ring atoms of which at least one ring atom is selected from S, 0 and N; the radical being joined to the rest of the molecule via any of the ring atoms. Representative, but not limiting examples are furyl, thienyl, pyridinyl, pyrazinyl, pyrimidinyl, pyrrolyl, imidazolyl, thiazolyl, oxazolyl, isooxazolyl, thiadiazolyl, oxadiazolyl, quinolinyl and isoquinolinyl.

The dyes provided by the present invention are cell permeant hydroxylated fluorescent dyes without isolated C=C bonds (see structures **Ia/Ib, IIa/IIb, IIIa/IIIb** and **4a/4b;** hydroxylation in non-allylic and non-benzylic positions).

All these dyes (Scheme 2) exist in equilibrium between the "open" form **(a)** and the."closed" form **(b).** They belong to the groups of rhodamine dyes (X = O), carborhodamines (X = C(Alkyl)₂), silicon-rhodamines (*SiRs;* X = Si(Alkyl)₂), or germanium-rhodamines (*GeRs;* X = Ge(Alkyl)₂).

In general, all dyes represented by the formulae **I, II** and **III** contain *one or two* elements -CH₂CH(OH)CH₂- incorporated into (a) six-membered ring(s) which is(are) fused with the fluorophore and include(s) the nitrogen atom(s), providing mono- or dihydroxylated fluorophore cores (for example, in structures **I** and **II,** R⁶ = H or OH) with at least one substituted 3-hydroxy-1,2,3,4-tetrahydroquinoline fragment incorporated into the structure of the fluorophore. In the case of two -CH₂CH(OH)CH₂- elements, they are symmetrically attached to both aromatic rings of the diarylmethane core of the fluorophore. In structure **III,** at least one of R⁶, R⁹, R¹⁰ or R¹¹ is a hydroxyl group.

In one preferred embodiment, a compound with structure **III** possesses two hydroxyl groups (either R⁶ = R⁹ = OH and R¹⁰ = R¹¹ = H, or R⁶ = R⁹ = H and R¹⁰ = R¹¹ = OH).

In some preferred embodiments, all alkyl groups are lower alkyl groups.

Importantly, the used synthetic approach (see following sections) imposes some limitations on the nature of residues R¹ - R⁵ in structures **I-III,** and the group R in structure **4.** In particular, R¹ and R² cannot be hydrogen atoms.

The claimed compounds of structures **I-III** above have not been described or suggested in the prior art.

US 6,828,159 B1 discloses carbopyronine dyes (e.g., X = C(Alkyl)₂ in structures **I-III** of Scheme 2), including those with cyclic systems incorporating nitrogen atoms and one or more multiple bonds, but this document does not mention hydroxyl groups attached to these cycles. The closely related WO2013/029650 and WO2014/152298 do not disclose fluorescent dyes (silicon and germanium rhodamines) with *hydroxylated* five- or six-membered rings incorporating nitrogen atoms of the fluorophore and fused with the positions adjacent to these nitrogen substituents.

Structure **4** represents a novel combination of a germanium atom and hydroxylated (at C-3) azetidine rings (saturated four-membered nitrogen heterocyles with hydroxyl groups attached to C-3) incorporated into the germanium-rhodamine (*GeR*) structure.

WO2015/153813 A1 claims various fluorescent dyes with nitrogen atoms incorporated into azetidine cycles (hydroxylated azetidines were claimed but not specifically exemplified), but this document does not mention germanium-rhodamines. WO2014/152298 claims GeRs but does not disclose *GeRs* with azetidine cycles or hydroxylated azetidine cycles incorporating nitrogen atoms.

As it will be demonstrated in the examples with images and with the photophysical data, the presence of hydroxyl groups in structures **I-III** and **4** in Scheme 2 is critical as it allows to impart the hydrophilic properties to the dyes, reduce their unspecific binding with hydrophobic structures in biological objects and improve their performance in optical microscopy (by reducing the unspecific binding and the fluorescence background associated with it) while maintaining sufficient photostability in super-resolution optical microscopy (e. g., in stimulated emission depletion - STED - microscopy using very high light intensities).

Furthermore, the presence of the hydroxyl groups at the positions remote from the carboxylic group (R³ or R⁴ in structures **I-III** and R = OH in compound **4** in Scheme 2) does not preclude the formation of the amino reactive esters, and does not diminish their stability (as compared to the case of dyes containing two allyl alcohol fragments in close proximity to the carboxylic group incorporated into R³, which were demonstrated to be prone to formation of a macrocyclic lactone in the course of an attempted activation of this carboxylic group; see e.g. G. Y. Mitronova et al. in Eur. J. Org. Chem. 2015, 337-349). Moreover, the hydroxylation of homobenzylic rather than benzylic positions of the fluorophore ensures improved chemical and metabolic stability of the probes. Taken together, these data illustrate the novelty, inventiveness and potential industrial applications of the dyes in Scheme 2.

### General synthesis of the novel fluorescent dyes of the invention

### Synthesis of dyes according to formulae I and II

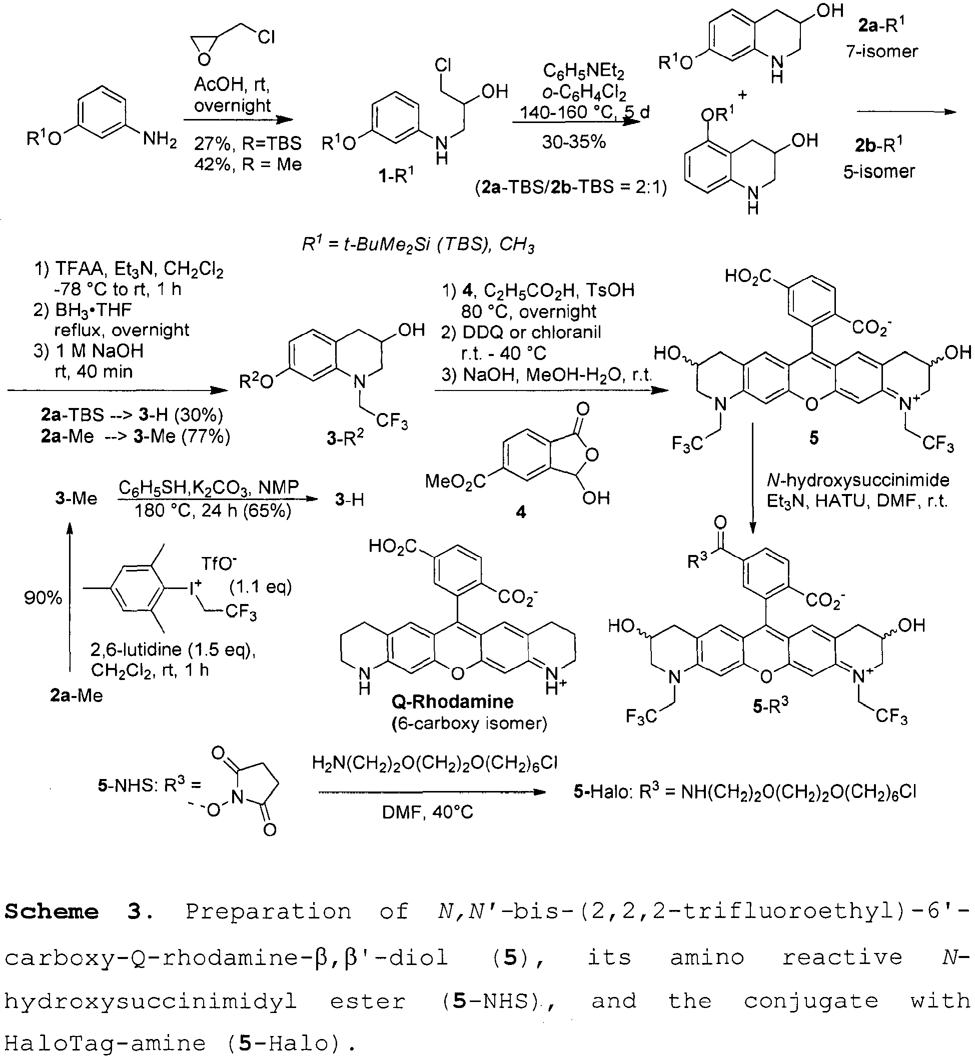

The synthesis of *N,N*'-bis-(2,2,2-trifluoroethyl)-6'-carboxy-Q-rhodamine-β,β'-diol **(5),** a sample dye of type **I,** is shown in Scheme 3. The dye was developed based on a Q-Rhodamine fluorophore core (V. P. Boyarskiy, V. N. Belov, R. Medda, B. Hein, M. Bossi, S. W. Hell, Chem. Eur. J. 2008, 14, 1784-1792). The parent compound 5(6)-carboxy-Q-rhodamine has an absorption maximum at 540 nm (in aqueous PBS buffer), ε = 70 000 M⁻¹ cm⁻¹, an emission maximum at 561 nm and fluorescence quantum yield 0.79. The spectral properties of 5(6)-carboxy-Q-rhodamine are similar to those of a common dye Rhodamine 6G (in ethanol, absorption and emission maxima at 530 nm and 556 nm, respectively (ε = 115 000 M⁻¹ cm⁻¹), fluorescence quantum yield 0.95), which is rather lipophilic and has not found wide use in life sciences.

The secondary amino groups of Q-rhodamine are prone to acylation reactions; as a result, 5(6)-carboxy-Q-rhodamine (Scheme 3) does not form a stable N-hydroxysuccinimidyl ester. Another drawback of 5(6)-carboxy-Q-rhodamine is its low solubility in water and organic solvents (due to its highly symmetrical polycyclic structure).

The present inventors designed and prepared *N,N*'-bis-(2,2,2-trifluoroethyl)-6'-carboxy-Q-rhodamine-β,β'-diol (compound **5** in Scheme 3) which possesses a fluorophore of Q-Rhodamine, but does not have its drawbacks. Indeed, the two free NH groups in the compound **5** are protected from *N*-acylation with 2,2,2-trifluoroethyl substituents. Therefore, dye **5** forms a stable NHS ester. These groups are neither strong electron-acceptors, nor electron-donors, and have an inductive effect similar to hydrogen atoms: they do not shift the positions of the absorption and emission bands compared to the parent Q-Rhodamine. As 2,2,2-trifluoroethyl groups substantially increase the lipophilic character of a dye, the presence of two hydroxyl groups in structure **5** is necessary to offset this effect. The resulting dye **5** is water-soluble and highly fluorescent in aqueous buffers. The conjugate with Halo-Tag-amine (**5**-Halo) was prepared from the amino-reactive dye **5**-NHS (Scheme 3).

Dye **5** (Scheme 4) exists as a mixture of 3 diastereomers due to the possible positioning of its two hydroxyl groups in *syn-* and anti-positions to each other during the condensation step. The *syn*-isomer of compound **5** has a symmetry plane and *anti***-5** has a *C*₂ rotational axis. Because of the hindered rotation around the single C-C bond between the pendant phenyl ring and the fluorophore core, *syn-***5** exists in the form of two diastereoisomers (with the carboxyl group in the ortho-position of the phenyl ring on the same side of the molecular plane as the hydroxyl groups in the xanthene core, or on the opposite side). All 3 diastereomers of **5** were detected by HPLC analysis, and 2 diastereomers were isolated and demonstrated identical optical properties, making the separation of diastereomeric labels unnecessary for any imaging application.

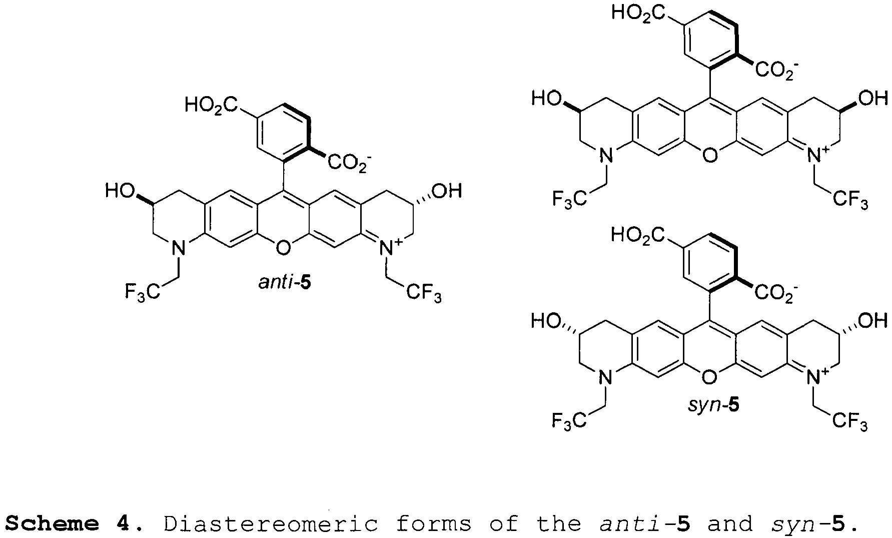

Following the same approach, the dyes of type **II** can be prepared with minor variation of the starting materials.

### Synthesis of dyes according to formula III

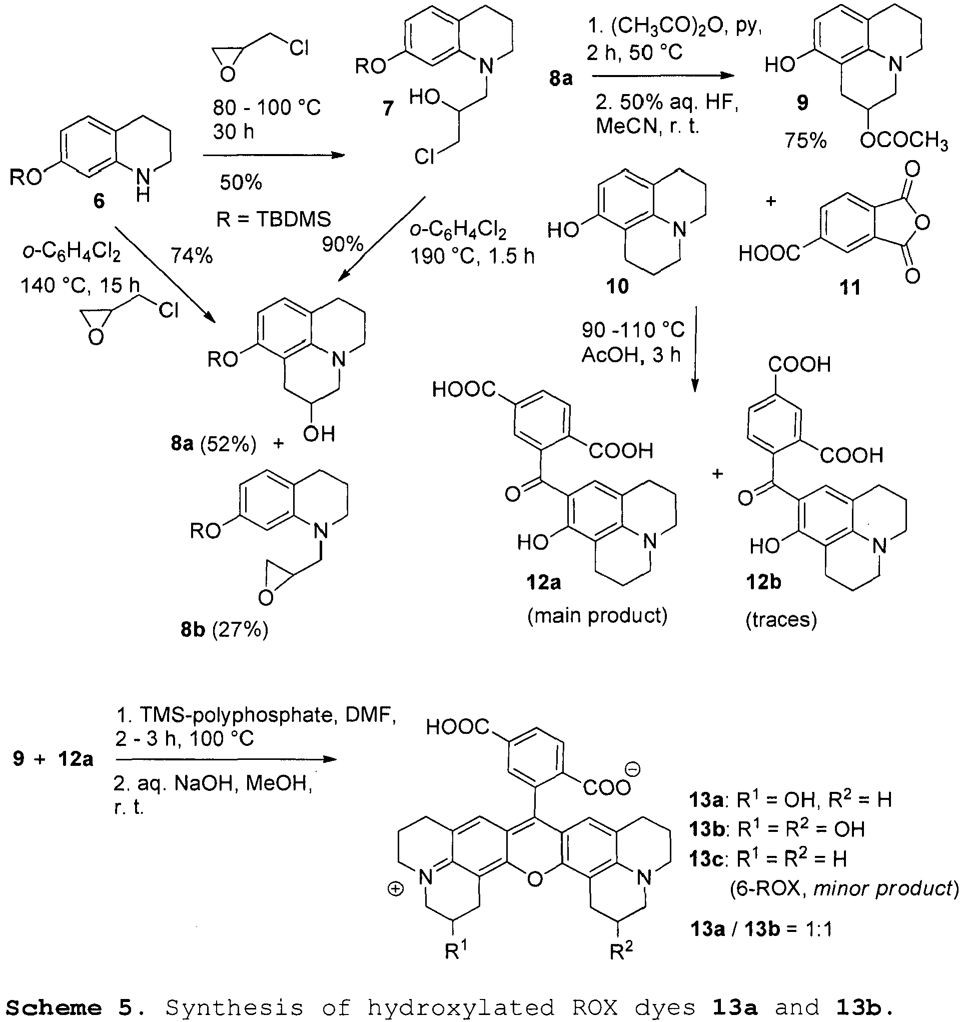

The synthesis of hydroxylated ROX dyes (6'-carboxy isomers) is shown in Scheme 5. These dyes are derivatives of Rhodamine 101 (or X-Rhodamine), which has an outstanding fluorescence quantum yield of more than 0.90 in both organic and aqueous solutions. The main structural feature of Rhodamine 101 and its derivatives is the presence of julolidine fragments - tricyclic systems incorporating nitrogen atoms and rigidizing the fluorophore, which results in the red shift of both absorption and emission maxima and lowers the contribution of nonradiative decay pathways, both desirable features. 5'-Carboxy and 6'-carboxy derivatives of Rhodamine 101 are commercially available as 5- and 6-ROX dyes; the structure of 6-ROX is given in Scheme 5 (compound **13c).** ROX dyes have absorption and emission maxima at 572 nm and 602 nm, respectively, and the extinction coefficients of about 80 000 M⁻¹ cm⁻¹. Interestingly, the reaction between acetate **9** and benzophenone **12a** results in three products which, after removal of the acetate protecting groups, afforded three dyes: **13a, 13b** and **13c** (only **13a** was initially expected). This result may be explained by acid-catalyzed retro-Friedel-Crafts acylation of **12a** leading to compound **10** and followed by Friedel-Crafts acylation with formation of a new benzophenone with 2-hydroxylated or 2-acetoxylated julolidine fragment.

Because of the hindered rotation around the single C-C bond between the pendant phenyl ring and the fluorophore core, dye **13a** exists in the form of two diastereoisomers (with the carboxyl group in the ortho-position of the phenyl ring on the same side of the molecular plane as the hydroxyl group in the julolidine fragment, or on the opposite side; Scheme 5). Dye **13b** exists as a mixture of 3 diastereomers due to the possible positioning of its two hydroxyl groups in *syn-* and *anti-*positions to each other during the condensation step. As with the compound **5,** the *syn*-isomer of compound **13b** has a symmetry plane and *anti***-13b** has a *C*₂ rotational axis. Because of the hindered rotation around the single C-C bond between the pendant phenyl ring and the fluorophore core, *syn-***13b** exists in the form of two diastereoisomers (with the carboxyl group in the *ortho-*position of the phenyl ring on the same side of the molecular plane as the hydroxyl groups in the xanthene core, or on the opposite side). Indeed, all three isomers of **13b** were detected by HPLC analysis (see experimental section). The remarkable feature of the synthesis in Scheme 5 is that it creates a separable mixture of 5 ROX analogs in one step. This may be advantageous, as the specificity and labeling efficiency in a living specimen is known to depend on subtle structural changes of the fluorescent markers; however, the identical optical properties of these dyes allow using the hydroxylated ROX labels **13a,b** without separation of individual diastereoisomers.

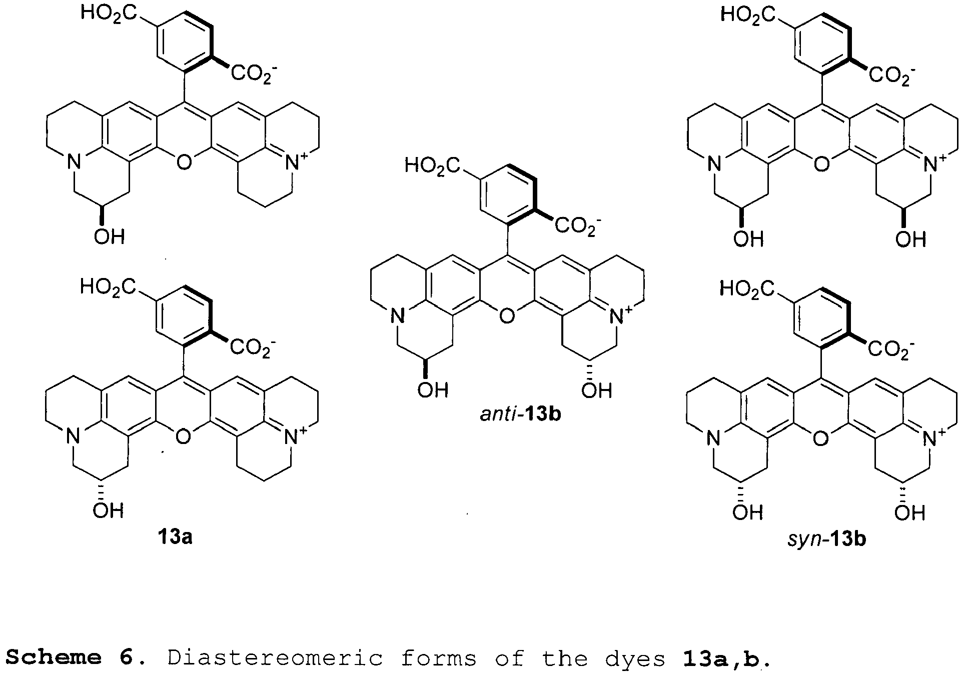

### Synthesis of dyes according to formula 4

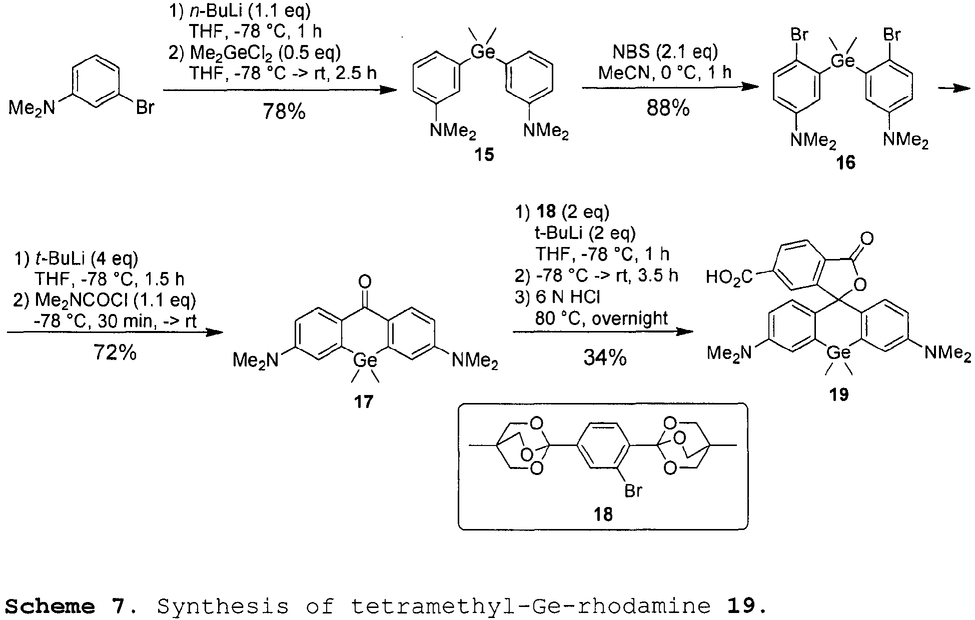

The parent tetramethyl-*GeR* with a free 6'-carboxy group **(19)** for conjugation to biomolecules or small molecule ligands has not been reported in the literature. The synthesis of the dye **19** is shown in the Scheme 7. It is prepared by nucleophilic addition of an aryllithium intermediate, generated at -78 °C from bis-OBO-ester of 2-bromoterephthalic acid **18** (as described by A. N. Butkevich et al. in S. W. Hell, Angew. Chem. Int. Ed. 2016, 55, 3290-3294), to a corresponding 10-germaanthrone (2,7-bis(dimethylamino)dibenzo[*b,e*]germin-10(5H)-one) **17.** A convenient approach to the latter was established by regioselective bis-*para*-bromination of an appropriately substituted diaryldimethylgermane **15** to give dibromide **16,** followed by double lithium-halogen exchange with tert-butyllithium and reaction of the dilithium intermediate with *N,N*-dimethylcarbamoyl chloride (49% yield over 3 steps). Tetramethyl-*GeR* **19** demonstrated a behavior very similar to *SiR* (absorption/emission maxima in PBS 7.4 for **19:** 634/655 nm, quantum yield 0.43, versus absorption/emission maxima for *SiR:* 645/661 nm, quantum yield 0.41). A small blue shift (-11 nm for absorption, -6 nm for emission maximum) can be explained by smaller σ*-π* overlap mainly due to increasing C_{Ar}-Ge bond length as compared to C_{Ar}-Si, which accounts for higher LUMO energy. The dye **19** is expected to show fluorogenic behavior similar to that of *SiR* (D_{0.5} in 1,4-dioxane/water 65.2 for **19,** 64.5 for *SiR)* and indeed demonstrated high contrast images when applied to living cells as Halo-tag **(19-Halo)** or tubulin **(19-tubulin)** ligand (see Example III).

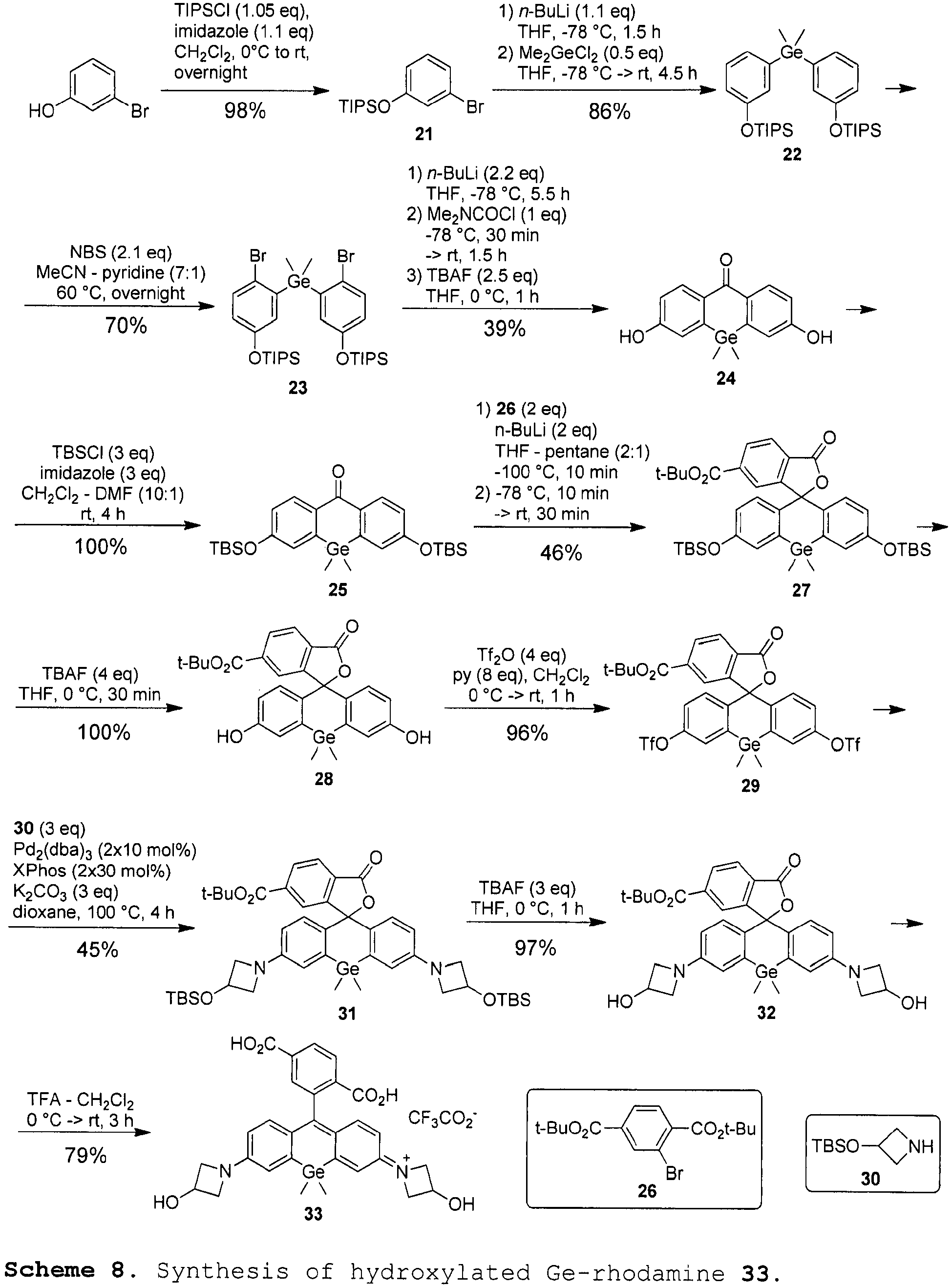

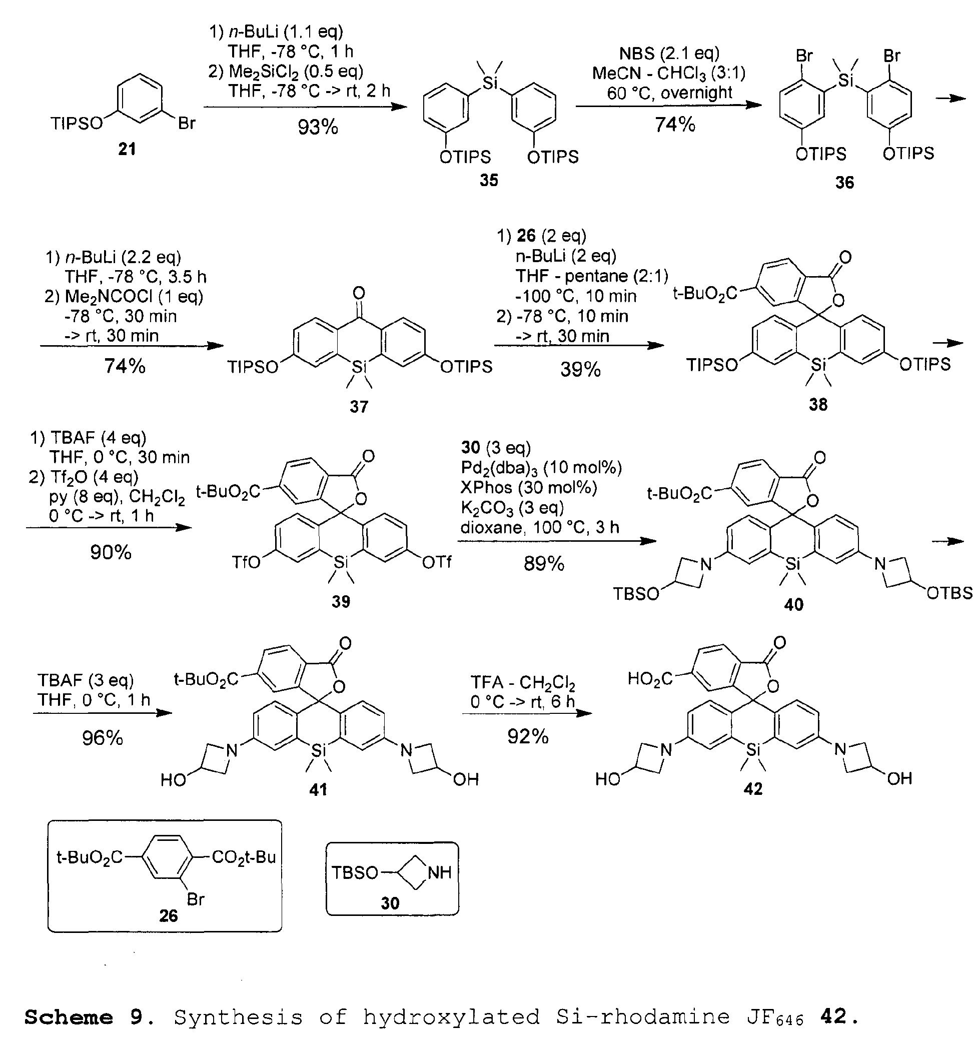

The hydrophilized analogs of bis-(*N*-azetidinyl)-*SiR* (JF₆₄₆) and unknown bis-(*N*-azetidinyl)-*GeR* were prepared with the intention to evaluate the performance of hydroxylated fluorophores (Schemes 8 and 9). Introduction of a hydroxyl group was done in 3-position of the azetidine substituent (the only possible location on the auxofluoric group yielding a hydrolytically stable product). The starting 10-silaxanthone **37** and 10-germaxanthone **24** were prepared relying on the following original method involving regioselective bromination of the protected precursors **35** and **22.** The key bromination of the precursor **35** to **36** is done in a mixed solvent (acetonitrile-CHCl₃ 3:1, to ensure homogeneous conditions) at 60 °C (the reaction with phenol ethers is significantly slower than with anilines). In case of diaryldimethylgermane **22,** bromination was performed in acetonitrile-pyridine (7:1) to suppress a bromodegermylation side reaction. Use of TIPS (triisopropylsilyl) protecting groups was a prerequisite for regioselective bromination; however, the resulting di-TIPSO-germaxanthone was found too lipophilic for successful purification by chromatography, and deprotection to **24** followed by reprotection with TBSCl was necessary to obtain pure 10-germaxanthone block **25.** The nucleophilic addition step **(25** or **37** + metalated **26)** with a turbo-Grignard reagent (*i*-PrMgCl·LiCl), as reported by Lavis et al. in Nat. Meth. 2015, 12, 244-2509, could not be reproduced with these substrates. Instead, the bromide **26** is amenable to low temperature lithium-halogen exchange (e.g. A. Nagaki et al., Chem. Eur. J. 2010, 16, 11167-11177) in mixed solvent (THF-pentane 2:1) or 2-MeTHF, reproducibly providing moderate yields (40-50%) of target lactones **38** and **27** with 10-silaxanthone **37** and 10-germaxanthone **25,** respectively. The following synthetic steps followed the procedure by Lavis et al. (ibid) employing 3-(tert-butylsilyloxy)azetidine **30** as an amine partner in Buchwald-Hartwig amination step **(29 → 31, 39 → 40).** Finally, desilylation and tert-butyl ester hydrolysis provided the target dyes **33** and **42.** The absorption/emission maxima are within 5 nm from the parent tetramethyl-*SiR* and tetramethyl-GeR analogs (641/662 nm for **42,** 645/661 nm for *SiR;* 631/651 nm for **33,** 634/655 nm for *GeR* **19).** Lower extinction coefficients of **42** (51500 M⁻¹cm⁻¹) and **33** (61000 M⁻¹cm⁻¹) compared to the parent *N,N,N',N*'-tetramethyl dyes (93000 M⁻¹cm⁻¹ for *SiR,* 97000 M⁻¹cm⁻¹ for *GeR* **19)** are due to higher propensity of **42** and **33** for closing into a colorless spirolactone form (e.g., D_{0.5} in 1,4-dioxane/water is 72.4 for **42** as compared to 64.5 for *SiR).* Remarkably, compared to both **19** (0.43), *SiR* (0.41) and **42** (0.42) and similarly to JF₆₄₆ (0.54), bis(3-hydroxyazetidinyl)-*GeR* **33** demonstrates an improved fluorescence quantum yield in PBS (0.60).

The chemical routes leading to dyes **33** and **42** (presented in Schemes 8 and 9) have universal features and represent a new method for preparing 10-germa- and 10-silaxanthones. These routes are based on common intermediates for the preparation not only of germa- and silafluoresceins, but also -rhodols and -rhodamines and comprise the following steps:
a) a regioselective bromination of di-*O*-TIPS-protected bis (3-hydroxyphenyl)silanes or -germanes to the corresponding di-O-TIPS-protected bis(2-bromo-5-hydroxyphenyl)silanes or - germanes using an electrophilic brominating reagent, preferably N-bromosuccinimide, similarly to the transformations **22 → 23** and **35 → 36;**
b) double metal-halogen exchange on the dibromide resulting from the step a) followed by reaction with a carbamoyl chloride, preferably N,N-dimethylcarbamoyl chloride, similarly to the transformations **23 → 24(25)** and **36 → 37.**

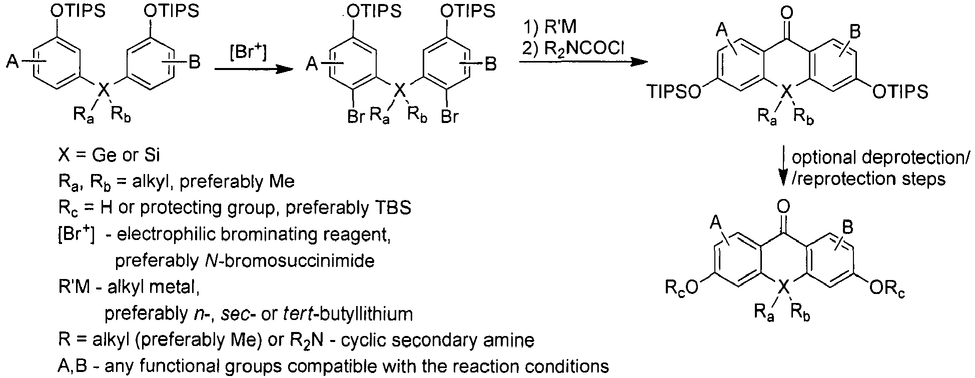

Consequently, a further aspect of the present invention relates to a method for preparing germa- and silaxanthones comprising the following steps:
a) a regioselective bromination of di-*O*-TIPS-protected bis (3-hydroxyphenyl)silanes or -germanes to the corresponding di-O-TIPS-protected bis(2-bromo-5-hydroxyphenyl)silanes or -germanes using an electrophilic brominating reagent, preferably *N*-bromosuccinimide;
b) double metal-halogen exchange on the dibromide resulting from the step a) followed by reaction with a carbamoyl chloride, preferably *N,N*-dimethylcarbamoyl chloride;
c) optionally further deprotection/reprotection steps.

This method is in particular advantageously applicable for preparing the compounds **24, 37-OH** below or their *O*-protected analoga (such as compounds **25** in Scheme 4 and **37** in Scheme 5):

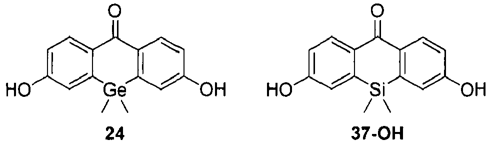

A further related aspect of the invention relates to the use of compounds according to formulae **I-III** and **4** above as reagents for conjugation or bioconjugation. More specifically, the conjugation or bioconjugation comprises formation of at least one covalent chemical bond or at least one molecular complex with a chemical entity or substance, such as amine, thiol, carboxylic acid, aldehyde, alcohol, aromatic compound, heterocycle (e.g, tetrazine), alkyne, alkene (including strained and bicyclic alkenes, e. g. *trans*-cyclooctene and norbornene derivatives), organic azide, dye, amino acid, amino acid residue coupled to any chemical entity, peptide, protein (including enzymes and immunoglobulins), antibody, "nanobody" (single-domain antibody), carbohydrate (including carbohydrate residue attached to a protein), nucleic acid, toxin, lipid, virus, virus-like particle.

Consequently, a closely related aspect of the invention represents the resulting conjugates or bioconjugates comprising a dye of the invention coupled via at least one covalent chemical bond or at least one molecular complex to a chemical entity or substance, such as amine, thiol, carboxylic acid, aldehyde, alcohol, aromatic compound, heterocycle, e.g. tetrazine, alkyne, alkene including strained and bicyclic alkenes, e. g. trans-cyclooctene and norbornene derivatives, organic azide, dye, amino acid, amino acid residue coupled to any chemical entity, peptide, protein, in particular enzymes and immunoglobulins, antibody, single-domain antibody, carbohydrate including a carbohydrate residue attached to a protein, nucleic acid, toxin, lipid, virus, virus-like particle.

In a more specific embodiment of the invention, the conjugates or bioconjugates comprise a dye as defined above coupled via a covalent bond to Pepstatin A, Jasplakinolide derivatives, Docetaxel derivative, and Aminophalloidin depicted below.

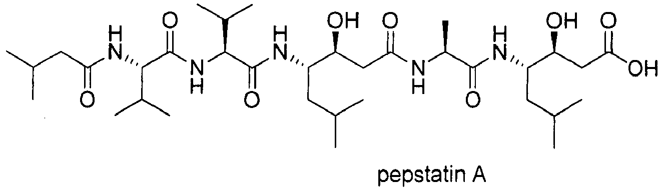

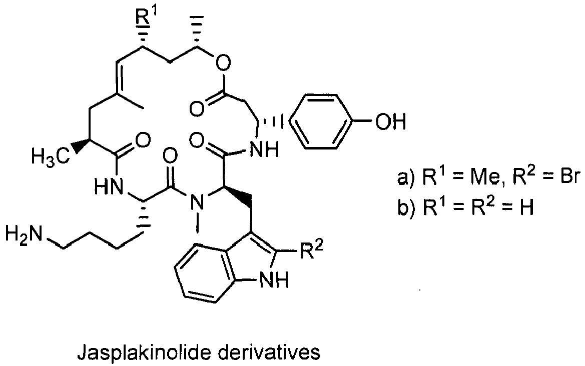

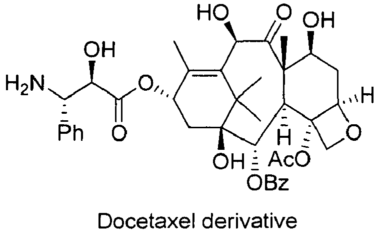

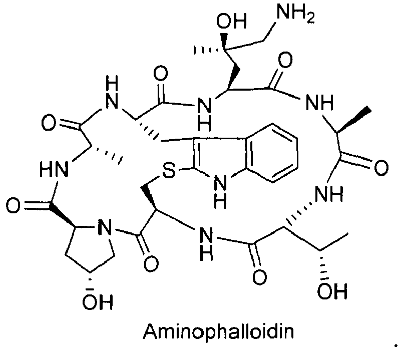

The compounds of formulae **I-III** and **4** above and their conjugates are particularly suitable and advantageous for use as cell permeant substances penetrating through membranes of living and fixed cells.

Consequently, closely related aspects of the present invention relate to this use (in *vitro* or in *vivo*) as well as to the use (in *vitro* or in *vivo*) of these compounds or of their conjugates for tracking and monitoring dynamic processes in a sample (in particular a tissue or cell sample) or in an object (including an organism or an animal body).

More specifically, the present invention relates to the use of said compounds or of their conjugates as fluorescent tags, analytical reagents and labels in optical microscopy, imaging techniques, protein tracking, nucleic acid labelling and flow cytometry.

The optical microscopy and imaging techniques wherein the claimed compounds may be advantageously used are not especially limited and for example comprise confocal microscopy, structured illumination microscopy, stimulated emission depletion microscopy [STED], fluorescence correlation spectroscopy [FCS], combination of STED and FCS (STED-FCS), fluorescence recovery after photobleaching [FRAP], fluorescence lifetime imaging [FLIM], ground state depletion with individual molecular return [GSD or GSDIM], RESOLFT microscopy (RESOLFT - reversible saturable (switchable) optically linear fluorescence transitions), fluorescence resonant energy transfer [FRET] (the dyes of the present invention may be used as FRET-donors and/or FRET-acceptors), single molecule switching techniques (SMS: diffraction unlimited optical resolution achieved by recording the fluorescence signals of single molecules, provided that they are reversibly or irreversibly switched between "dark" and "bright" states), such as single molecule localization microscopy [SMLM], photoactivation localization microscopy [PALM, PALMIRA, fPALM], and stochastic optical reconstruction microscopy [STORM].

The invention is further illustrated by the following non-limiting Examples and Figures.

### Figures

**Fig. 1** shows a) a STED image of a transfected HeLa cell expressing a vimentin-HaloTag fusion protein, live-labeled with **5**-Halo, counterpart confocal image in the lower left corner. b) and c) Close-ups of the boxed region from a) in confocal (b) and STED (c) mode.
**Fig. 2** shows a) a STED image of a transfected U2OS cell expressing the vimentin-HaloTag fusion protein, live-labeled with **14a**-Halo; b) and c) Close-ups of the boxed region from a) in confocal (b) and STED (c) mode.
**Fig. 3** shows a) a STED image of a U2OS cell expressing vimentin-HaloTag fusion protein, live-labeled with **6**-ROX-Halo; b) and c) Close-ups of the boxed region from a) in confocal (b) and STED (c) mode.
**Fig. 4** shows a) a STED image of a transfected HeLa cell, live-labeled with **19**-Tubulin; b) and c) Close-ups of the boxed region from a) in confocal (b) and STED (c) mode.
**Fig. 5** shows a) a STED image of a U2OS cell expressing vimentin-HaloTag fusion protein, live-labeled with **19-**Halo; b) and c) Close-ups of the boxed region from a) in confocal (b) and STED (c) mode.
**Fig. 6** shows a) a STED image of a transfected HeLa cell, live-labeled with **33-**Tubulin; (b) and c) Close-ups of the boxed region from a) in confocal (b) and STED (c) mode.
**Fig. 7** shows a) a STED image of a U2OS cell expressing vimentin-HaloTag fusion protein, live-labeled with **33-**Halo; b) and c) Close-ups of the boxed region from a) in confocal (b) and STED (c) mode.
**Fig. 8** shows a) a STED image of a transfected HeLa cell expressing vimentin-HaloTag fusion protein, live-labeled simultaneously with **14a**-Halo and **33-**Tubulin; b) and c) Close-ups of the boxed region from a1) in STED (b) and confocal (c) mode for vimentin-HaloTag fusion protein labeled with **14a-**Halo; d) and e) Close-ups of the boxed region from a2) in STED (d) and confocal (e) mode for **33-**Tubulin.

### General Materials and Methods

### Thin Layer Chromatography

Normal phase TLC was performed on *silica gel 60 F₂₅₄ (Merck Millipore, Germany).* For TLC on reversed phase *silica gel 60 RP-18 F_{254S} (Merck Millipore)* was used. Preparative TLC was performed on *HPTLC Silica gel 60 F₂₅₄ with concentrating zone 10 x 2.5 cm (Merck Millipore).* Compounds were detected by exposing TLC plates to UV-light (254 or 366 nm) or heating with vanillin stain (6 g vanillin and 1.5 mL conc. H₂SO₄ in 100 mL ethanol).

### Column Chromatography

*Silica gel 60* with a particle size of 40-63 µm was purchased from *Merck Millipore.* Reversed phase column chromatography was performed on *POLYGOPREP® 60-50 C₁₈ (Macherey Nagel GmbH* & *Co. KG, Germany).* Deactivated *silica gel 60* was purchased from *MP Biomedical.* Routine separation was performed with an automated *Isolera™ One* system *(Biotage AG, Sweden)* with commercially available cartridges.

### Absorption Spectroscopy

Absorption spectra were recorded with a Varian Cary 4000 UV-Vis double-beam spectrophotometer *(Agilent Technologies, USA).* For the determination of the absorption spectra, quartz cells with a 1 cm path length were used. Emission spectra and fluorescence quantum yield were obtained on a *Quantaurus-QY Absolute PL* quantum yield spectrometer C11347 *(Hamamatsu Photonics, Japan)* or on a Varian Cary Eclipse fluorescence spectrometer *(Agilent Technologies, USA).*

### Nuclear Magnetic Resonance (NMR)

NMR Spectra were recorded on an *Agilent 400MR DD2* spectrometer at room temperature (RT; 25°C). All spectra are referenced to tetramethylsilane as an internal standard (δ = 0.00 ppm) using the signals of the residual protons of CHCl₃ (7.26 ppm) in CDCl₃, CHD₂OD (3.31 ppm) in CD₃OD, CHD₂COCD₃ (2.05 ppm) in (CD₃)₂CO or DMSO-*d*₅ in DMSO-*d*₆. Multiplicities of the signals are described as follows: s = singlet, br. s = broad singlet, d = doublet, t = triplet, q = quartet, m = multiplet. Coupling constants ⁿ*J*_{x,y} are given in Hz, where n is the number of bonds between the coupled nuclei x and y. For ¹³C-signals, which were revealed by indirect detection by HSQC, only resonances of the carbon atoms linked to H-atoms were recorded.

### Mass-Spectrometry (MS)

Low resolution mass spectra (50 - 3500 m/z) with electro-spray ionization (ESI) were obtained on a *Varian 500-MS* spectrometer *(Agilent Technologies, USA).* High resolution mass spectra (ESI-HRMS) were obtained on a *Bruker micro TOF (ESI-TOF-MS)* spectrometer *(Bruker Corporation, USA).*

### High-Performance Liquid Chromatography (HPLC)

HPLC system *(Knauer, Germany):* Smartline pump 1000 (2x) with 10 mL pump-head, UV detector 2500, column thermostat 4000, mixing chamber, injection valve with a 20 or 50 µL loop for the analytical and 500 µL loop for preparative columns; 6-port-3-channel switching valve; analytical column: *Eurospher-100 C18* 5 µm (if not stated otherwise), or *Kinetex C18 100,* 5 µm, 250x4 mm, 1.2 mL/min; preparative column: *Kinetex C₁₈ 100,* 5 µm, 250x20 mm, 10 mL min/mL, solvent A: water + 0.1% v/v trifluoroacetic acid (TFA); solvent B: MeCN + 0.1% v/v TFA (if not stated otherwise.) For isolation and purification of the acid sensitive dyes or their derivatives, acetonitrile - aqueous systems containing 0.05 - 0.1 M of Et₃N*H₂CO₃ buffer (pH = 8; Sigma, or self-prepared from 1 M aq. Et₃N and CO₂ gas obtained by evaporation of solid CO₂).

### Cell culture and live-labeling

Either HeLa or U2OS cells were used. Cells were grown on #1.5 coverslips and cultured at 37°C with 5% CO2 in DMEM (Dulbecco's modified Eagle's medium) supplemented with 10% FBS (fetal bovine serum), 1% sodium pyruvate and 1% penicillin/streptomycin. The U2OS cell line is a stable cell line which has a stable expression of the vimentin-HaloTag fusion protein (M. Ratz, I. Testa, S.W. Hell, S. Jakobs, Sci. Rep. 2015, 5, 9592). For the expression of vimentin-HaloTag fusion protein in HeLa cells, cells were transfected with 3 µg DNA/well using TurboFectTM Transfection Reagent (Fermentas/Thermo ScientificTM, Life Technologies brand) in a 6 well plate. 24 hrs after transfection cells were incubated with the respective dye (typically 1 µM) for 20 minutes at growth conditions, washed for 10-30 minutes in HDMEM at 37°C and placed in an imaging chamber with pre-warmed HDMEM (37°C, DMEM lacking Phenol Red, 10 mM HEPES, pH7.4 1% penicillin/streptomycin). Live STED imaging was performed at room temperature.

### Live STED imaging

STED and confocal counterpart images were acquired using the commercially available two-color STED 775 quad scanning microscope from Abberior Instruments (Göttingen, Germany) equipped with an Olympus IX83 microscope stand and an Olympus UplanSApo 100x/1.4 OIL objective.

Dyes were excited respective their excitation maxima with either a pulsed 561 nm or a pulsed 640 nm laser. The STED laser was pulsed at 775 nm.

Fluorescence detection was done in two spectral detection channels, 605-625 nm and 650-720 nm.

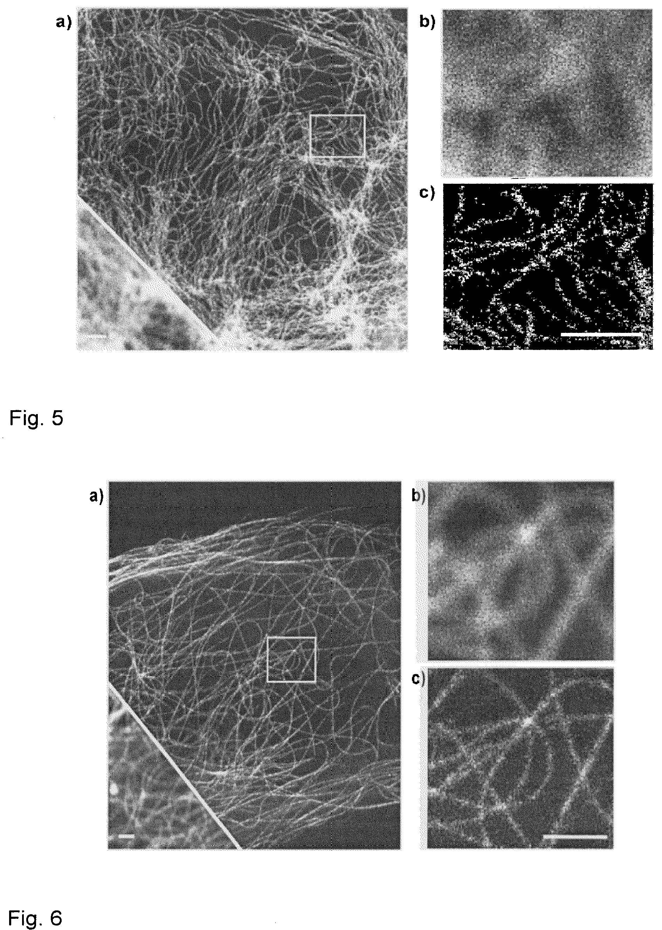

For compound **5**-Halo the excitation laser was pulsed at 532 nm and the STED laser at 631 nm (Figure 6). Fluorescence detection was from 540-580 nm.

Imaging and image processing was done with ImSpector software. Images are displayed as raw data unless stated in the figure legend. Pixel size was usually ∼20-30 nm.

### EXAMPLE 1

### Synthesis of fluorescent rhodamine dyes and their precursors

### N,N'-Bis-(2,2,2-trifluoroethyl)-6'-carboxy-Q-rhodamine-β,β'-diol (5)

**Compound 1-TBS.** A mixture of 3-(tert-butyldimethyl-silyloxy)aniline (prepared according to M. S. Hossain et al., Org. Biomol. Chem. 2015, 13, 5082-5085) (2.24 g, 10.0 mmol) and epichlorohydrin (0.94 g, 10.0 mmol) in acetic acid (20 mL) was stirred overnight at RT. The mixture was poured into aq. NaHCO₃ (30 g in 300 mL water), extracted with ethyl acetate (3 × 50 mL), and the combined organic layers were washed with brine and dried over MgSO₄. The product **1**-TBS was isolated by flash column chromatography (Biotage SNAP Ultra 100 g; gradient 0% to 5% ethyl acetate - CH₂Cl₂) as light yellow oil, yield 0.84 g (27%). ¹H NMR (400 MHz, CDCl₃): δ 7. 02 (t, *J* = 8.0 Hz, 1H), 6.29-6.23 (m, 2H), 6.16 (t, *J* = 2.2 Hz, 1H), 4.09 - 4.03 (m, 1H), 3.68 (dd, *J* = 11.3, 4.5 Hz, 1H), 3.63 (dd, *J* = 11.2, 6.1 Hz, 1H), 3.35 (dd, *J* = 13.3, 4.4 Hz, 1H), 3.21 (dd, *J* = 13.3, 7.1 Hz, 1H), 0.98 (s, 9H), 0.19 (s, 6H). ¹³C NMR (101 MHz, CDCl₃): δ 156.8, 149.1, 129.9, 110.1, 106.8, 105.2, 69.9, 47.7, 47.1, 25.7, 18.2, -4.4. ESI-MS, positive mode: *m*/*z* (rel. int., %) = 316.3 (100) [M+H]⁺.

**Compound 1-Me.** A mixture of 3-methoxyaniline (m-anisidine; 2.46 g, 20.0 mmol) and epichlorohydrin (2.03 g, 22.0 mmol, 1.1 eq) in acetic acid (7 mL) was stirred overnight at RT. The mixture was poured into aq. NaHCO₃ (15 g in 200 mL water), extracted with ethyl acetate (2 × 50 mL), the combined organic layers were washed with brine and dried over MgSO₄. The product **1-Me** was isolated by flash column chromatography (Teledyne Isco RediSep Rf 120 g; gradient 0% to 5% ethyl acetate - CH₂Cl₂) as light yellow oil, yield 1.66 g (38%). ¹H NMR (400 MHz, CDCl₃): 5 7.10 (t, *J* = 8.1 Hz, 1H), 6.32 (ddt, *J* = 8.2, 2.5, 0.7 Hz, 1H), 6.27 (ddt, *J* = 8.1, 2.3, 0.6 Hz, 1H), 6.22 (t, *J* = 2.3 Hz, 1H), 4.06 (ddtd, *J* = 7.2, 6.1, 4.5, 0.5 Hz, 1H), 3.77 (s, 3H), 3.69 - 3.58 (m, 2H), 3.36 (dd, *J* = 13.3, 4.4 Hz, 1H), 3.21 (dd, *J* = 13.3, 7.2 Hz, 1H). ¹³C NMR (101 MHz, CDCl₃) : δ 160.9, 149.2, 130.2, 106.3, 103.3, 99.4, 69.8, 55.2, 47.7, 47.1.

**Compounds 2a-TBS and 2b-TBS.** A solution of **1**-TBS (343 mg, 1.09 mmol) and N,N-diethylaniline (810 mg, 5.43 mmol, 5 eq) in 1,2-dichlorobenzene (15 mL) was heated at 140 °C (bath temperature) in a sealed vessel for 5 days. Upon cooling, 1 M NaOH (10 mL) was added, the organic layer was separated, and the aqueous layer containing viscous residue was extracted with CH₂Cl₂ (20 mL). The combined organic layers were washed with 1 M NaOH, water and dried over MgSO₄ . TLC control (SiO₂ / 20% ethyl acetate in CH₂Cl₂): *R*_{f} (product) = 0.3. The product was isolated by flash column chromatography (Teledyne Isco RediSep Rf 24 g; gradient 0% to 20% ethyl acetate - CH₂Cl₂) as light yellow oil, yield 0.84 g (27%), as a 2:1 mixture of regioisomers **2a**-TBS and **2b**-TBS, used in the next step without separation. Major 7-isomer (**2a**-TBS): ¹H NMR (400 MHz, CDCl₃): δ 6.81 (d, *J* = 8.3 Hz, 1H), 6.23-6.17 (m, 1H), 6.04 (d, *J* = 2.3 Hz, 1H), 4.23-4.18 (m, 1H), 3.32-3.28 (m, 1H), 3.23-3.20 (m, 1H), 2.97 (ddt, *J* = 16.1, 4.3, 1.3 Hz, 1H), 2.75-2.67 (m, 1H), 0.97 (s, 9H), 0.18 (s, 6H). Minor 5-isomer (**2b**-TBS): ¹H NMR (400 MHz, CDCl₃): δ 6.87 (t, *J* = 8.0 Hz, 1H), 6.23-6.17 (m, 2H), 4.27-4.22 (m, 1H), 3.28-3.24 (m, 1H), 3.20-3.17 (m, 1H), 2.85 (dd, *J* = 17.4, 4.7 Hz, 1H), 2.80-2.73 (m, 1H), 1.00 (s, 9H), 0.23 (s, 3H), 0.22 (s, 3H). ¹³C NMR (101 MHz, CDCl₃; **2a-**TBS/**2b**-TBS): 5 155.0, 154.8, 145.1, 144.3, 131.1, 126.9, 111.5, 110.3, 108.1, 107.3, 105.5, 63.6, 63.4, 47.6, 47.3, 34.9, 30.7, 25.8, 25.7, 18.3, 18.2, -4.2, -4.4. ESI-MS, positive mode: *m*/*z* (rel. int., %) = 250.2 (100) [M+H]⁺.

**Compound 2a-Me** *(7-isomer).* A solution of **1**-Me (1.61 g, 7.46 mmol) and *N,N*-diethylaniline (5.56 g, 37.3 mmol, 5 eq) in 1,2-dichlorobenzene (50 mL) was heated at 160 °C (bath temperature) in a sealed vessel for 5 days. Upon cooling, the volatiles were removed on a rotavapor (bath temperature 75 °C), the residue was dissolved in CH₂Cl₂ (100 mL), washed with 1 M NaOH and water (50 mL each). The organic layer was dried over MgSO₄, filtered and dried in vacuo at 85 °C to remove most of *N,N*-diethylaniline. TLC control (SiO₂ / 20% ethyl acetate in CH₂Cl₂): R_{f} (product) = 0.3. The product was isolated by flash column chromatography (Teledyne Isco RediSep Rf 80 g; gradient 0% to 30% ethyl acetate - CH₂Cl₂) as light yellow oil, yield 468 mg (35%) of **2a**-Me (7-isomer, nearly free from 5-isomer). ¹H NMR (400 MHz, CDCl₃): δ 6.88 (d, *J* = 8.3 Hz, 1H), 6.28 (dd, *J* = 8.3, 2.5 Hz, 1H), 6.10 (d, *J* = 2.5 Hz, 1H), 4.22 (qd, *J* = 4.6, 2.3 Hz, 1H), 3.74 (s, 3H), 3.31 (dtd, *J* = 11.3, 1.6, 0.8 Hz, 1H), 3.22 (ddd, *J* = 11.3, 5.1, 2.0 Hz, 1H), 3.02 - 2.92 (m, 1H), 2.78 - 2.67 (m, 1H), 2.32 (br.s, 1H). ¹³C NMR (101 MHz, CDCl₃): δ 159.2, 144.6, 131.4, 111.1, 104.2, 99.6, 63.7, 55.3, 47.7, 34.9. ESI-MS, positive mode: *m*/*z* (rel. int., %) = 180.2 (100) [M+H]⁺.

**Compound 3-Me** *(method A).* A solution of **2a-Me** (205 mg, 1.14 mmol) and triethylamine (289 mg, 2.86 mmol, 2.5 eq) in CH₂Cl₂ (10 mL) was cooled in dry ice-acetone bath, and trifluoroacetic anhydride (TFAA; 365 µL, 551 mg, 2.62 mmol, 2.3 eq) was added. The resulting mixture was stirred for 10 min, allowed to warm up to RT and stirred for 1 h. The mixture was diluted with CH₂Cl₂ (10 mL), washed sequentially with water, 10% aq. KHSO₄, sat. aq. NaHCO₃, brine (15 mL of each) and dried over MgSO₄. The filtrate was evaporated, the residue was dissolved in THF (10 mL) and BH₃·THF (1 M in THF, 8 mL, 8 mmol) was added. The reaction mixture was refluxed under argon overnight, cooled in ice-water bath and quenched by careful addition of methanol. The solution was evaporated, 1 N NaOH (15 mL) was added to the residue and the mixture was stirred for 30 min at RT. The reaction mixture was extracted with diethyl ether (3×20 mL), the organic layer was washed with sat. aq. NaHCO₃, brine (20 mL of each) and dried over MgSO₄. The product was isolated by flash column chromatography (Büchi Sepacore Silica HP 12 g; gradient 0% to 10% ethyl acetate - CH₂Cl₂), yielding 230 mg (77% over 2 steps) of **3-Me** as yellowish oil.
*Method B:* Solid 2,2,2-trifluoroethyl(mesityl)iodonium trifluoromethanesulfonate (T. Umemoto, Y. Gotoh, J. Fluorine Chem. 1986, 31, 231-236; G. L. Tolnai, A. Székely, Z. Makó, T. Gáti, J. Daru, T. Bihari, A. Stirling, Z. Novák, Chem. Commun. 2015, 51, 4488-4491) was added portionwise over 2-3 min to a solution of **2a**-Me (90 mg, 0.5 mmol) and 2,6-lutidine (87 µL, 80 mg, 0.75 mmol, 1.5 eq) in dry CH₂Cl₂ (3 mL), and the resulting clear solution was stirred at RT for 1 h. The mixture was diluted with sat. aq. NaHCO₃ (10 mL), extracted with CH₂Cl₂ (3×20 mL), the combined extracts were washed with brine and dried over Na₂SO₄. The product was isolated by flash column chromatography (Büchi Sepacore Silica HP 12 g; gradient 20% to 100% ethyl acetate - hexane), fractions containing the product were evaporated and dried in vacuo to remove any residual 2,6-lutidine. Yield 118 mg (90%) of **3**-Me as viscous yellowish oil. ¹H NMR (400 MHz, CDCl₃): δ 6.92 (dt, *J* = 8.2, 1.0 Hz, 1H), 6.33 (dd, *J* = 8.2, 2.4 Hz, 1H), 6.28 (d, *J* = 2.3 Hz, 1H), 4.21 (qd, *J* = 5.4, 2.7 Hz, 1H), 3.96 - 3.83 (m, 1H), 3.82 - 3.72 (m, 1H), 3.77 (s, 3H), 3.50 (dt, *J* = 11.7, 2.1 Hz, 1H), 3.29 (ddd, *J* = 11.7, 5.7, 1.8 Hz, 1H), 3.05 - 2.95 (m, 1H), 2.74 (ddt, *J* = 15.9, 5.6, 1.2 Hz, 1H), 2.14 (br.s, 1H). ¹³C NMR (101 MHz, CDCl₃) : δ 159.5, 144.3, 131.4, 125.6 (q, *J* = 283.3 Hz), 112.0, 103.4, 98.6 (q, *J* = 1.8 Hz), 63.3, 56.5, 55.4, 53.5 (q, J = 32.8 Hz), 35.6. ¹⁹F NMR (376 MHz, CDCl₃): δ -69.8. ESI-MS, positive mode: *m*/*z* (rel. int., %) = 262.1 (100) [M+H]⁺.

**Compound 3-H** *(from **2a,b**-TBS).* A solution of mixed isomers **2a,b**-TBS (169 mg, 0.605 mmol) and triethylamine (153 mg, 1.51 mmol, 2.5 eq) in CH₂Cl₂ (10 mL) was cooled in dry ice-acetone bath, and trifluoroacetic anhydride (TFAA; 193 µL, 292 mg, 1.40 mmol, 2.3 eq) was added. The resulting mixture was stirred at -78 °C for 10 min, allowed to warm up to RT and stirred for 1 h. The mixture was diluted with CH₂Cl₂ (10 mL), washed sequentially with water, 10% aq. KHSO₄, sat. aq. NaHCO₃, brine (15 mL of each) and dried over MgSO₄. The filtrate was evaporated, the residue was dissolved in THF (10 mL) and BH₃·THF (1 M in THF, 4.5 mL, 4.5 mmol) was added. The reaction mixture was refluxed under argon overnight, cooled in ice-water bath and quenched by careful addition of methanol. The solution was evaporated, the residue was treated with 1 M NaOH (20 mL), extracted with diethyl ether (10 mL), the organic layer was discarded, and the aqueous layer was carefully neutralized with 1 M HCl and then with phosphate buffer to pH 7.5. The resulting aqueous solution was saturated with NaCl and extracted with diethyl ether (3×20 mL), the combined extracts were dried over MgSO₄. The products were isolated by flash column chromatography (Interchim Puriflash 15 µm 25 g; gradient 0% to 40% ethyl acetate - CH₂Cl₂), yielding 45 mg (30%) of **3**-H (less polar 7-isomer, eluted first) as yellowish oil, along with 12 mg (∼8%) of impure 5-regioisomer.

**Compound 3-H** *(from **3**-Me).* A solution of **3**-Me (650 mg, 3.0 mmol), thiophenol (0.40 g, 3.6 mmol) and K₂CO₃ (20 mg) was heated in N-methylpyrrolidone at 180 °C (bath temperature) for 24 h. The reaction mixture was cooled down, diluted with diethyl ether (100 mL) and washed with 1 M NaOH (20 mL). The organic layer was discarded, and the aqueous layer was neutralized to pH ∼ 8 with 5% aq. citric acid. The title product (and, partially, thiophenol) was extracted with ether (3 x 20 mL). Combined organic solutions were dried (Na₂SO₄) and evaporated. The residue was subjected to chromatography on 25 cm³ SiO₂ (Isolera), and the title compound (0.4 g, 65%) isolated by elution with a CH₂Cl₂ - EtOAc mixture (10 - 50% EtOAc). ¹H NMR (400 MHz, CD₃CN) : δ 6.78 (dt, *J* = 8.0, 1.0 Hz, 1H), 6.63 (s, 1H), 6.19 (br.d, *J* = 2.6 Hz, 1H), 6.14 (dd, *J* = 8.0, 2.3 Hz, 1H), 4.05 (dddd, *J* = 11.9, 7.1, 4.7, 3.1 Hz, 1H), 4.00 - 3.81 (m, 2H), 3.42 (ddd, *J* = 11.6, 3.3, 1.9 Hz, 1H), 3.21 - 3.14 (m, 1H), 3.05 (d, *J* = 5.1 Hz, 1H), 2.87 (ddt, *J* = 15.5, 4.7, 1.3 Hz, 1H), 2.58 (ddt, *J* = 15.5, 7.1, 1.2 Hz, 1H). ¹⁹F NMR (376 MHz, CD₃CN) : δ -70.7. ¹³C NMR (101 MHz, CD₃CN) : δ 157.2, 145.7, 131.9, 127.2 (q, *J* = 282.7 Hz), 113.1, 105.9, 99.6 (q, *J* = 1.6 Hz), 63.7, 57.3, 53.7 (q, *J* = 32.2 Hz), 36.3. ESI-MS, positive mode: *m*/*z* (rel. int., %) = 248.1 (100) [M+H]⁺.

Compound **4** (Scheme 2) was prepared according to a published procedure (G. Mudd, I. Pérez Pi, N. Fethers, P. G. Dodd, O. R. Barbeau, M. Auer, Methods Appl. Fluoresc. 2015, 3, 045002).

### N,N'-Bis-(2,2,2-trifluoroethyl)-6'-carboxy-Q-rhodamine-β,β'-diol (5-OH)

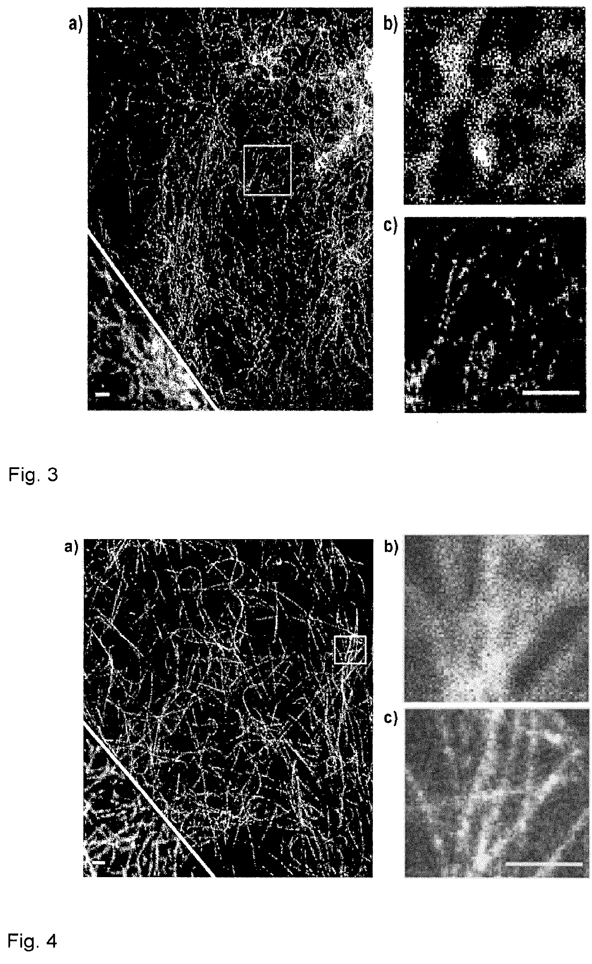

Compound **3** (25 mg, 0.1 mmol), compound **4** (15 mg, 0.072 mmol) and *p*-toluenesulfonic acid monohydrate (5 mg) were heated in propionic acid (1 mL) at 80 °C overnight. DDQ (16 mg, 0.07 mmol) was added to the reaction mixture at room temperature (RT), it was heated to 40 °C, and stirred at this temperature for 1 h. All volatile materials have been removed in vacuo, and the residue dissolved in CH₂Clz (100 mL). The organic solution was washed with water and sat. aq. NaHCO₃. Combined aqueous solutions were extracted with CH₂Cl₂ (20 mL). Combined organic solutions were dried (Na₂SO₄), filtered, and dichloromethane was evaporated in vacuo. The residue was dissolved in MeOH (3 mL), and 2 M aq. NaOH (0.5 mL) was added to the methanolic solution. The reaction mixture was kept overnight at room temperature, and all volatile materials were evaporated in vacuo. The residue was dissolved in aqueous MeCN and acidified with aq. TFA. TLC on reversed phase (VWR International, TLC Siliga gel 60 RP-18 F₂₅₄s) in the solvent system MeCN/H₂O = 1:2 (+0.3% HCOOH) revealed a brightly fluorescent orange spot. The title dye was isolated by RP chromatography on Polygoprep 60-50 C₁₈ (Macherey Nagel). Elution with aqueous acetonitrile (MeCN/H₂O = 1:2 +0.1% TFA) followed by direct lyophilization of the pooled fractions containing diastereomers of dye **5** afforded 8 mg of red voluminous solid (mixture of 3 diastereomers). HPLC: analytical column: *Kinetex C18 100,* 5 µm, 250x4.6 mm, 1.2 mL/min; solvent A: water + 0.1% v/v trifluoroacetic acid (TFA); solvent B: MeCN + 0.1% v/v TFA. Analytical method: A/B: 70/30 **→** 0/100 in 20 min, UV-VIS detection with dioden array. *t*_{R} = 5.4 min (isomers 1+2 are inseparable under these conditions), 5.6 min (isomer 3) in ratio ca. 2.5:1. Substances with *t*_{R} = 5.4 min and *t*_{R} = 5.4 min were isolated by preparative HPLC; preparative column: *Kinetex C18* 100, 5 µm, 250x20 mm, 10 mL min/mL, solvent A: water + 0.05% v/v trifluoroacetic acid (TFA); solvent B: MeCN. By analytical HPLC using another buffer [solvent A: water + 0.05 M Et₃N*H₂CO₃ buffer with pH = 8; solvent B: MeCN; method: A/B: 80/20 **→** 0/100 in 20 min, UV-VIS detection with dioden array], it turned out that the substance with *t*_{R} = 5.4 min (see above) was a mixture of two isomers (1+2) with *t*_{R} = 5.0 min and 5.2 min (∼2:1). Isomers 1+2 were used in the preparation of an NHS ester and an amide from Halo-Tag-amine (see below). Isomers differ in mutual orientations of hydroxyl groups (*syn* and *anti*)*.* For the *syn-*isomer, an additional pair of diastereomers is possible, due to the hindered rotation of the phenyl ring with 2 carboxylic acid groups (see Figure 4). Isomers can be separated by repeated chromatography on RP (using aqueous acetonitrile with 0.05 M Et₃N*H₂CO₃ buffer; pH = 8) followed by chromatography on regular SiO₂ using acetonitrile - dichloromethane solvent system (10/1) with increasing content of water (5 - 12%). ¹H NMR (400 MHz, CD₃OD, isomer 3): *δ* = 8.37 (m, 2H), 7.93 (d, J = 1.2 Hz, 1H), 7.22 (s, 2H), 6.95 (s, 2H), 4.58 (dd, ²*J* _{HH} = 16.6, ³*J* _{HF} = 8.6 Hz, 2H), 4.37 (dd, ²*J* _{HH} = 16. 6, ³ *J* _{HF} = 8.6 Hz, 2H), 4.22 (m, 2H, CHOH), 3.79 (m, 2H), 3.55 (ddd, *J* = 10.6, 5.8 and 3.1 Hz, 2H), 2.98 (m, 2H), 2.77 (dd, J = 16.3, 5.8, 2H) ppm. ¹⁹F NMR (376 MHz, CD₃OD, isomer 3): *δ* = - 70.2 (t, ³*J* _{HF} = 8.8 Hz) ppm. The following spectral data mere obtained for aqueous solutions. Isomer 3: absorption λₘₐₓ, nm (ε, M⁻¹ cm⁻¹) = 532 (56000), emission λₘₐₓ, nm (*Φ*_{fl} -fluorescence quantum yield) = 553 (0.89 - absolute value; excitation at 488 nm). C₃₁H₂₄F₆N₂O₇ (650.1488). HR-MS, ESI (positive mode): m/z = 651.1549 (found [M+H]⁺), 651.1560 (calc.); ESI (negative mode): *m*/*z* (rel. int., %) = 649.1408 (100) (found [M-H]⁻); 649.1415 (calc.).

### N-Hydroxysuccinimidyl ester of N,N'-bis-(2,2,2-trifluoroethyl)-6'-carboxy-Q-rhodamine-β,β'-diol (5-NHS) and its conjugate with Halo-Tag amine (5-Halo)

Three stock solutions (A, B and C) were prepared. Solution A: 22 mg HATU (1-[bis(dimethylamino)methylene]-1H-1,2,3-triazolo[4,5-b]pyridinium 3-oxid hexafluorophosphate) in dry DMF (0.10 mL) ; solution B: Et₃N (10 mg) in dry DMF (0.10 mL) ; solution C: N-hydroxysuccinimide (19 mg) in dry DMF (0.10 mL). To a solution of dye 5 (1.7 mg, 2.6 µmol) in dry DMF (0.10 mL), solutions A, B and C were added successively (14 µL of each solutions) under argon, and the mixture was stirred for 30 min at room temperature. HPLC analysis indicated the conversion degree of 94-95%. *(Eurospher-100 C18* 5 µm 250x4 mm, 1.2 mL/min; solvent A: water + 0.1% v/v trifluoroacetic acid (TFA); solvent B: MeCN + 0.1% v/v TFA. A/B: 30/70 - 100/0, 25 min, *t*_{R} = 6.7 min (compound **5),** 8.3/8.6 min (mono-NHS esters, mixture of diastereomers). The product (6'-NHS ester of dye **5,** a stable compound **5**-NHS) can be isolated by preparative HPLC (after removing DMF in vacuo), or used *in situ.* In the latter case, Halo-Tag-amine (H₂N(CH₂)₂O(CH₂)₂O(CH₂)₆Cl = C₁₀H₂₂ClNO₂, 1.5 mg, 6.7 µM) was added to the reaction mixture as a solution in DMF (20-40 µL), and the reaction mixture was stirred at 40 °C. After several hours, HPLC control indicated that the conversion to new products with *t*_{R} = 11.9/12.2 min (**5**-Halo, mixture of dye diastereomers) was nearly complete (93%; HPLC area). After removing DMF in vacuo, amide **5**-Halo was isolated by preparative HPLC (∼1 mg, 46%), dissolved in DMSO (400 (µL), and this stock-solution was used (after dilution with aqueous PBS to 1 - 5 µM) in labeling experiments with living cells (see section with imaging results). ESI-MS (C₄₁H₄₄ClF₆N₃O₈, 855.27), positive mode: *m*/*z* (rel. int., %) = 856 (100) [M+H]⁺. HR-MS, ESI (positive mode): *m*/*z* = 878.2591 (found [M+Na]⁺), 878.2613 (calc.).

### Hydroxylated ROX dyes (13a,b)

**7-(tert-Butyl-dimethyl-silanyloxy)-1,2,3,4-tetrahydroquinoline (6)** in Scheme 2 was prepared according to the published method of S. M. Pauff and S. C. Miller (Org. Lett. 2011, 13, 6196-6199) with modifications (Kolmakov et al., Chem. Eur. J. 2015, 21, 13344-13356).

**Compound 7.** Compound **6** (90 mg) was dissolved in neat epichlorohydrin (1 mL), and the solution was heated at 80 °C for 30 h. The reaction mixture was applied onto column with SiO₂ (10 g) packed in hexane - dichloromethane mixture (3:1). Elution with hexane - dichloromethane (3:1 **→** 1:1) afforded 60 mg of compound **7** (oil). ¹H NMR (400 MHz, CDCl₃): *δ* = 6.80 (dd, *J* = 8.1, 0.9 Hz, 1H), 6.16 (d, *J* = 2.3 Hz, 1H), 6.13 (dd, *J* = 8.1, 2.3 Hz, 1H), 4.15 (m, 1H), 3.70 (dd, *J* = 11.2, 4.3 Hz, 1H), 3.62 (dd, *J* = 11.3, 5.4 Hz, 1H), 3.35 (dd, *J* = 6.4, 4.0 Hz, 1H), 3.30 (dd, *J* = 6.4, 4.0 Hz, 1H), 3.30 (td, *J* = 5.3, 1.3 Hz, 2H), 2.70 (t, *J* = 6.4 Hz, 2H), 2.49 (d, *J* = 4.6 Hz, 1H), 1.92 (m, 2H), 0.98 (s, 9H), 0.20 (s, 6H). ¹³C NMR (101 MHz, CDCl₃): *δ* = 156.8, 149.0, 129.9, 110.1, 106.8, 105.2, 69.9, 47.7, 47.1, 25.70, 18.2, -4.4. MS (ESI): *m*/*z* (positive mode, %) = 356 (100) [M+H]⁺.

**Alcohol 8a and oxirane 8b (*tert*-butyl-dimethyl-[[1-(oxiran-2-ylmethyl)-3,4-dihydro-2*H*-quinolin-7-yl]-oxy]silane.** Amine **6** (1.60 g, 6.08 mmol) was dissolved in a mixture of epichlorohydrine (7.28 g, 79.1 mmol, 6.15 mL) and 1,2-dichlorobenzene (15 mL). The mixture was heated at 140°C (oil bath temperature) for 18 h in a thick-walled screw-cup test tube. Then all volatile materials were distilled-off under reduced pressure (0.5-1 mbar, bath temperature 50-100°C) leaving 2.64 g of colorless oil. TLC (hexane - ethyl acetate, 3:1, or dichloromethane - ethyl acetate, 10:1) indicated complete consumption of the starting material (compound **6)** and formation of two new compounds. The residual oil was subjected to chromatography over regular silica gel *(Isolera, RediSep Rf* cartridge with 40 g SiO₂ hexane - ethyl acetate, from 95:5 to 70:30). Alcohol **8a** was isolated as a red-brown viscous oil (1.02 g, 52%); oxirane **8b** (0.52 g, 27%) - as a light yellow viscous oil.

Compound **8a.** ¹H NMR (400 MHz, CDCl₃) : *δ* = 6.70 (dt, *J* = 8.1, 0.9 Hz, 1H), 6.16 (d, *J* = 8.1 Hz, 1H), 4.36 (dddd, *J* = 14.4, 7.7, 6.8, 4.9 Hz, 1H), 3.16 - 2.99 (m, 3H), 2.90 - 2.61 (m, 4H), 2.37 (d, *J* = 9.3 Hz, 1H), 2.10 - 1.92 (m, 2H), 0.99 (s, 9H), 0.20/0.21 (2×s, 6H). ¹³C NMR (101 MHz, CDCl₃): *δ* = 152.7, 143.3, 126.8, 115.1, 110.4, 107.4, 63.3, 55.4, 50.4, 31.50, 27.1, 25.8, 25.6, 22.3, 18.2, -4.1. MS (ESI): *m*/*z* (positive mode, %) = 320.2 (100) [M+H]⁺. HR-MS (C₁₈H₂₉NO₂Si): 320.2048 (found M+H), 320.2040 (calc.); 342.1864 (found for M+Na), 342.1860 (calc.).

Compound 8b. ¹H NMR (400 MHz, CDCl₃): *δ* = 6.77 (ddt, *J* = 7.8, 1.0, 0.5 Hz, 1H), 6.16 - 6.03 (m, 2H), 3.52 (dd, *J* = 12 and 3.2 Hz, 1H), 3.39 - 3.25 (m, 3H), 3.15 (dddd, *J* = 4.6, 4.0, 3.2, 2.7 Hz, 1H), 2.81 - 2.75 (m, 1H), 2.68 (t, *J* = 6.4 Hz, 2H), 2.58 (dd, *J* = 5.0, 2.7 Hz, 1H), 2.00 - 1.80 (m, 2H), 0.97 (s, 9H), 0.19 (s, 6H). ¹³C NMR (101 MHz, CDCl₃) *δ* = 154.9, 146.1, 129.5, 115.6, 107.7, 103.0, 52.9, 50.4, 50.3, 45.4, 27.3, 25.8, 22.4, 18.2, -4.4. MS (ESI): *mlz* (positive mode, %) = 320 (100) [M+H]⁺. HR-MS (C₁₈H₂₉NO₂Si): 320.2041 (found M+H), 320.2040 (calc.).

**8a-Acetate.** In an argon atmosphere, alcohol **8a** (0.66 g, 2.1 mmol) was dissolved in pyridine (2.22 g, 28.0 mmol, 2.26 mL) in a screw-cup tube containing a stirring bar. Acetic anhydride (1.19 g, 11.6 mmol, 1.10 mL) was added at room temperature, and the reaction mixture was stirred for 18 h. The course of the reaction was monitored by TLC using dichloromethane - hexane mixture (3:1) with 10% v/v of ethyl acetate. The reaction mixture was concentrated in vacuo to ca. 3/10 of its initial volume and diluted with ethyl acetate (100 mL). The organic solution was shaken with saturated aq. KHSO₄ (20 mL); an aqueous phase was separated and extracted with EtOAc (100 mL). The combined organic solutions were washed with water, saturated aqueous NaHCO₃ and brine. The organic phase was dried over Na₂SO₄, and the solvent was evaporated in vacuo. The residue - a reddish-brown viscous oil - was subjected to chromatography on regular SiO₂. Elution with dichloromethane - hexane (1:1 to 1:0) gave acetate **9** (0.564 g, 75%) as a red-orange viscous oil. ¹H NMR (400 MHz, CDC1₃): *δ* = 6.68 (dt, J = 8.1, 0.9 Hz, 1H), 6.11 (d, *J =* 8.1 Hz, 1H), 5.26 (m_{c}, 1H), 3.26 (ddd, *J* = 11.3, 3.5, 1.7 Hz, 1H), 3.17-2.92 (m, 4H), 2.76-2.62 (m, 3H), 2.07 (s, 3H), 2.02-1.91 (m, 2H), 0.99 (s, 9H), 0.21 (s, 6H). ¹³C NMR (101 MHz, CDCl₃): *δ* = 170.8, 152.1, 143.0, 126.8, 114.6, 109.5, 106.8, 66.9, 52.3, 50.1, 27.9, 27.1, 25.8, 22.2, 21.4, 18.3, -4.1. MS (ESI): *m*/*z* (positive mode, %) = 362.2 (100) [M+H]⁺, HR-MS (C₂₀H₃₁NO₃Si): 384.1964 (found for M+Na), 384.1965 (calc. for M+Na).

**Phenol 9.** A solution of **8a**-acetate (0.560 g, 1.55 mmol) in acetonitrile (14 mL) was prepared in a polyethylene screw-cap bottle and cooled with ice. An aqueous HF solution (0.2 mL of 50-55% solution) was added; the ice bath was removed, and the reaction mixture was stirred at room temperature for 20 h. TLC (dichloromethane/ethyl acetate, 10: 1) indicated the full conversion of the staring material. The reddish reaction solution was diluted with H₂O (50 mL) and extracted four times with ethyl acetate (50 mL and 3 × 25 mL). The combined organic solutions were washed with brine and dried over Na₂SO₄. The solvents were removed under reduced pressure, and the residue (0.46 g of a green viscous semi-solid) was subjected to chromatography (PuriFlash HC, 25 g SiO₂, 15 µm, dichloromethane/ethyl acetate 95: 5 → 80:20). Yield - 0.314 g (82%) of phenol **9** as a white solid. ¹H NMR (400 MHz, CDCl₃): *δ* = 7.26 (s, 1H), 6.69 (d, *J* = 8.0, 1H), 6.07 (d, *J* = 8.0 Hz, 1H), 5.26 (tdd, *J* = 6.8, 5.8, 3.5 Hz, 1H), 4.48 (s, 1H), 3.28 (ddd, *J* = 11.4, 3.5, 1.7 Hz, 1H), 3.19-2.98 (m, 4H), 2.78-2.66 (m, 3H), 2.07 (s, 3H), 2.11-1.89 (m, 2H). ¹³C NMR (101 MHz, CDCl₃): *δ* = 170.7, 152.0, 142.9, 127.1, 114.3, 105.2, 103.3, 66.6, 52.2, 50.1, 27.1, 22.2, 21.3. MS (ESI): *m*/*z* (positive mode, %) = 248.1 (100) [M+H]⁺. HR-MS (C₁₄H₁₇NO₃) : 270.1105 (found for M+Na), 270.1101 (calc.).

**Benzophenone 12a.** In a screw-cap bottle, 8-hydroxyjulolidine **10** (0.60 g, 3.17 mmol) and trimellitic anhydride **11** (1.83 g, 9.51 mmol) were suspended in acetic acid (15 mL) and flushed with argon. Conc. H₂SO₄ (1 drop) was added, and the reaction mixture was gradually heated in an oil bath (up to 100 °C) and stirred for 2.5 h. The precipitate dissolved at about 80-90° C, and a red solution formed. After about 0.5 h, a "new" precipitate was formed. After cooling to room temperature, the purple suspension was transferred into centrifuge test-tubes, sonicated for several seconds in an ultrasonic bath, centrifuged, and the purple supernatant solution was discarded. The precipitate was washed with acetic acid (5 mL); the same sonication - centrifugation cycles were repeated 5 - 6 times, until the supernatant solution became colorless. The solid residue was dried in vacuo to obtain 0.48 g (40%) of benzophenone **12a** with traces of **12b** (>95:<5). Beige solid; TLC: ACN/H₂O/DCM, 9:1:1. HPLC *(Eurospher-100 C₁₈* 5 µm 250x4 mm, 1.2 mL/min; solvent A: water + 0.1% v/v trifluoroacetic acid (TFA); solvent B: MeCN + 0.1% v/v TFA). A/B: 30/70 - 100/0, 25 min, *t*_{R} = 9.7 min **(12a)**, 10.7 min **(12b).** ¹H NMR (400 MHz, DMSO-*d₆*) δ 12.82 (s, 1H), 8.09 (dd, *J* = 8.1, 1.7 Hz, 1H), 8.02 (d, *J* = 8.1 Hz, 1H), 7.76 (d, *J* = 1.7 Hz, 1H), 6.39 (s, 1H), 3.24 (td, *J* = 7.4, 4.8 Hz, 4H), 2.58 (t, *J* = 6.4 Hz, 2H), 2.40 (t, *J* = 6.2 Hz, 2H), 1.91-1.78 (m, 2H), 1.80-1.69 (m, 2H). C₂₁H₁₉NO₆ (381.12124). MS (ESI): m/z (negative mode, %) = 380 (100) [M-H]⁻.

**Compounds 13a-c.** Benzophenone **12a** (23 mg, 0.060 mmol) and acetate **9** (15 mg, 0.061 mmol) were dissolved in 2.3 mL DMF, trimethylsilyl polyphosphate (170 mg) in DMF (0.4 mL) were added, and the reaction mixture was heated under argon for 3 h at 100°C (or 2 h at 120°C). After cooling down to room temperature, the reaction mixture was diluted with 40 mL of the half-saturated brine and transferred into a separation funnel with CH₂Cl₂ (40 mL) and 5 mL acetonitrile. After shaking and separation of the organic layer, the aqueous phase was extracted with CH₂Cl₂ - acetonitrile mixture (40 mL + 5 mL) and with CH₂Cl₂ (20 mL). Combined organic solutions were washed with water (20 mL), and the aqueous extract was washed with CH₂Cl₂ (40 mL). HPLC analysis of the reaction mixture: column - *Eurospher-100 C18* 5 µm, 250x4 mm, 1.2 mL/min; solvent A: water + 0.1% v/v trifluoroacetic acid (TFA); solvent B: MeCN + 0.1% v/v TFA A/B = 70/30 - 0/100 in 20 min, detection at 580 nm. Retention times (*t*_{R}), min (peak areas, %, compounds): 10.2 (30, **13b)**, 11 (46, **13a),** 12.3 (24, **13c).** Combined organic solutions were evaporated in vacuo. and the residue was applied onto a column with regular SiO₂ (55 g). Elution with acetonitrile - water mixture (10:1 **→** 5:1 + 5% v/v CH₂Cl₂) afforded magenta-colored fractions; first these with compound **13c,** and then - inseparable mixture of mono- and diacetates of dyes **13a** and **13b** (displaying a single spot on TLC). These fractions were combined, evaporated in vacuo, and the residue was dissolved in 10 mL of 0.1 M aq. NaOH. After keeping overnight at room temperature, the reaction mixture was acidified with TFA (0.2 mL). Then the reaction mixture saturated with NH₄Cl, and extracted twice with CH₂Cl₂ - acetonitrile mixtures (5:1 and 5:2, 60 mL and 70 mL, respectively). Combined organic solutions were evaporated in vacuo, and the residue was subjected to chromatography on regular SiO₂ using the solvent system CH₂Cl₂ - MeOH - H₂O (75/30/2). Dyes **13a** and 13b were isolated in approximately equal amounts and additionally purified by RP chromatography on RP C18 (Macherey Nagel, Polygoprep 60-50) using acetonitrile - water mixture (1/5 **→** 1/1; +0.2% v/v HCOOH). Homogeneous fractions were pooled, evaporated and afforded diastereomers **13a** and and 7.7 min (in ratio ca. 2:3:1; HPLC conditions as above). ¹H NMR (400 MHz, CD₃OD, **13a,** mixture of 3 diastereomers): *δ* = 8.17 (dd, *J* = 8.1 and 1.7 Hz, 1H), 8.06 (d, *J* = 7.9 Hz, 1H), 7.78 - 7.71 (m, 1H), 6.85-6.79 ("m", 1H), 4.57 (m, 1H), 4.36 (m, 2H), 3.64 (br. d, *J* = 13.0 Hz, 2H), 3.50 (dq, *J* = 17.5, 6.1 Hz, 4H), 3.38 (dd, *J* = 13.4, 5.2 Hz, 1H), 3.21 - 3.13 (m, 1H), 3.08 (t, *J* = 6.5 Hz, 1H), 2.70 (s, 4H), 2.12 (m, 2H), 1.99 - 1.89 (m, 4H). ¹H NMR (400 MHz, CD₃OD, compound **13b;** main diastereomer): *δ* = 8.16 (dd, *J* = 8.1 and 1.7 Hz, 1H), 8.04 (d, *J* = 8.0 Hz, 1H), 7.73 (m, 1H), 6.85 (s, 2H), 4.36 (m, 2H, CHOH), 3.65 (br. d, *J* = 13.5 Hz, 2H), 3.48 (m, *J* = 5.6 Hz, 4H), 3.38 (dd, *J* = 13.3 and 5.2 Hz, 2H), 3.18 (m, 4 H), 2.71 (m, 4H), 1.96 (m, 4H) ppm. The following spectral data mere obtained for aqueous solutions containing PBS buffer (pH 7.4). **13a:** absorption λ_{max,} nm (*ε*, M⁻¹ cm⁻¹) = 574 (55000), emission λ_{max,} nm (*Φ*_{fl} - fluorescence quantum yield) = 597 (0.74 - absolute value; excitation at 540-550 nm). **13b:** absorption λ_{max,} nm (*ε*, M⁻¹ cm⁻¹) = 564 (60000), emission λ_{max,} nm (*Φ*_{fl} -fluorescence quantum yield) = 588 (0.63 - absolute value; excitation at 550 nm). **13a** (C₃₃H₃₀N₂O₆, M = 550). HR-MS (ESI): *m*/*z* (positive mode) = 551.2181 (found [M+H]⁺), 551.2177 (calculated). MS (ESI): *m*/*z* (negative mode, %) = 549 (100) [M-H]⁻. **13b** (C₃₃H₃₀N₂O₇, M = 566). HR-MS (ESI): *m*/*z* (negative mode) = 565.1976 (found [M-H]⁻), 565.1980 (calculated).

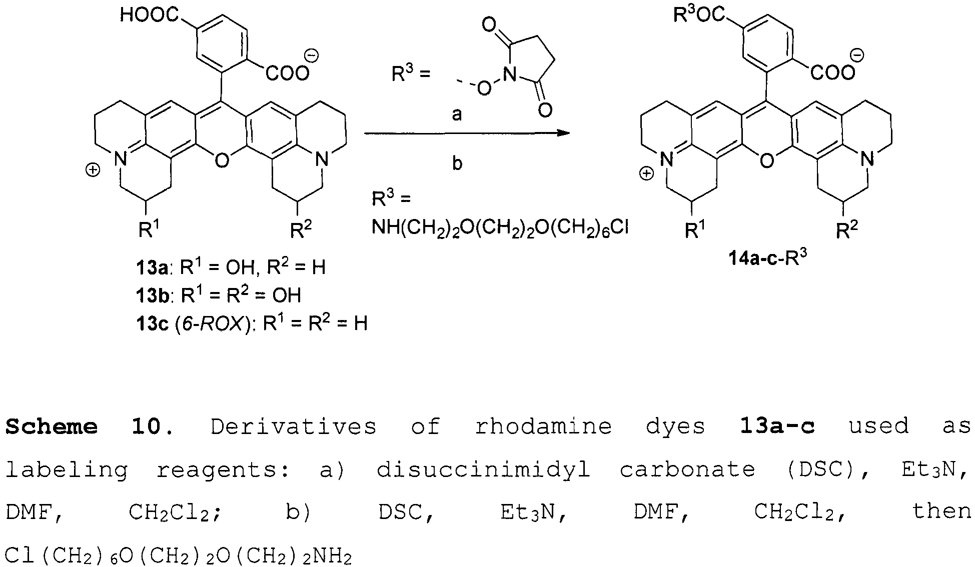

**General method for obtaining *mono*-NHS esters 14a-c-NHS.** A dye **(13a, 13b** or **13c)** in an amount of ca. 3.5 mg (ca. 6.3 µM) is dissolved in the mixture of DMF (1.5 mL) and CH₂Cl₂ (0.5 mL) and treated with Et₃N (portions of 2.5 µL) and disuccinimidyl carbonate (portions of 5 mg). After addition of each portion, the course of the reaction is monitored by HPLC [column - *Eurospher-100 C18* 5 µm, 250x4 mm, 1.2 mL/min; solvent A: water + 0.1% v/v trifluoroacetic acid (TFA); solvent B: MeCN + 0.1% v/v TFA A/B = 70/30 - 0/100 in 20 min, detection at 580 nm] and/or TLC (regular SiO₂, ACN/H₂O, 5:1). The reaction was complete, when 20 mg of DSC and 15 µL of Et₃N were added. The spot of the double NHS ester was also detected (with highest *R*_{f}; blue color), and (sometimes) the spot of the mono-NHS ester of the sterically more hindered carboxylic acid group. The latter had the lowest *R*_{f}-value (and gave the bluish spot). The reaction mixture was diluted with the equal volume of dry CAN and applied onto a column with regular SiO₂ (10 g). Elution with ACN/CH₂Cl₂/H₂O (10:1:1) followed by ACN/H₂O (5:1) afforded the required mono-NHS esters **14a-c**-NHS as magenta-colored solutions. They were concentrated, filtered through the Rotilabo membrane filters (attached to syringes; in order to remove SiO₂) and lyophilized. **14a**-NHS (C₃₇H₃₃N₃O₈, M = 647). MS (ESI): *m*/*z* (positive mode, %) = 648 (100) [M+H]⁺; **14b**-NHS (C₃₇H₃₃N₃O₉, M = 663.2217). HR-MS (ESI): *m*/*z* (positive mode, %) = 664.2252 (found [M+H]⁺), 664.2290 (calculated).

**General method for obtaining mono-amides 14a-c-NH(CH₂)₂O(CH₂)₂O(CH₂)₆Cl.** *Mono-NHS* esters **14a-c-NHS** in amounts of ca. 1 mg (ca. 2 µM) were dissolved in ca. 200 µL of the solution of Cl(CH₂)₆O(CH₂)₂O(CH₂)₂NH₂ in DMF (20 mg/mL), and Et₃N (10 µL) was added with stirring. The course of the reaction was monitored by TLC. When the reaction was complete (ca. 2 h), the reaction mixture was diluted with CH₂Cl₂ - acetonitrile mixture (1:3) and extracted with equal volume of the saturated aqueous NaHCO₃ solution (lower phase). The upper organic layer was separated and extracted with half-satuarated aqueous NH₄Cl acidified with trifluoroacetic acid (TFA; in order to remove excess of the amine). The organic solution was evaporated in vacuo, and the residue applied onto a column with regular SiO₂ (5 g). Elution with CH2Cl2 - methanol (10:1 → 8:1) afforded the title amides which were additionally purified by chromatography on regular SiO₂ (5 g) using MeCN - H₂O mixture (10:1 → 5:1). Pure fractions were pooled, concentrated, filtered through the Rotilabo syringe filters (0.22 µm), and lyophilized. **14a-NH(CH₂)₂O(CH₂) ₂O(CH₂)₆Cl** (C₄₃H₅₀N₃O₇Cl): HR-MS (ESI): m/z (positive mode, %) = 756.3384 (found [M+H]⁺), 756.3410 (calculated). HPLC [column - *Kinetex* 5 µm C18 100, 250x4 mm, 1.2 mL/min; solvent A: 0.05 M Et₃N*H₂CO₃ buffer (TEAB); solvent B: MeCN, A/B = 70/30 - 0/100 in 20 min, dioden-array detection at 600 nm]; *t*_{R} = 9.7 and 10.5 min (in ratio 1:1) - 2 diastereomers differing in the mutual position of OH and COOH groups; see main text. The following spectral data were obtained for aqueous solutions containing PBS buffer

(pH 7.4). Absorption λₘₐₓ, nm (*ε*, M⁻¹ cm⁻¹) = 578 (55000), emission λₘₐₓ, nm (*Φ*_{fl}) = 601 (0.74 - relative value obtained with Rhodamine 101 as a reference dye with *Φ*_{fl} = 0.96 in ethanol; excitation at 540 nm). **14b-NH (CH₂)₂O(CH₂)₂O(CH₂)₆Cl** (C₄₃H₅₀N₃O₈Cl): HR-MS (ESI): *m*/*z* (positive mode, %) = 772.3329 (found [M+H]⁺), 772.3359 (calculated). HPLC [column - *Kinetex* 5 µm C18 100, 250x4 mm, 1.2 mL/min; solvent A: 0.05 M Et₃N*H₂CO₃ buffer (TEAB); solvent B: MeCN, A/B = 70/30 - 0/100 in 20 min, diode array detection in the range 200-700 nm]; *t*_{R} = 8.0 and 8.0 min (in ratio 1:1) - 2 diastereomers differing in the mutual position of OH and COOH groups; see main text. The following spectral data were obtained for aqueous solutions containing PBS buffer (pH 7.4). Absorption λₘₐₓ, nm (*ε*, M⁻¹ cm⁻¹) = 575 (63000), emission λₘₐₓ, nm (*Φ*_{fl}) = 598 (0.55 - relative value obtained with Rhodamine 101 as a reference dye with *Φ*_{fl} = 0.96 in ethanol; excitation at 540 nm). **14c-NH(CH₂)₂O(CH₂)₂O(CH₂)₆Cl.** The following spectral data were obtained for aqueous solutions containing PBS buffer (pH 7.4). Absorption λₘₐₓ, nm (*ε*, M⁻¹ cm⁻¹) = 582 (55000), emission λₘₐₓ, nm (*Φ*_{fl}) = 605 (0.64 - relative value obtained with Rhodamine 101 as a reference dye with *Φ*_{fl} = 0.96 in ethanol; excitation at 540 nm).

### EXAMPLE 2

### Synthesis of Ge-rhodamine dyes

### Synthesis of tetramethyl-Ge-rhodamine 19

**Compound 15.** A solution of 3-bromo-*N,N*-dimethylaniline (2.32 g, 11.6 mmol, 2 eq) in anhydrous THF (40 mL) was degassed on a Schlenk line and cooled to -78 °C under argon. n-butyllithium (5.1 mL of 2.5 M solution in hexanes, 12.76 mmol, 2.2 eq) was injected with a syringe quickly dropwise, and the mixture was stirred at -78 °C for 1 h. Dimethylgermanium dichloride (1.01 g, 5.8 mmol), dissolved in anhydrous THF (3 mL), was injected dropwise with a syringe. The mixture was allowed to warm up to RT and stirred for 2.5 h. Brine (50 mL) was then added, the mixture was extracted with ethyl acetate (3 × 40 mL), and the combined organic layers were dried over Na₂SO₄. TLC control (SiO₂ / 10% ethyl acetate in hexane): R*_{f}* (product) = 0.37. The product **15** was isolated by flash column chromatography (Biotage SNAP Ultra 50 g, gradient 1% to 10% ethyl acetate - hexane over 15 column volumes) as colorless oil, yield 1.56 g (78%). ¹H NMR (400 MHz, CDCl₃) : δ 7.27 (ddd, J = 8.3, 7.1, 0.5 Hz, 2H), 6.93 (br.d, J = 2.9 Hz, 2H), 6.90 (dt, J = 7.1, 1.0 Hz, 2H), 2.96 (s, 8H), 0.66 (s, 6H). ¹³C NMR (101 MHz, CDCl₃) : δ 150.3, 141.1, 128.7, 122.2, 117.9, 113.1, 40.8, -2.7. ESI-MS, positive mode: *m*/*z* (rel. int., %) = 345.2 (100) [M+H]⁺. HR-MS (ESI, positive mode): 345.1387 [M+H]⁺ (found), 345.1384 (calculated for C₁₈H₂₇N₂Ge, [M+H]⁺).

**Compound 16.** *N*-Bromosuccinimide (NBS; 1.69 g, 9.51 mmol, 2.1 eq) was added portionwise to a solution of 15 (2.61 g, 4.34 mmol) in acetonitrile (25 mL), cooled in ice-water bath. Yellow color appeared first, followed by precipitation. The addition of NBS was terminated when yellow color of the suspension abruptly turned brownish, and the mixture was left stirring in ice-water bath for 1 h. TLC control (SiO₂ / CH₂Cl₂): R*_{f}* (product) = 0.65, R*_{f}* (starting material) = 0.55. Sat. aqueous NaHCO₃ (40 mL) was added, the mixture was extracted with CH₂Cl₂ (3 × 40 mL), the combined extracts were washed with brine and dried over Na₂SO₄. The product **16** was isolated by flash column chromatography (Biotage SNAP Ultra 50 g, gradient 10% to 100% CH₂Cl₂ - hexane over 15 CV) as yellowish solid, yield 2.00 g (88%). ¹H NMR (400 MHz, CDCl₃) : δ 7.36 (d, J = 8.7 Hz, 2H), 6.75 (d, J = 3.2 Hz, 2H), 6.58 (dd, J = 8.8, 3.2 Hz, 2H), 2.87 (s, 12H), 0.87 (s, 6H). ¹³C NMR (101 MHz, CDCl₃) : δ 149.1, 141.5, 132.8, 120.9, 116.8, 115.0, 40.7, -0.1. ESI-MS, positive mode: *m*/*z* (rel. int., %) = 501.0 (100) [M+H]⁺. HR-MS (ESI, positive mode): 500.9590 [M+H]⁺ (found), 500.9581 (calculated for C₁₈H₂₅N₂GeBr₂, [M+H]⁺).

**Compound 17.** A solution of **16** (500 mg, 0.994 mmol) in anhydrous THF (20 mL) was degassed on a Schlenk line and cooled to -78 °C under argon. *tert*-Butyllithium (2.4 mL of 1.7 M solution in pentane, 3.98 mmol, 4 eq) was added dropwise, and the resulting bright yellow solution was stirred at -78 °C for 1.5 h. Dimethylcarbamyl chloride (100 µL, 1.09 mmol) was then injected dropwise with a Hamilton syringe. The mixture was stirred at -78 °C for 30 min, allowed to warm up to RT and quenched with sat. aq. NH₄Cl (10 mL). TLC control (SiO₂ / ethyl acetate: R*_{f}* (product) = 0.50 (bright yellow), R*_{f}* (impurity) = 0.71 (colorless). The mixture was extracted with ethyl acetate (3 × 20 mL) and the combined organic layers were dried over Na₂SO₄. The product **17** was isolated by flash column chromatography (Sepacore Silica HP 12 g; gradient 10% to 100% ethyl acetate - hexane) as bright yellow solid, quickly turning greenish, yield 264 g (72%). ¹H NMR (400 MHz, CDCl₃) : δ 8.42 (d, J = 9.0 Hz, 2H), 6.80 (dd, J = 9.1, 2.8 Hz, 2H), 6.72 (d, J = 2.8 Hz, 2H), 3.08 (s, 12H), 0.60 (s, 6H). ¹³C NMR (101 MHz, CDCl₃): 5 185.2, 151.5, 143.3, 132.1, 129.7, 114.3, 112.7, 40.1, -1.3. ESI-MS, positive mode: *m*/*z* (rel. int., %) = 371.1 (100) [M+H]⁺. HR-MS (ESI, positive mode): 371.1175 [M+H]⁺ (found), 371.1177 (calculated for C₁₉H₂₅N₂OGe, [M+H]⁺).

**Compound 19.** In a 100 mL round-bottom flask, a degassed solution of compound **18** (Scheme 7; (223 mg, 0.54 mmol, 2 eq) in anhydrous THF (10 mL) was cooled to -78 °C. tert-Butyllithium (0.32 mL of 1.7 M solution in pentane, 0.54 mmol, 2 eq) was added dropwise, and the resulting yellow-orange solution was stirred at -78 °C for 1 h. A solution of ketone **17** (100 mg, 0.27 mmol) in THF (8 mL) was added quickly dropwise, the light orange mixture was allowed to warm up to room temperature (RT) and stirred for 3.5 h. The mixture was then cooled in ice-water bath and acetic acid (1.4 mL) was added. The resulting blue solution was evaporated to a viscous residue, 6 N HCl (16 mL) was added, and the mixture was stirred at 80 °C (bath temperature) overnight. The mixture was adjusted to pH 1-2 with NaHCO₃, extracted with CH₂Cl₂ (4×50 mL), washed with 0.1 HCl (2×50 mL), brine and dried over Na₂SO₄. The combined organic layers were washed with brine and dried over Na₂SO₄. The product was isolated by flash column chromatography (Sepacore Silica HP 12 g; gradient 0% to 10% methanol - CH₂Cl₂) and freeze-dried from dioxane to give 19 as blue fluffy solid (47 mg, 34% yield). UV-Vis (PBS 7.4): λₘₐₓ (*ε*) = 634 nm (97000 M⁻¹cm⁻¹); fluorescence (PBS 7.4) : λ_{excit} = 590 nm, λₑₘ = 655 nm; *Φ*_{fl} (relative to Oxazine 1, *Φ*_{fl} = 0.14 in ethanol) = 0.43. ¹H NMR (400 MHz, CDCl₃): δ 8.29 (dd, J = 7.9, 1.3 Hz, 1H), 8.23 (dd, *J* = 1.3, 0.8 Hz, 1H), 8.05 (dd, *J* = 8.0, 0.7 Hz, 1H), 6.98 (d, J = 2.9 Hz, 2H), 6.86 (d, J = 8.9 Hz, 2H), 6.52 (dd, J = 8.9, 2.9 Hz, 2H), 2.96 (s, 12H), 0.81 (s, 3H), 0.80 (s, 3H). ¹³C NMR (101 MHz, CDCl₃): δ 170.4, 169.5, 153.4, 149.6, 140.4, 134.1, 131.6, 131.2, 130.7, 127.5, 127.1, 126.3, 117.5, 112.7, 40.5, 0.8, -2.2. ESI-MS, positive mode: *m*/*z* (rel. int., %) = 519.1 (100) [M+H]⁺. HR-MS (ESI, positive mode): 519.1330 [M+H]⁺ (found), 519.1339 (calculated for C₂₇H₂₈N₂O₄Ge, [M+H]⁺).

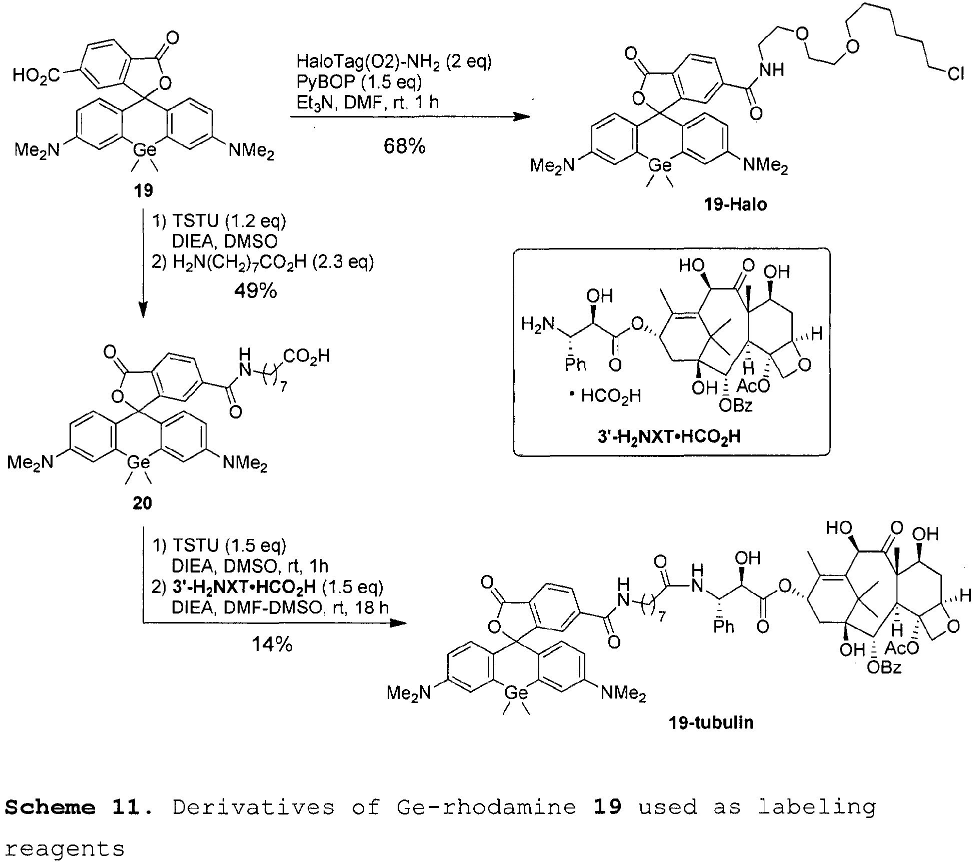

**Compound 19-Halo.** PyBOP (30 µL of 20 mg/100 µL DMF stock solution, 11.6 µmol, 1.5 eq) was added to a solution of 19 (4 mg, 7.74 µmol), HaloTag Amine (02) (2-(2-((6-chlorohexyl)oxy)ethoxy)ethanamine; 3.5 mg, 15.63 µmol, 2 eq) and trimethylamine (12 µL) in DMF (100 µL). After 1 h, the solvent was evaporated *in vacuo* at RT, and the product was isolated by preparative TLC (silica, 3% methanol - CH₂Cl₂, then CH₂Cl₂) giving an impure material, which was repurified by preparative HPLC (Kinetex 5µm C18 100, 21mm×25cm, 11 mL/min, isocratic 65/35 A/B, A - acetonitrile, B - water + 0.05% TFA). Yield 3.8 mg (68%), purity (HPLC) ∼95%. HPLC (30/70-100/0 A/B over 20 min, Kinetex 5µm C18 100 4.6×250 mm, 1.2 mL/min, detection at 254 nm or 636 nm): τ = 12.50 min. ¹H NMR (400 MHz, CDCl₃) : δ 7.99 (dd, J = 7.9, 0.7 Hz, 1H), 7.93 (dd, *J* = 7.9, 1.4 Hz, 1H), 7.91 (dd, J = 1.4, 0.8 Hz, 1H), 6.96 (d, J = 2.9 Hz, 2H), 6.84 (d, J = 8.9 Hz, 2H), 6.78 (br.s, 1H), 6.50 (dd, *J* = 8.9, 2.9 Hz, 2H), 3.71 - 3.63 (m, 6H), 3.59 - 3.55 (m, 2H), 3.49 (t, J = 6.7 Hz, 2H), 3.41 (t, J = 6.7 Hz, 2H), 2.96 (s, 12H), 1.71 (m, 2H + H₂O), 1.57 - 1.25 (m, 6H), 0.79 (s, 3H), 0.79 (s, 3H). ESI-MS, positive mode: *m*/*z* (rel. int., %) = 724.3 [M+H]⁺. HR-MS (ESI, positive mode): 724.2572 (found), 724.2571 (calculated for C₃₇H₄₉ClGeN₃O₅, [M+H]⁺).

**Compound 20.** TSTU (N,N,N',N'-tetramethyl-O-(N-succinimidyl)-uronium tetrafluoroborate; 20 µL of 14 mg/100 µL DMSO stock solution, 9.1 µmol, 1.2 eq) was added to a solution of 19 (4 mg, 7.74 µmol) and DIEA (N-ethyldiisopropylamine; 12 µL) in DMSO (200 µL). After stirring for 5 min, 8-aminooctanoic acid (20 µL of 15 mg/100 µL DMSO stock suspension, prepared by sonication; 18.2 µmol, 2.3 eq) was added, the resulting suspension was sonicated at RT for 10 min followed by vigorous stirring for 15 min. Water (50 µL) was then added, and the mixture was stirred for further 30 min. Acetic acid (50 µL) was added, and the solvents were evaporated *in vacuo* at RT. The product **20** was isolated by preparative HPLC (Kinetex 5µm C18 100, 21mm×25cm, 11 mL/min, isocratic 50/50 A/B, A - acetonitrile, B - water + 0.05% TFA). Yield 2.5 mg (49%). HPLC (30/70-100/0 A/B over 20 min, Kinetex 5µm C18 100 4.6×250 mm, 1.2 mL/min, detection at 254 nm or 636 nm): τ = 8.60 min. ¹H NMR (400 MHz, DMSO-*d*₆) : δ 8.75 (t, J = 5.6 Hz, 1H), 8.11 (dd, J = 8.0, 1.4 Hz, 1H), 8.03 (br.d, J = 8.2 Hz, 1H), 7.86 (br.s, 1H), 7.05 (br.s, 2H), 6.71 (br.d, J = 9.0 Hz, 2H), 6.61 (br.d, J = 8.1 Hz, 2H), 3.24 (q, J = 6.7 Hz, 2H), 2.94 (br.s, 12H), 2.17 (t, J = 7.4 Hz, 2H), 1.49 (m, 4H), 1.27 (m, 6H), 0.77 (s, 3H), 0.68 (s, 3H). ESI-MS, positive mode: *m*/*z* (rel. int., %) = 660.4 (100) [M+H]⁺.

**Compound 19-tubulin.** TSTU (*N,N,N',N'*-tetramethyl-*O*-(*N-*succinimidyl)uronium tetrafluoroborate; 10 µL of 17 mg/100 µL DMF stock solution, 5.7 µmol, 1.5 eq) was added to a solution of **20** (2.5 mg, 3.8 µmol) and DIEA (*N*-ethyldiisopropylamine; 10 µL) in DMSO (200 µL), and the reaction mixture was stirred for 1 h. DIEA (20 µL) followed by **3'-H₂NXT·HCO₂H** ("3'-aminodocetaxel formate" (G. Lukinavičius et al., Nat. Methods 2014, 11, 731-733); 4.3 mg, 5.7 µmol, 1.5 eq, dissolved in 100 µL DMF + 100 µL DMSO) were added, and the reaction mixture was left stirring at RT overnight. The solvents were evaporated *in vacuo* at RT, and the product was isolated by preparative HPLC (Kinetex 5µm C18 100, 10mm×25cm, gradient 30/70-80/20 A/B over 20 min; A - acetonitrile, B - water + 0.05% TFA). Yield 0.73 mg (14%), determined spectrophotometrically (G. Lukinavičius et al., Nat. Methods 2014, 11, 731-733). HPLC (20/80-80/20 A/B over 20 min, Kinetex 5µm C18 100 4.6x250 mm, 1.2 mL/min, detection at 650 nm): τ = 14.90 min. ESI-MS, positive mode: *m*/*z* (rel. int., %) = 1371.5 [M+Na]⁺. HR-MS (ESI, positive mode): 1371.5111 (found), 1371.5165 (calculated for C₇₃H₈₆N₄O₁₆GeNa, [M+Na]⁺).

### Hydroxylated Ge-rhodamine (33)

**Compound 21.** A solution of 3-bromophenol (8.1 g, 46.8 mmol) and imidazole (3.5 g, 51.5 mmol, 1.1 eq) in CH₂Cl₂ (55 mL) was cooled in ice-water bath. To the cold mixture, a solution of triisopropylsilyl chloride (TIPSCl; 10.4 mL, 49.2 mmol, 1.05 eq) in CH₂Cl₂ (40 mL) was added dropwise over ∼20 min. The resulting suspension was removed from ice-water bath and stirred at RT overnight. TLC control (SiO₂ / 10% EtOAc in hexane): R*_{f}* (product) = 0.70, R*_{f}* (3-bromophenol) = 0.18. The mixture was diluted with water (150 mL), extracted with CH₂Cl₂ (3 × 50 mL), the combined organic layers were washed with brine and dried over Na₂SO₄. The product **21** was isolated by flash chromatography on a 1 L glass Büchner funnel with ∼200 g silica (3.5 cm layer), eluting with 0% → 5% → 10% CH₂Cl₂ - hexane in 400 mL portions. Colorless oil, yield 15.07 g (98%). ¹H NMR (400 MHz, CDCl₃): δ 7.08 - 7.06 (m, 2H), 7.05 - 7.03 (m, 1H), 6.84 - 6.77 (m, 1H), 1.32 - 1.18 (m, 3H), 1.10 (d, J = 7.2 Hz, 18H).

**Compound 22.** A solution of TIPS-protected bromophenol 21 (3.54 g, 10.76 mmol, 2 eq) in anhydrous THF (40 mL) was degassed on a Schlenk line and cooled to -78 °C under argon. *n-*Butyllithium (4.7 mL of 2.5 M solution in hexanes, 11.84 mmol, 2.2 eq) was injected with a syringe quickly dropwise, and the mixture was stirred at -78 °C for 1 h, turning into a thin suspension. Dimethylgermanium dichloride (0.62 mL, 5.38 mmol), dissolved in anhydrous THF (2.5 mL), was injected dropwise with a syringe. The mixture was allowed to warm up to RT (the solids dissolved), and the resulting solution was stirred at RT for 4.5 h. Brine (40 mL) was then added, the mixture was extracted with ethyl acetate (3 × 40 mL), and the combined organic layers were dried over Na₂SO₄. TLC control (SiO₂ / 10% CH₂Cl₂ in hexane): R*_{f}* (product) = 0.34, R*_{f}* (impurity) = 0.50. The product **22** was isolated by flash column chromatography (Teledyne Isco RediSep Rf 80 g, isocratic in pure hexane for 5 CV, then gradient 0% to 20% CH₂Cl₂ - hexane over 8 CV) as colorless oil, yield 2.79 g (86%). ¹H NMR (400 MHz, CDCl₃) : δ 7.23 - 7.17 (m, 2H), 7.01 (dt, J = 7.1, 1.1 Hz, 2H), 6.98 - 6.96 (m, 2H), 6.85 (ddd, *J* = 8.1, 2.6, 1.1 Hz, 2H), 1.27 - 1.16 (m, 6H), 1.08 (d, J = 7.2 Hz, 36H), 0.60 (s, 6H). ¹³C NMR (101 MHz, CDCl₃): δ 155.9, 141.6, 129.1, 126.2, 125.0, 120.2, 18.1, 12.8, -3.0. ESI-MS, positive mode: *m*/*z* (rel. int., %) = 625.3 (100) [M+Na]⁺. HR-MS (ESI, positive mode): 625.2897 [M+Na]⁺ (found), 625.2930 (calculated for C₃₂H₅₆O₆Si₂GeNa, [M+Na]⁺).

**Compound 23.** N-Bromosuccinimide (1.62 g, 9.11 mmol, 2.1 eq) was added portionwise over 5 min to a solution of 22 (2.61 g, 4.34 mmol) in the mixture of acetonitrile (60 mL) and pyridine (8.5 mL). The resulting mixture was stirred at 60 °C (bath temperature) overnight (22 h), turning into a brown solution. TLC control (SiO₂ / 5% CH₂Cl₂ in hexane): R*_{f}* (product) = 0.27, R*_{f}* (starting material) = 0.19. The solvents were removed on a rotary evaporator; the residue was redissolved in CH₂Cl₂ (120 mL) and the solution was washed with sat. aqueous NaHCO₃, water, brine (100 mL each) and dried over Na₂SO₄. The product **23** was isolated by flash column chromatography (Teledyne Isco RediSep Rf 80 g, gradient 0% to 25% CH₂Cl₂ - hexane over 15 CV) as viscous colorless oil with purity ∼85%, yield 2.64 g (70% considering purity). The material was used in the next step without further purification. ¹H NMR (400 MHz, CDCl₃): δ 7.35 (d, J = 8.6 Hz, 2H), 6.85 (d, J = 3.0 Hz, 2H), 6.74 (dd, J = 8.6, 3.0 Hz, 2H), 1.23 - 1.12 (m, 6H), 1.05 (d, J = 7.0 Hz, 36H), 0.84 (s, 6H). ¹³C NMR (101 MHz, CDCl₃): δ 155.1, 142.4, 133.5, 127.5, 122.5, 120.9, 18.0, 12.8, -0.3. ESI-MS, positive mode: *m*/*z* (rel. int., %) = 783.1 (100) [M+Na]⁺. HR-MS (ESI, positive mode): 783.1103 [M+Na]⁺ (found), 783.1119 (calculated for C₃₂H₅₄O₂Si₂GeBr₂Na, [M+Na]⁺).

**Compound 24.** A solution of **23** (2.9 g, 3.82 mmol) in anhydrous THF (60 mL) was degassed on a Schlenk line and cooled to -78 °C under argon. *n*-Butyllithium (3.2 mL of 2.5 M solution in hexanes, 8.02 mmol, 2.1 eq) was added dropwise, and the resulting light yellow solution was stirred at -78 °C for 5.5 h. Dimethylcarbamyl chloride (0.36 mL, 3.82 mmol) was then injected dropwise with a syringe. The mixture was stirred at - 78 °C for 30 min, turning nearly colorless, and was then allowed to warm up to RT and stirred for further 1.5 h. TLC control (SiO₂ / 10% ethyl acetate in hexane): R*_{f}* (product) = 0.62 (yellow upon heating with NaOH), R*_{f}* (starting material) = 0.72. The reaction mixture was quenched with sat. aq. NH₄Cl (30 mL), extracted with ethyl acetate (3 × 30 mL), and the combined organic layers were dried over Na₂SO₄ The product was isolated by flash column chromatography (Teledyne Isco RediSep Rf 80 g, gradient 0% to 100% A:B, A = 10% ethyl acetate - hexane, B = hexane) as light yellow viscous oil with purity ∼70 %, yield 2.13 g (62% considering purity). The impure material was used directly for deprotection. ¹H NMR (400 MHz, CDCl₃): δ 8.37 (d, J = 8.7 Hz, 2H), 7. 02 (d, J = 2. 6 Hz, 2H), 6.98 (dd, J = 8.7, 2.6 Hz, 2H), 1.36 - 1.21 (m, 6H), 1.13 (d, *J* = 7.3 Hz, 36H), 0.59 (s, 6H). ESI-MS, positive mode: *m*/*z* (rel. int., %) = 629.3 (100) [M+H]⁺. HR-MS (ESI, positive mode): 629.2898 [M+H]⁺ (found), 629.2903 (calculated for C₃₃H₅₅GeO₃Si₂, [M+H]⁺).

Tetrabutylammonium fluoride trihydrate (2.68 g, 8.5 mmol, 2.5 equiv), dissolved in THF (20 mL), was added a solution of crude di-TIPS ether from the previous step (2.13 g, ∼70% purity) in THF (25 mL), cooled in ice-water bath. The resulting bright yellow solution was stirred at 0 °C for 1 h. TLC control (SiO₂ / 50% ethyl acetate in hexane): R*_{f}* (product) = 0.26 (yellow with NaOH), R*_{f}* (impurity) = 0.48, R*_{f}* (starting material) = 0.76. Sat. aq. NH₄Cl (50 mL) was added followed by minimal amount of water necessary to dissolve the solids, the mixture was extracted with ethyl acetate (3×40 mL), the combined organic layers were washed with brine and dried over Na₂SO₄. The product was isolated by flash column chromatography (Teledyne Isco RediSep Rf 80 g; gradient 20% to 80% ethyl acetate - hexane) to give pure **24** as white solid. Yield 475 mg (39% over 2 steps). ¹H NMR (400 MHz, acetone-*d*₆): δ 9.09 (s, 2H), 8.31 (d, J = 8.7 Hz, 2H), 7.12 (d, *J* = 2.6 Hz, 2H), 7.00 (dd, *J* = 8.8, 2.6 Hz, 2H), 0.60 (s, 6H). ¹³C NMR (101 MHz, acetone-*d*₆): δ 161.1, 144.7, 134.1, 133.3, 119.9, 117.6, -1.9. ESI-MS, positive mode: *m*/*z* (rel. int., %) = 339.0 (100) [M+Na]⁺. HR-MS (ESI, positive mode): 339.0052 [M+Na]⁺ (found), 339.0050 (calculated for C₁₅H₁₄GeO₃Na, [M+Na]⁺).

**Compound 25.** A solution of **24** (475 mg, 1.51 mmol) and imidazole (308 mg, 4.53 mmol, 3 eq) in CH₂Cl₂ (30 mL) and DMF (3 mL) was cooled in ice-water bath. To the cold mixture, a solution of tert-butyldimethylsilyl chloride (TBSCl; 684 mg, 4.53 mmol, 3 eq) in CH₂Cl₂ (5 mL) was added quickly dropwise. The resulting white suspension was removed from ice-water bath and stirred at RT for 4 h. TLC control (SiO₂ / 10% EtOAc in hexane): R*_{f}* (product) = 0.56, R*_{f}* (starting material) = 0. The mixture was diluted with water (300 mL), extracted with CH₂Cl₂ (3 × 50 mL), the combined organic layers were washed with water (200 mL), sat. aq. NaHCO₃, brine (100 mL each) and dried over Na₂SO₄. The product **25** was isolated by flash column chromatography (Teledyne Isco RediSep Rf 40 g; gradient 0% to 100% A:B, A = 10% ethyl acetate - hexane, B = hexane). White solid, yield 817 g (100%). ¹H NMR (400 MHz, CDCl₃) : δ 8.37 (d, J = 8.7 Hz, 2H), 6.98 (d, J = 2.6 Hz, 2H), 6.95 (dd, J = 8.7, 2.6 Hz, 2H), 1.01 (s, 18H), 0.60 (s, 6H), 0.26 (s, 12H). ¹³C NMR (101 MHz, CDCl₃): δ 186.2, 158.8, 143.8, 134.8, 132.7, 123.9, 121.3, 25.8, 18.5, -1.7, -4.2. ESI-MS, positive mode: *m*/*z* (rel. int., %) = 545.2 (100) [M+H]⁺. HR-MS (ESI, positive mode): 545.1958 [M+H]⁺ (found), 545.1962 (calculated for C₂₇H₄₃O₃Si₂Ge, [M+H]⁺).

**Compound 27.** In a 100 mL round-bottom flask, a degassed solution of compound **26** (Scheme 9; 1.04 g, 2.9 mmol, 2 eq) in anhydrous THF (10 mL) and pentane (5 mL) was cooled to -100 °C (bath temperature, diethyl ether - liquid N₂). *n*-Butyllithium (1.2 mL of 2.5 M solution in hexanes, 2.9 mmol, 2 eq) was carefully introduced through a needle along the wall of the flask. Clear solution quickly turned orange and then brown-orange; it was stirred at -100 °C for 10 min, and the solution of ketone **25** (787 mg, 1.45 mmol) in THF (8 mL) was injected over 1-2 min along the wall of the flask. The flask was then placed into a -78° bath (dry ice - acetone) and the brown solution was stirred for 10 min. The cooling bath was removed, the mixture was allowed to warm up to RT and stirred for further 30 min. TLC control (SiO₂ / 10% ethyl acetate in hexane): R*_{f}* (product) = 0.35 (purple upon heating with NaOH), R*_{f}* (starting material) = 0.47. The reaction mixture was quenched with water (50 mL), adjusted to pH ∼ 4 with acetic acid, extracted with ethyl acetate (3×30 mL), the combined organic layers were washed with brine and dried over Na₂SO₄. The product was isolated by flash column chromatography (Biotage SNAP Ultra 100 g; gradient 0% to 100% A:B, A = 15% ethyl acetate - hexane, B = hexane) and freeze-dried from dioxane to give **27** as white solid (501 mg, 46% yield). ¹H NMR (400 MHz, CDCl₃): δ 8.18 (dd, *J* = 8.0, 1.3 Hz, 1H), 8.08 (dd, J = 1.3, 0.7 Hz, 1H), 7.99 (dd, *J* = 8.0, 0.7 Hz, 1H), 7.10 (d, J = 2.7 Hz, 2H), 6.96 (d, J = 8.7 Hz, 2H), 6.65 (dd, J = 8.7, 2.7 Hz, 2H), 1.60 (s, 9H), 0.98 (s, 18H), 0.795 (s, 3H), 0.790 (s, 3H), 0.19 (s, 12H). ¹³C NMR (101 MHz, CDCl₃): δ 169.4, 164.5, 155.5, 153.0, 140.6, 137.0, 136.2, 130.4, 129.7, 127.8, 126.2, 125.9, 125.5, 120.2, 91.2, 82.6, 28.3, 25.8, 18.4, 0.5, -1.9, -4.21, -4.23. ESI-MS, positive mode: *m*/*z* (rel. int., %) = 749.3 (100) [M+H]⁺. HR-MS (ESI, positive mode): 749.2732 [M+H]⁺ (found), 749.2753 (calculated for C₃₉H₅₅O₆GeSi₂, [M+H]⁺).

**Compound 28.** Tetrabutylammonium fluoride trihydrate (850 mg, 2.68 mmol, 4 eq), dissolved in THF (5 mL), was added a solution of **27** (500 mg, 0.67 mmol) in THF (7 mL), cooled in ice-water bath. The resulting blue solution with intense red fluorescence was stirred at 0 °C for 30 min. TLC control (SiO₂ / 10% ethyl acetate in CH₂Cl₂): R*_{f}* (product) = 0.31, R*_{f}* (starting material) = 0.83. Sat. aq. NH₄Cl (20 mL) was added followed by the minimal amount of water necessary to dissolve the solids, the mixture was extracted with ethyl acetate (3×25 mL), the combined organic layers were washed with brine and dried over Na₂SO₄. The product was isolated by flash column chromatography (Teledyne Isco RediSep Rf 24 g; gradient 0% to 30% ethyl acetate - CH₂Cl₂), evaporated to viscous colorless oil and freeze-dried from dioxane to give **28** as white solid (393 mg, contains 0.65 mol dioxane per mol of product, 100% yield). ¹H NMR (400 MHz, CDCl₃): δ 8.18 (dd, J = 8.0, 1.3 Hz, 1H), 8.03 (dd, J = 1.3, 0.8 Hz, 1H), 7.99 (dd, J = 8.0, 0.8 Hz, 1H), 7.08 (d, J = 2.8 Hz, 2H), 6.86 (d, J = 8.7 Hz, 2H), 6.60 (dd, J = 8.7, 2.8 Hz, 2H), 6.40 (d, J = 4.0 Hz, 2H), 1.59 (s, 9H), 0.67 (s, 3H), 0.63 (s, 3H). ¹³C NMR (101 MHz, CDCl₃): δ 170.6, 164.7, 155.7, 153.4, 140.8, 137.2, 134.9, 130.5, 129.4, 127.9, 126.3, 125.8, 120.9, 115.9, 92.1, 83.1, 28.3, 0.3, -1.9. ESI-MS, positive mode: *m*/*z* (rel. int., %) = 543.1 (100) [M+Na]⁺. HR-MS (ESI, positive mode): 543.0838 [M+Na]⁺ (found), 543.0839 (calculated for C₂₇H₂₆GeO₆Na, [M+Na]⁺).

**Compound 29.** Trifluoromethanesulfonic anhydride (Tf₂O; 155 µL, 0.92 mmol, 4 eq) was added dropwise to a solution of 28 (133 mg of material containing 0.65 mol dioxane per mol of 28, 0.23 mmol) and pyridine (150 µL, 1.84 mmol, 8 eq) in dry CH₂Cl₂ (5 mL), cooled in ice-water bath. Pink color appeared upon addition of each drop and vanished immediately. The flask was then removed from the cooling bath, and the mixture was stirred at RT for 1 h. TLC control (SiO₂ / 10% ethyl acetate in hexane): R*_{f}* (product) = 0.18 (pink with NaOH). The mixture was diluted with cold water (20 mL), extracted with CH₂Cl₂ (3×20 mL), the combined extracts were washed with brine and dried over Na₂SO₄. The product was isolated by flash column chromatography (Sepacore Silica HP 12 g; gradient 0% to 40% ethyl acetate - hexane) and freeze-dried from dioxane to give **29** as white fluffy solid, yield 173 mg (96%). ¹H NMR (400 MHz, CDCl₃): δ 8.26 (dd, *J* = 8.0, 1.2 Hz, 1H), 8.16 (dd, *J* = 1.3, 0.7 Hz, 1H), 8.05 (dd, *J* = 8.0, 0.8 Hz, 1H), 7.54 (d, *J* = 2.7 Hz, 2H), 7.31 (d, *J* = 8.8 Hz, 2H), 7.16 (dd, *J* = 8.8, 2.7 Hz, 2H), 1.62 (d, *J* = 0.8 Hz, 9H), 0.93 (s, 3H), 0.92 (s, 3H). ¹⁹F NMR (376 MHz, CDCl₃) : δ -72.78. ¹³C NMR (101 MHz, CDCl₃): δ 168.1, 164.0, 150.8, 149.5, 143.3, 142.5, 137.7, 131.4, 129.2, 128.4, 127.0, 126.7, 125.7, 122.1, 118.8 (q, *J* = 320.8 Hz), 89.2, 83.2, 28.3, 0.9, -1.7. ESI-MS, positive mode: *m*/*z* (rel. int., %) = 807.0 (100) [M+Na]⁺. HR-MS (ESI, positive mode): 806.9805 [M+Na]⁺ (found), 806.9824 (calculated for C₂₉H₂₄O₁₀GeS₂F₆Na, [M+Na]⁺).

**Compound 31.** A mixture of **29** (170 mg, 0.22 mmol), 3-(tert-butyldimethylsilyloxy)azetidine **30** (123 mg, 0.66 mmol, 3 eq), Pd₂(dba)₃ (20 mg, 0.022 mmol, 10 mol%), XPhos (31 mg, 0.066 mmol, 30 mol%) and K₂CO₃ (91 mg, 0.66 mmol, 3 eq) in dry dioxane (2 mL) was degassed on a Schlenk line and stirred at 100 °C under argon (bath temperature) in a sealed flask for 2 h. TLC control showed incomplete conversion (SiO₂ / hexane - CH₂Cl₂ - ethyl acetate 70:25:5, stained by heating with 1 M NaOH): R*_{f}* (product) = 0.2 (blue), R*_{f}* (monosubstitution product) = 0.33 (violet), R*_{f}* (starting material) = 0.38 (pink). Additional portions of Pd₂(dba)₃ (20 mg, 0.022 mmol, 10 mol%) and XPhos (31 mg, 0.066 mmol, 30 mol%) were added, the mixture was degassed stirred at 100 °C for further 2 h (4 h total time). The consumption of both the starting material and the monotriflate intermediate was verified by TLC, and upon cooling the brown mixture was diluted with water (20 mL), extracted with CH₂Cl₂ (3×20 mL), the combined organic layers were washed with brine and dried over Na₂SO₄. The filtrate was evaporated on Celite and the product was isolated by flash column chromatography (Interchim Puriflash 15 µm 25 g; isocratic in pure CH₂Cl₂ until the first peak is eluted (6 CV), then gradient 0% to 100% A:B, A = 10% ethyl acetate in CH₂Cl₂, B = CH₂Cl₂) and freeze-dried from 1,4-dioxane to yield 84 mg (45%) of **31** as light yellow fluffy solid. ¹H NMR (400 MHz, CDCl₃): δ 8.16 (dd, *J* = 8.0, 1.3 Hz, 1H), 8.03 (t, *J* = 1.0 Hz, 1H), 7.97 (dd, *J* = 8.0, 0.7 Hz, 1H), 6.88 (d, *J* = 8.6 Hz, 2H), 6.67 (d, *J* = 2.6 Hz, 2H), 6.26 (dd, J = 8.7, 2.6 Hz, 2H), 4.74 (tt, *J* = 6.3, 5.0 Hz, 2H), 4.15 (td, *J* = 6.5, 1.3 Hz, 4H), 3.65 (dd, *J* = 7.6, 5.0 Hz, 4H), 1.59 (s, 9H), 0.89 (s, 18H), 0.78 (s, 3H), 0.78 (s, 3H), 0.07 (s, 12H). ¹³C NMR (101 MHz, CDCl₃): δ 169.7, 164.6, 153.6, 150.9, 139.8, 136.8, 132.3, 130.1, 130.0, 127.2, 125.94, 125.92, 116.6, 112.0, 92.2, 82.4, 62.5, 62.2, 62.1, 28.3, 25.9, 18.1, 0.6, -1.8, -4.8. ESI-MS, positive mode: *m*/*z* (rel. int., %) = 859.4 (100) [M+H]⁺. HR-MS (ESI, positive mode): 859.3586 [M+H]⁺ (found), 859.3599 (calculated for C₄₅H₆₅N₂O₆Si₂Ge, [M+H]⁺).

**Compound 32.** Tetrabutylammonium fluoride trihydrate (93 mg, 294 µmol, 3 equiv), dissolved in THF (5 mL), was added a solution of **31** (84 mg, 98 µmol) in THF (10 mL), cooled in ice-water bath. The reaction mixture was stirred at 0 °C for 1 h. Water (20 mL), acetic acid (0.5 mL) and brine (20 mL) were then added, and the mixture was extracted with ethyl acetate (3×20 mL), the combined organic layers were dried over Na₂SO₄, filtered, evaporated and re-evaporated twice with ethyl acetate (2×20 mL). The product was isolated by flash column chromatography (Sepacore Silica HP 12 g; gradient 20% to 100% ethyl acetate - CH₂Cl₂) and freeze-dried from 1,4-dioxane to give **32** as light green fluffy solid. Yield 60 mg (97%). ¹H NMR (400 MHz, CD₃OD): 5 8.16 (dd, J= 8.1, 1.3 Hz, 1H), 7.96 (dd, J = 8.0, 0.7 Hz, 1H), 7.90 (t, J = 1.0 Hz, 1H), 6.79 (d, J = 8.7 Hz, 2H), 6.73 (d, *J* = 2.6 Hz, 2H), 6.28 (ddd, *J* = 8.7, 2.7, 0.9 Hz, 2H), 4.85 (s, 2H + H₂O), 4.63 (tt, *J* = 6.4, 4.8 Hz, 2H), 4.16 - 4.07 (m, 4H), 3.67 - 3.56 (m, 4H), 1.55 (s, 9H), 0.76 (s, 3H), 0.70 (s, 3H). ¹³C NMR (101 MHz, CD₃OD): δ 171.5, 165.6, 155.7, 152.5, 140.3, 138.3, 133.0, 131.1, 130.5, 128.1, 126.9, 126.5, 117.6, 113.4, 83.6, 63.0, 62.5, 28.3, 0.1, -1.7. ESI-MS, positive mode: *m*/*z* (rel. int., %) = 631.2 (100) [M+H]⁺. HR-MS (ESI, positive mode): 631.1851 [M+H]⁺ (found), 631.1865 (calculated for C₃₃H₃₇N₂O₆Ge, [M+H]⁺).

**Compound 33.** Trifluoroacetic acid (1.6 mL) was added to a solution of **32** (50 mg, 79.5 µmol) in CH₂Cl₂ (8 mL), cooled in ice-water bath. The cooling bath was removed, and the reaction mixture was stirred at RT for 3 h. The solvents were evaporated, and the residue was re-evaporated twice with toluene (2×20 mL). The product was isolated by flash column chromatography (20 g silica, acetonitrile - CH₂Cl₂ - water 10:1:0.5, then 10:1:1), followed by chromatography on a reversed phase (RP-C₁₈ 10 g; gradient 67% to 33% water - acetonitrile). Fractions containing the product were evaporated, dissolved in 1,4-dioxane with addition of minimal amount of water, a small amount of viscous insoluble material was centrifuged off, the supernatant was microfiltered and freeze-dried to give 51 mg (79%) blue fluffy solid (trifluoroacetate salt, containing 1.4 mol of 1,4-dioxane per mol of the dye). HPLC (10/90-100/0 over 25 min, column 4×250 mm, 1.2 mL/min, detection at 254 nm): τ = 10.32 min. UV-Vis (PBS 7.4): λₘₐₓ (*ε*) = 631 nm (61000 M⁻¹cm⁻¹); fluorescence (PBS 7.4): λ_{excit} = 590 nm, λₑₘ = 651 nm; *Φ*_{fl} (abs.) = 0.60. ¹H NMR (400 MHz, CD₃OD): δ 8.22 (br.d, *J* = 7.0 Hz, 1H), 8.07 (br.s, 1H), 7.92 (d, *J* = 7.0 Hz, 1H), 6.78 (d, *J* = 8.6 Hz, 2H), 6.73 (d, *J* = 2.5 Hz, 2H), 6.25 (dd, *J* = 8.6, 2.6 Hz, 2H), 4.63 (tt, *J* = 6.2, 4.8 Hz, 2H), 4.16 - 4.08 (m, 4H), 3.62 - 3.57 (m, 4H), 0.74 (s, 3H), 0.71 (s, 3H). ¹⁹F NMR (376 MHz, CD₃OD): δ -76.95. ¹³C NMR (101 MHz, CD₃OD): δ 171.7, 154.1, 152.5, 141.4, 133.4, 130.1, 128.3, 126.4, 117.7, 112.9, 94.8, 63.0, 62.5, 0.5, - 2.6. ESI-MS, positive mode: *m*/*z* (rel. int., %) = 575.2 (100) [M+H]⁺. HR-MS (ESI, positive mode): 575.1225 (found), 575.1238 (calculated for C₂₉H₂₉N₂O₆Ge, [M+H]⁺).

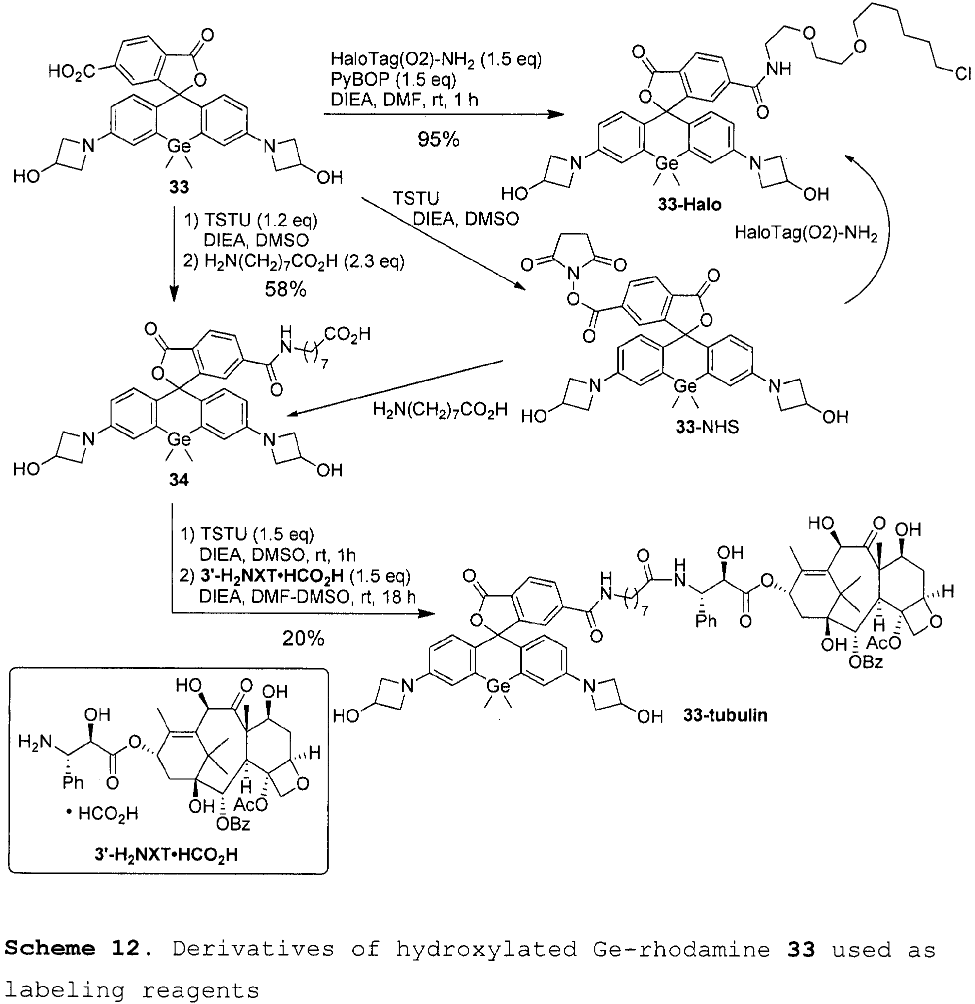

**Compound 33-Halo.** PyBOP (20 µL of 20 mg/100 µL stock solution, 7.41 µmol, 1.5 equiv) was added to a solution of **33** (4 mg, 5.82 µmol), HaloTag Amine (02) (2-(2-((6-chlorohexyl)-oxy)ethoxy)ethanamine; 2.0 mg, 8.73 µmol, 1.5 equiv) and DIEA (N-ethyldiisopropylamine; 10 µL) in DMF (100 µL). After 1 h, the solvent was evaporated *in vacuo* at RT, and the product **33-Halo** was isolated by preparative TLC (silica, 5% methanol - CH₂Cl₂, then 10% methanol - CH₂Cl₂). Yield 4.3 mg (95%), purity (HPLC) ∼95%. HPLC (10/90-100/0 A/B over 25 min, column 4×250 mm, 1.2 mL/min, detection at 254 nm or 640 nm): τ = 14.60 min. UV-Vis (PBS 7.4): λₘₐₓ = 639 nm; fluorescence (PBS 7.4): λ_{excit} = 610 nm, λₑₘ = 660 nm. ESI-MS, positive mode: *m*/*z* (rel. int., %) = 780.3 [M+H]⁺. HR-MS (ESI, positive mode): 780.2471 (found), 780.2470 (calculated for C₃₉H₄₉ClGeN₃O₇, [M+H]⁺).

**Compound 34.** TSTU (*N,N,N',N'*-tetramethyl-*O*-(*N*-succinimidyl) *-* uronium tetrafluoroborate; 20 µL of 18 mg/100 µL DMSO stock solution, 12 µmol, 1.2 eq) was added to a solution of **33** (6 mg, 10 µmol) and DIEA (*N*-ethyldiisopropylamine; 12 µL) in DMSO (250 µL). After stirring for 5 min, 8-aminooctanoic acid (20 µL of 18 mg/100 µL DMSO stock suspension, prepared by sonication; 23 µmol, 2.3 eq) was added, the resulting suspension was sonicated at RT for 10 min followed by vigorous stirring for 15 min. Water (50 µL) was then added, and the mixture was stirred for further 30 min. Acetic acid (50 µL) was added, and the solvents were evaporated *in vacuo* at RT. The product was isolated by preparative HPLC (Kinetex 5µm C18 100, 21mm×25cm, 11 mL/min, gradient 20/75-70/30 A/B over 20 min, A - acetonitrile, B - water + 0.05% TFA). Yield 2.5 mg (49%). HPLC (20/80-70/30 A/B over 20 min, Kinetex 5µm C18 100 4.6x250 mm, 1.2 mL/min, detection at 254 nm): τ = 10.90 min. ¹H NMR (400 MHz, DMSO-*d*₆): δ 8.75 (t, *J* = 5.6 Hz, 1H), 8.11 (dd, J = 8.0, 1.4 Hz, 1H), 8.03 (br.d, *J* = 8.2 Hz, 1H), 7.86 (br.s, 1H), 7.05 (br.s, 2H), 6.71 (br.d, *J* = 9.0 Hz, 2H), 6.61 (br.d, *J* = 8.1 Hz, 2H), 3.24 (q, *J* = 6.7 Hz, 2H), 2.94 (br.s, 12H), 2.17 (t, *J* = 7.4 Hz, 2H), 1.49 (m, 4H), 1.27 (m, 6H), 0.77 (s, 3H), 0.68 (s, 3H). ESI-MS, positive mode: *m*/*z* (rel. int., %) = 716.2 [M+H]⁺. HR-MS (ESI, positive mode): 716.2374 (found), 716.2394 (calculated for C₃₇H₄₄N₃O₇Ge, [M+H]⁺).

**Compound 33-tubulin.** TSTU (*N,N,N',N'*-tetramethyl-*O*-(*N-*succinimidyl)uronium tetrafluoroborate; 13 µL of 20 mg/100 µL DMF stock solution, 8.67 µmol, 1.5 eq) was added to a solution of **34** (5.78 µmol in 500 µL DMSO) and DIEA *(N-*ethyldiisopropylamine; 16 µL), and the reaction mixture was stirred for 1 h. DIEA (30 µL) followed by **3'-H₂NXT·HCO₂H** ("3'-aminodocetaxel formate"; 6.5 mg, 8.7 µmol, 1.5 eq, dissolved in 150 µL DMF) were added, and the reaction mixture was left stirring at RT overnight. The solvents were evaporated *in vacuo* at RT, and the product was isolated by preparative HPLC (Kinetex 5µm C18 100, 10mm×25cm, isocratic 50/50 A/B, A - acetonitrile, B - water + 0.05% TFA). Yield 1.59 mg (20%), determined spectrophotometrically. HPLC (10/90-80/20 A/B over 2 min, column 4×250 mm, 1.2 mL/min, detection at 254 nm): τ = 15.83 min. ESI-MS, positive mode: *m*/*z* (rel. int., %) = 1405.5 (100) [M+H]⁺. HR-MS (ESI, positive mode): 1405.5237 (found), 1405.5244 (calculated for C₇₅H₈₇N₄O₁₈Ge, [M+H]⁺).

### Hydroxylated Si-rhodamine JF₆₄₆ (42)

**Compound 35.** A solution of TIPS-protected bromophenol (**21**; 5.27 g, 16.02 mmol, 2 eq) in anhydrous THF (60 mL) was degassed on a Schlenk line and cooled to -78 °C under argon. *n*-Butyllithium (7.05 mL of 2.5 M solution in hexanes, 17.62 mmol, 2.2 eq) was injected with a syringe quickly dropwise, and the mixture was stirred at -78 °C for 1 h, turning into a thick suspension. Dichlorodimethylsilane (0.97 mL, 8.01 mmol) was added dropwise with a syringe. The mixture was allowed to warm up to RT (the solids dissolved), and the resulting solution was stirred at RT for 2 h. Brine (50 mL) and water (20 mL) were then added, the mixture was extracted with ethyl acetate (3 × 50 mL), and the combined organic layers were dried over Na₂SO₄. TLC control (SiO₂ / 10% CH₂Cl₂ in hexane): R*_{f}* (product) = 0.36, R*_{f}* (starting material) = 0.55. The product **35** was isolated by flash column chromatography (90 g silica, gradient 0% to 30% CH₂Cl₂ - hexane) as colorless oil, yield 4.12 g (93%). ¹H NMR (400 MHz, CDCl₃): δ 7.23 - 7.18 (m, 2H), 7.06 (dt, *J* = 7.2, 1.1 Hz, 2H), 7.02 - 7.00 (m, 2H), 6.88 (ddd, J = 8.1, 2.6, 1.1 Hz, 2H), 1.29 - 1.16 (m, 6H), 1.08 (d, J = 7.1 Hz, 36H), 0.51 (s, 6H). ¹³C NMR (101 MHz, CDCl₃): δ 155.7, 139.8, 129.0, 126.8, 125.6, 120.8, 18.1, 12.8, -2.3. ESI-MS, positive mode: *m*/*z* (rel. int., %) = 595.3 (100) [M+K]⁺. HR-MS (ESI, positive mode): 595.3215 [M+K]⁺ (found), 595.3220 (calculated for C₃₂H₅₆O₂Si₃K, [M+K]⁺).

**Compound 36.** N-Bromosuccinimide (2.33 g, 13.07 mmol, 2.1 eq) was added portionwise over 5 min to a solution of **35** (3.46 g, 6.22 mmol) in the mixture of acetonitrile (85 mL) and chloroform (25 mL). The resulting solution was stirred at 60 °C (bath temperature) overnight (16 h), turning light yellow. TLC control (SiO₂ / 5% CH₂Cl₂ in hexane): R*_{f}* (product) = 0.44, R*_{f}* (starting material) = 0.36. The solvents were removed on a rotary evaporator; the residue was redissolved in CH₂Cl₂ (150 mL) and the solution was washed with sat. aqueous NaHCO₃, water, brine (100 mL each) and dried over Na₂SO₄. The product **36** was isolated by flash column chromatography (100 g silica, gradient 0% to 15% CH₂Cl₂ - hexane) as colorless oil, yield 3.27 g (74%). ¹H NMR (400 MHz, CDCl₃): δ 7.34 (d, J = 8.6 Hz, 2H), 6.97 (d, J = 3.0 Hz, 2H), 6.76 (dd, J = 8.6, 3.0 Hz, 2H), 1.25 - 1.15 (m, 6H), 1.07 (d, J = 7.3 Hz, 36H), 0.72 (s, 6H). ¹³C NMR (101 MHz, CDCl₃): δ 155.0, 140.0, 133.8, 128.5, 123.0, 121.0, 18.0, 12.8, -1.0. ESI-MS, positive mode: *m*/*z* (rel. int., %) = 737.2 (100) [M+Na]⁺. HR-MS (ESI, positive mode): 737.1664 [M+Na]⁺ (found), 737.1673 (calculated for C₃₂H₅₄Br₂O₂Si₃Na, [M+Na]⁺).

**Compound 37.** A solution of **36** (4.06 g, 5.69 mmol) in anhydrous THF (80 mL) was degassed on a Schlenk line and cooled to -78 °C under argon. *n*-Butyllithium (5 mL of 2.5 M solution in hexanes, 12.5 mmol, 2.2 eq) was added dropwise, and the mixture was stirred at -78 °C for 3.5 h, turning into a clear light yellow solution. Dimethylcarbamyl chloride (0.53 mL, 5.69 mmol, 1 eq) was then injected dropwise with a syringe. The mixture was stirred at -78 °C for 30 min, allowed to warm up to RT and stirred for further 30 min. TLC control (SiO₂ / 10% ethyl acetate in hexane): R*_{f}* (product) = 0.59 (yellow upon heating with NaOH), R*_{f}* (starting material) = 0.67. The reaction mixture was quenched with sat. aq. NH₄Cl (30 mL), extracted with ethyl acetate (3 × 40 mL), and the combined organic layers were dried over Na₂SO₄. The product **37** was isolated by flash column chromatography (Teledyne Isco RediSep Rf 80 g, gradient 0% to 100% A:B, A = 10% ethyl acetate - hexane, B = hexane) as light yellow viscous oil, yield 2.46 g (74%). ¹H NMR (400 MHz, CDCl₃): δ 8.36 (d, *J* = 8.7 Hz, 2H), 7.08 (d, *J* = 2.5 Hz, 2H), 7.02 (dd, *J* = 8.7, 2.6 Hz, 2H), 1.36 - 1.25 (m, 6H), 1.13 (d, *J* = 7.3 Hz, 36H), 0.45 (s, 6H). ¹³C NMR (101 MHz, CDCl₃): δ 186.1, 159.3, 141.2, 134.5, 132.4, 123.6, 121.8, 18.1, 12.9, -1.4. ESI-MS, positive mode: *m*/*z* (rel. int., %) = 583.3 [M+H]⁺. HR-MS (ESI, positive mode): 583.3428 [M+H]⁺ (found), 583.3454 (calculated for C₃₃H₅₅O₃Si₃, [M+H]⁺).

**Compound 38.** In a 50 mL round-bottom flask, a degassed solution of **26** (Scheme 9; 427 mg, 1.2 mmol, 2 eq) in anhydrous THF (4 mL) and pentane (2 mL) was cooled to -100 °C (bath temperature, diethyl ether - liquid N₂). *n*-Butyllithium (0.48 mL of 2.5 M solution in hexanes, 1.2 mmol, 2 eq) was carefully introduced through a needle along the wall of the flask. Clear solution quickly turned orange and then purple; it was stirred at -100 °C for 10 min, and the solution of ketone **37** (349 mg, 0.6 mmol) in THF (2 mL) was injected over 1-2 min along the wall of the flask. The flask was then placed into a -78° bath (dry ice - acetone) and the stirring was continued for 10 min. The cooling bath was removed, the mixture was allowed to warm up to RT and stirred for further 30 min. TLC control (SiO₂ / 10% ethyl acetate in hexane): R*_{f}* (product) = 0.45 (purple upon heating with NaOH), R*_{f}* (starting material) = 0.62. The reaction mixture was quenched with water (20 mL), adjusted to pH ∼ 4 with acetic acid, extracted with ethyl acetate (3×20 mL), the combined organic layers were washed with brine and dried over Na₂SO₄. The product was isolated by flash column chromatography (Interchim Puriflash HP 15µm 25 g; gradient 0% to 20% ethyl acetate - hexane); fractions containing the product were repurified by flash column chromatography (Grace Reveleris HP 12 g; gradient 30% to 100% CH₂Cl₂ - pentane over 20 CV), and the product was freeze-dried from 1,4-dioxane to give **38** as white solid. Yield 186 mg (39%). ¹H NMR (400 MHz, acetone-*d*₆): δ 8.17 (dd, *J* = 8.0, 1.3 Hz, 1H), 8.04 (d, *J* = 8.0 Hz, 1H), 7.81 (dd, *J* = 1.3, 0.7 Hz, 1H), 7.35 (d, *J* = 2.7 Hz, 2H), 7.05 (d, *J* = 8.7 Hz, 2H), 6.93 (dd, *J* = 8.8, 2.7 Hz, 2H), 1.54 (s, 9H), 1.36 - 1.24 (m, 6H), 1.11 (d, *J* = 7.4 Hz, 36H), 0.74 (s, 3H), 0.61 (s, 3H). ¹³C NMR (101 MHz, acetone-*d*₆): δ 170.0, 164.5, 156.53, 156.52, 138.4, 137.7, 137.0, 130.9, 130.0, 129.0, 128.7, 126.7, 125.7, 125.0, 122.6, 90.3, 82.9, 28.2, 18.3, 13.4, -0.3, -0.4. ESI-MS, positive mode: *m*/*z* (rel. int., %) = 787.5 (100) [M+H]⁺. HR-MS (ESI, positive mode): 787.4224 [M+H]⁺ (found), 787.4240 (calculated for C₄₅H₆₇O₆Si₃, [M+H]⁺).

**Compound 39.** Tetrabutylammonium fluoride trihydrate (290 mg, 0.92 mmol, 4 eq), dissolved in THF (3.5 mL), was added a solution of **38** (180 mg, 0.23 mmol) in THF (3 mL), cooled in ice-water bath. An intense blue solution with red fluorescence formed. The solution was stirred at 0 °C for 30 min. TLC control (SiO₂ / 10% ethyl acetate in CH₂Cl₂): R*_{f}* (product) = 0.47 (orange; purple with NaOH). Sat. aq. NH₄Cl (20 mL) was added, the mixture was extracted with ethyl acetate (3×20 mL), the combined organic layers were washed with brine and dried over Na₂SO₄. The filtrate was evaporated to light orange oil, which was used directly in the next step.

Trifluoromethanesulfonic anhydride (Tf₂O; 155 µL, 0.92 mmol, 4 eq) was added dropwise to a solution of the crude material from the deprotection step and pyridine (150 µL, 1.84 mmol, 8 eq) in dry CH₂Cl₂ (5 mL), cooled in ice-water bath. Pink color appeared upon addition of each drop and vanished immediately. The flask was then removed from the cooling bath, and the yellow mixture was stirred at RT for 1 h. TLC control (SiO₂ / 10% ethyl acetate in hexane): R*_{f}* (product) = 0.22 (purple with NaOH). The mixture was diluted with cold water (30 mL), extracted with CH₂Cl₂ (3×20 mL), the combined extracts were washed with brine and dried over Na₂SO₄. The product was isolated by flash column chromatography (Sepacore Silica HP 12 g; gradient 0% to 20% ethyl acetate - pentane) and pure fraction were evaporated to give **39** as white foam, yield 153 mg (90%). ¹H NMR (400 MHz, CDCl₃): δ 8.21 (dd, *J* = 8.0, 1.3 Hz, 1H), 8.04 (dd, *J* = 8.0, 0.8 Hz, 1H), 7.91 (t, *J* = 1.0 Hz, 1H), 7.57 (d, *J* = 2.6 Hz, 2H), 7.28 (d, *J* = 8.9 Hz, 2H), 7.21 (dd, *J* = 8.9, 2.7 Hz, 2H), 1.58 (s, 9H), 0.81 (s, 3H), 0.71 (s, 3H). ¹⁹F NMR (376 MHz, CDCl₃): δ -72.77. ESI-MS, positive mode: *m*/*z* (rel. int., %) = 739.3 (100) [M+H]⁺.

**Compound 40.** A mixture of **39** (150 mg, 0.2 mmol), 3-(tert-butyldimethylsilyloxy)azetidine **30** (112 mg, 0.6 mmol, 3 eq), Pd₂(dba)₃ (18 mg, 0.02 mmol, 10 mol%), XPhos (29 mg, 0.06 mmol, 30 mol%) and K₂CO₃ (83 mg, 0.6 mmol, 3 eq) in dry dioxane (2 mL) was degassed on a Schlenk line and stirred at 100 °C under argon (bath temperature) in a sealed flask for 3 h. TLC control (SiO₂ / pentane - CH₂Cl₂ - ethyl acetate 70:25:5, stained by heating): R*_{f}* (product) = 0.50 (blue), R*_{f}* (starting material) = 0.61 (colorless). Upon cooling, the reaction mixture was diluted with water (20 mL), extracted with CH₂Cl₂ (3x20 mL), the combined organic layers were washed with brine and dried over Na₂SO₄. The filtrate was evaporated on Celite, and the product was isolated by flash column chromatography (Interchim Puriflash 15 µm 25 g; gradient 0% to 100% A:B, A = hexane - CH₂Cl₂ - ethyl acetate 70:25:10, B = hexane - CH₂Cl₂ 70:25) and freeze-dried from 1,4-dioxane to yield 145 mg (89%) of **40** as light yellow fluffy solid.

¹H NMR (400 MHz, CDCl₃): δ 8.11 (dd, *J* = 8.0, 1. 3 Hz, 1H), 7. 96 (dd, *J* = 8.0, 0.7 Hz, 1H), 7.81 (s, 1H), 6.85 (d, *J* = 8.7 Hz, 2H), 6.69 (d, *J* = 2.6 Hz, 2H), 6.33 (dd, *J* = 8.8, 2.6 Hz, 2H), 4.78 - 4.69 (m, 2H), 4.19 - 4.12 (m, 4H), 3.69 - 3.62 (m, 4H), 1.55 (s, 9H), 0.89 (s, 18H), 0.66 (s, 3H), 0.58 (s, 3H), 0.07 (s, 12H). ¹³C NMR (101 MHz, CDCl₃): δ 170.3, 164.4, 155.4, 150.7, 137.3, 136.1, 132.7, 129.9, 129.0, 127.7, 125.7, 125.1, 116.3, 113.3, 82.3, 62.5, 62.1, 28.2, 25.9, 18.1, 0.2, -0.6, - 4.8. ESI-MS, positive mode: *m*/*z* (rel. int., %) = 813.4 (100) [M+H]⁺. HR-MS (ESI, positive mode): 813.4120 [M+H]⁺ (found), 813.4145 (calculated for C₄₅H₆₅N₂O₆Si₃, [M+H]⁺).

**Compound 41.** Tetrabutylammonium fluoride trihydrate (142 mg, 0.45 mmol, 3 eq), dissolved in THF (10 mL), was added a solution of **40** (122 mg, 0.15 mmol) in THF (15 mL), cooled in ice-water bath. The reaction mixture was stirred at 0 °C for 1 h. Water (20 mL), acetic acid (1.5 mL) and brine (20 mL) were then added, and the mixture was extracted with ethyl acetate (3×20 mL), the combined organic layers were dried over Na₂SO₄, filtered, evaporated and re-evaporated twice with ethyl acetate (2×20 mL). The product was isolated by flash column chromatography (13 g silica; gradient 10% to 80% ethyl acetate - CH₂Cl₂) and freeze-dried from 1,4-dioxane to give **41** as light green fluffy solid. Yield 84 mg (96%). ¹H NMR (400 MHz, CD₃OD): δ 8.12 (dd, *J* = 8.0, 1.3 Hz, 1H), 7.97 (dd, *J* = 8.1, 0.7 Hz, 1H), 7.69 (t, *J* = 1.0 Hz, 1H), 6.77 (d, *J* = 2.8 Hz, 2H), 6.76 (d, *J* = 8.9 Hz, 2H), 6.35 (dd, *J* = 8.9, 2.8, 2H), 4.63 (tt, J = 6.5, 4.8 Hz, 2H), 4.17 - 4.09 (m, 4H), 3. 64 - 3.57 (m, 4H), 1.51 (d, *J* = 0.8 Hz, 9H), 0.64 (s, 3H), 0.54 (s, 3H). ¹³C NMR (101 MHz, CD₃OD): δ 172.0, 165.4, 157.4, 152.4, 138.7, 136.9, 133.4, 130.9, 129.7, 128.5, 126.7, 125.6, 117.3, 114.6, 93.2, 83.5, 68.1, 63.0, 62.5, 28.3, -0.1, -0.5. ESI-MS, positive mode: *m*/*z* (rel. int., %) = 585.3 (100) [M+H]⁺. HR-MS (ESI, positive mode): 585.2429 [M+H]⁺ (found), 585.2415 (calculated for C₃₃H₃₇N₂O₆Si, [M+H]⁺).

**Compound 42.** Trifluoroacetic acid (2.8 mL) was added to a solution of **41** (84 mg, 144 µmol) in CH₂Cl₂ (14 mL), cooled in ice-water bath. The cooling bath was removed, and the reaction mixture was stirred at RT for 6 h. The solvents were evaporated, and the residue was re-evaporated twice with toluene (2×20 mL) and freeze-dried from 1,4-dioxane, giving 101 mg of impure material. The product was isolated by chromatography on a reversed phase (RP-C₁₈ 10 g; gradient 67% to 33% water - acetonitrile). Fractions containing the product were evaporated and freeze-dried from 1,4-dioxane to give 70 mg (92%) of **42** as blue fluffy solid (free base). HPLC (10/90-100/0 over 25 min, column 4×250 mm, 1.2 mL/min, detection at 254 nm) : τ = 9.98 min. UV-Vis (PBS 7.4): λₘₐₓ (*ε*) = 641 nm (51000 M⁻¹cm⁻¹); fluorescence (PBS 7.4): λ_{excit} = 610 nm, λₑₘ = 662 nm; *Φ*_{fl} (relative to Oxazine 1, Φ_{fl} = 0.14 in ethanol) = 0.42. ¹H NMR (400 MHz, CD₃OD): δ 8.22 (dd, *J* = 7.9, 1.3 Hz, 1H), 8.00 (d, *J* = 8.0 Hz, 1H), 7.83 (d, *J* = 1.0 Hz, 1H), 6.78 (d, *J* = 2.6 Hz, 2H), 6.74 (d, *J* = 8.7 Hz, 2H), 6.39 (dd, *J* = 8.7, 2.7 Hz, 2H), 4.66 (tt, *J* = 6.4, 4.8 Hz, 2H), 4.16 (ddd, *J* = 7.4, 6.4, 1.0 Hz, 4H), 3.66 - 3.60 (m, 4H), 0.64 (s, 3H), 0.54 (s, 3H). ¹³C NMR (101 MHz, CD₃OD): δ 171.7, 156.3, 152.5, 137.8, 133.5, 131.3, 130.5, 128.9, 126.8, 126.6, 117.5, 114.3, 63.0, 62.5, 0.1, -1.2. ESI-MS, positive mode: *m*/*z* (rel. int., %) = 529.2 (100) [M+H]⁺. HR-MS (ESI, positive mode): 529.1787 (found), 529.1789 (calculated for C₂₉H₂₉N₂O₆Si, [M+H]⁺).

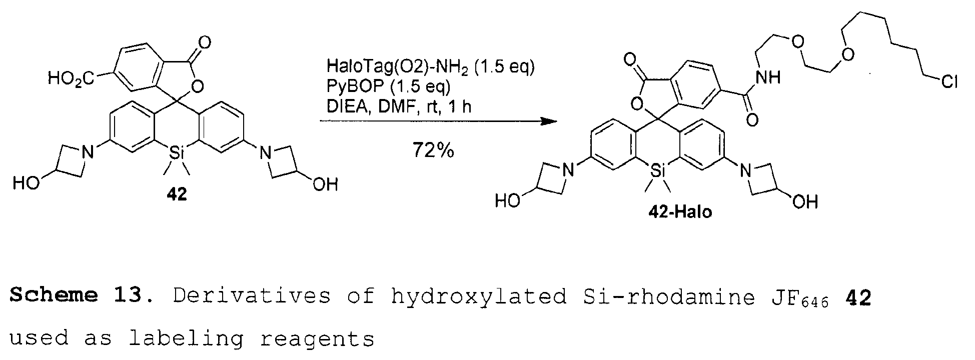

**Compound 42-Halo.** PyBOP (20 µL of 22 mg/100 µL stock solution, 8.52 µmol, 1.5 equiv) was added to a solution of **42** (3 mg, 5.68 µmol), HaloTag Amine (02) (2-(2-((6-chlorohexyl)-oxy)ethoxy)ethanamine; 1.9 mg, 8.52 µmol, 1.5 equiv) and DIEA (*N*-ethyldiisopropylamine; 10 µL) in DMF (100 µL). After 1 h, the solvent was evaporated *in vacuo* at RT, and the product **42-Halo** was isolated by preparative TLC (silica, 10% methanol - CH₂Cl₂, then 100% CH₂Cl₂). Yield 3.0 mg (72%), purity (HPLC) ∼96%. HPLC (10/90-100/0 over 25 min, column 4×250 mm, 1.2 mL/min, detection at 254 nm): τ = 14.98 min. ESI-MS, positive mode: *m*/*z* (rel. int., %) = 734.3 [M+H]⁺. HR-MS (ESI, positive mode): 734.3011 (found), 734.3023 (calculated for C₃₉H₄₉ClSiN₃O₇, [M+H]⁺).

### EXAMPLE III

### STED optical microscopy of cells using

### exemplary novel dyes of the invention

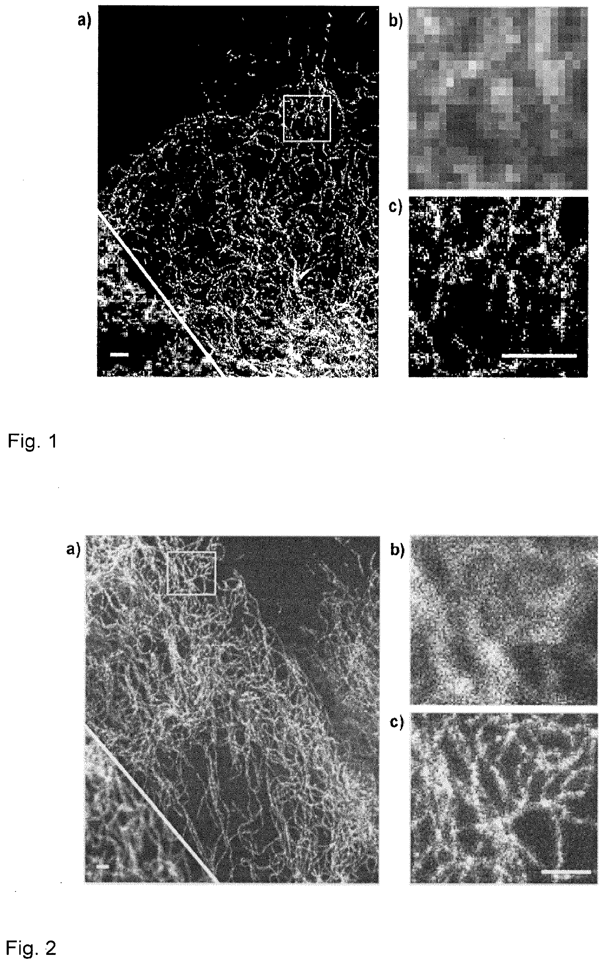

Dye **5**: In order to provide a suitable STED wavelength (STED = stimulated emission depletion, method of the super-resolution optical microscopy providing the optical resolution beyond the diffraction limit), the 660 nm STED laser of the commercial Leica STED microscope, such as Leica TCS SP8 STED 3X, can be advantageously used. Indeed, the difference between the emission maximum and the STED wavelength is 100 - 170 nm (for the dyes with small Stokes shifts). Figure 1 demonstrates the applicability in living cells and very good imaging performance of dye **5**-OH in confocal and STED microscopy (applied as conjugate with Halo-Tag® amine, - compound **5**-Halo in Scheme 2). Q-Rhodamine dye has absorption and emission maxima at 540 and 561 nm, respectively, while hydroxylated dye 5-OH - at 532 and 553 nm, respectively. The fluorescence quantum yield of dye **5**-OH (0.89) is higher than the fluorescence quantum yield of Q-Rhodamine (0.79). Dye **5**-OH is much better soluble in alcohols, DMSO, DMF and aqueous solutions than Q-rhodamine. Unlike Q-Rhodamine, it can be easily transformed into various derivatives, and forms a stable NHS ester (**5**-NHS).

Figure 1 shows a) a STED image of a transfected HeLa cell expressing vimentin-HaloTag fusion protein live-labeled with **5**-Halo (1 µM, 20 minutes at 37°C, washed, and imaged at room temperature), counterpart confocal image in the lower left corner; b) and c) Close-ups of the boxed region from a) in confocal (b) and STED (c) mode. **5**-Halo was excited with a pulsed 532 nm laser and fluorescence detection was at 560-580 nm. The STED laser was pulsed at 631 nm. For the STED image the fluorescence counts of 5 scans per line were accumulated with a pixel dwell time of 17 µs. Scale bars: 1 µm.

Dye **13a**: ROX dyes belong to the so-called "big four" group of dyes (FAM, JOE [6-JOE has absorption and emission maxima at 520 nm and 548 nm, respectively, ε = 75 000 M⁻¹ cm⁻¹], TAMRA and ROX) dominating in the DNA sequencing, and may be efficiently excited with an argon ion laser emitting at 488 nm (and 514 nm). As demonstrated here, the fluorescence of 6'-carboxy ROX dyes (**13a-c**) may be efficiently switched off with 775 nm STED laser. Figure 2 shows the applicability in living cells and very good imaging performance (confocal and STED) of dye **13a** (applied as conjugate with HaloTag® amine - compound **14a**-Halo in Scheme **10**)

Figure 2 shows a) a STED image of a U2OS cell, stable expression of vimentin-HaloTag fusion protein, live-labeled with **14a**-Halo (1 µM, 20 minutes at 37°C, washed, and imaged at room temperature), counterpart confocal image in the lower left corner; b) and c) Close-ups of the boxed region from a) in confocal (b) and STED (c) mode. **14a**-Halo was excited with a pulsed 561 nm laser and fluorescence was detected in both detection windows: 605-625 nm and 650-720 nm. The STED laser was pulsed at 775 nm. For the STED image the fluorescence counts of 3 scans per line were accumulated with a pixel dwell time of 15 µs. Scale bars: 1 µm.

Comparison of the mono-hydroxylated dye **13a** and the commercially available 6-ROX dye **(13c;** Fig. 1) applied as conjugates with Halo-Tag amine - compounds **14a**-Halo (Fig. 2) and **14c**-Halo (Fig. 3) - allows to conclude that mono-hydroxylated dye **13a** affords brighter images.

Figure 3 shows a) a STED image of a U2OS cell, stable expression of vimentin-HaloTag fusion protein, live-labeled with **6**-ROX-Halo (**14c**-Halo in Scheme 10) 1 µM, 20 minutes at 37°C, washed, and imaged at room temperature), counterpart confocal image in the lower left corner; b) and c) Close-ups of the boxed region from a) in confocal (b) and STED (c) mode. 6-ROX-Halo was excited with a pulsed 561 nm laser and fluorescence was detected in two detection windows: 605-625 nm and 650-720 nm. The STED laser was pulsed at 775 nm. For the STED image the fluorescence counts of 3 scans per line were accumulated with a pixel dwell time of 15 µs. The STED image was smoothed with a low pass gaussian filter (Imspector software, *Abberior Instruments*). Scale bars: 1 µm.

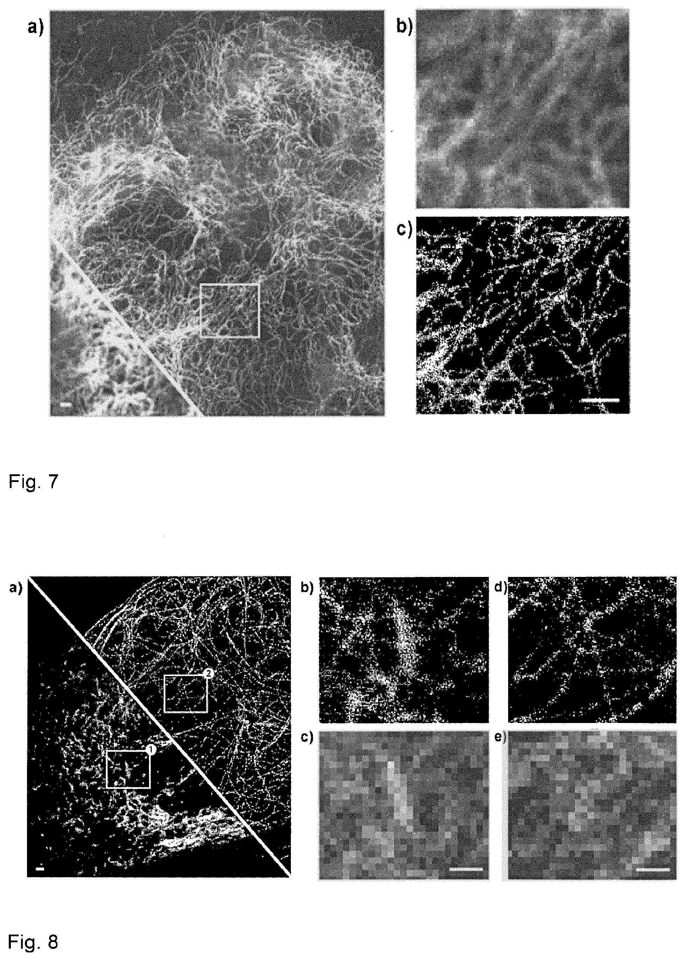

In the case of germanium-containing dyes - **19** and **33 -** hydroxylation was combined with the incorporation of the nitrogen atoms into azetidine rings. The spectral properties of dye 19 (with dimethylamino auxofluoric groups) and dye 33 (with 3-hydroxyazetidine groups) are very similar. However, the fluorescence quantum yield of dye **33** in aqueous buffers (0.60) is higher than the fluorescence quantum yield of dye **19** (0.43). This finding illustrates the positive influence of hydroxyl groups on the fluorescence quantum yield of *GeR* dyes. The conjugates of both dyes (with docetaxel and HaloTag amine) enable a clear visualization of tubulin filaments and HaloTag-fusion proteins in living cells (see Fig. 4-7). Comparison of figures 6 and 8 illustrates the better imaging performance (higher brightness) of the hydroxylated germanium-containing dye **33.**

Figure 4 shows a) a STED image of a transfected HeLa cell live-labeled with **19**-Tubulin (Scheme 11, 1 µM, 20 minutes at 37°C, washed, and imaged in HDMEM at room temperature), counterpart confocal image in the lower left corner; b) and c) Close-ups of the boxed region from a) in confocal (b) and STED (c) mode. 19-Tubulin was excited with a pulsed 640 nm laser and fluorescence was detected from 650-720 nm. The STED laser was pulsed at 775 nm. For the STED image the fluorescence counts of 4 scans per line were accumulated with a pixel dwell time of 7 µs. The STED image was smoothed with a low pass gaussian filter. Scale bars: 1 µm.

Figure 5 shows a) a STED image of a U2OS cell, stable expression of vimentin-HaloTag fusion protein, live-labeled with **19**-Halo (Scheme 11; 1 µM, 20 minutes at 37°C, washed, and imaged at room temperature), counterpart confocal image in the lower left corner; b) and c) Close-ups of the boxed region from a) in confocal (b) and STED (c) mode. 19-Halo was excited with a pulsed 640 nm laser and fluorescence was detected from 650-720 nm. The STED laser was pulsed at 775 nm. The STED image was recorded with a pixel dwell time of 15 µs and smoothed with a low pass gaussian filter. Scale bars: 1 µm.

Figure 6 shows a) a STED image of a transfected HeLa cell live-labeled with **33**-Tubulin (5 µM, 20 minutes at 37°C, washed, and imaged at room temperature), counterpart confocal image in the lower left corner; b) and c) Close-ups of the boxed region from a) in confocal (b) and STED (c) mode. 33-Tubulin was excited with a pulsed 640 nm laser and fluorescence was detected from 650-720 nm. The STED laser was pulsed at 775 nm. The STED image was recorded with a pixel dwell time of 15 µs. Scale bars: 1 µm.

Figure 7 shows a) a STED image of a U2OS cell, stable expression of vimentin-HaloTag fusion protein, live-labeled with **33**-Halo (Scheme 12; 1 µM, 20 minutes at 37°C, washed, and imaged at room temperature), counterpart confocal image in the lower left corner; b) and c) Close-ups of the boxed region from a) in confocal (b) and STED (c) mode. 33-Halo was excited with a pulsed 640 nm laser and fluorescence was detected from 650-720 nm. For the STED image the fluorescence counts of 3 scans per line were accumulated with a pixel dwell time of 5 µs. The STED laser was pulsed at 775 nm. Scale bars: 1 µm.

Combination of hydroxylated dyes **13a** and **33** enabled to realize two-color STED microscopy in living cells (Figure 8). The compounds **14a**-Halo (prepared from **13a**) and **33**-tubulin (prepared from **33**) were applied simultaneously and the fluorescence of both of them was switched-off with the same STED laser (775 nm). The optical resolution of vimentin filaments (labeled with rhodamine dye **13a**) is worse than the optical resolution of tubulin network (stained with hydroxylated *GeR* **33**), because the STED laser is less efficient for the blue-shifted rhodamine **13a** (due to its smaller absorption cross-section at 775 nm). However, in single color imaging the dye **13a** can provide as good optical resolution as dye **33.** Remarkably, dye **13a** represents the most blue-shifted dye which can be still efficiently depleted with 775 nm STED laser.

Figure 8 shows a) a STED image of a transfected HeLa cell expressing vimentin-HaloTag fusion protein live-labeled simultaneously with **14a**-Halo (vimentin staining, lower left image, 1 µM) and **33**-Tubulin (upper right image, 5 µM, 20 minutes at 37°C, washed, and imaged at room temperature); b) and c) Close-ups of the boxed region from a1) in STED (b) and confocal (c) mode for vimentin-HaloTag fusion protein labeled with **14a**-Halo; d) and e) Close-ups of the boxed region from a2) in STED (d) and confocal (e) mode for **33**-Tubulin. **14a**-Halo was excited with a pulsed 561 nm laser and fluorescence was detected from 605-625 nm. 33-Tubulin was excited with a pulsed 640 nm laser and fluorescence was detected from 650-720 nm. For the STED image the fluorescence of both color channels were recorded linewise with a pixel dwell time of 15 µs. The STED laser was pulsed at 775 nm. Scale bars: 1 µm.

## Claims

1. A fluorescent dye selected from the group consisting of compounds of the general formulae **I-III** or **4** below 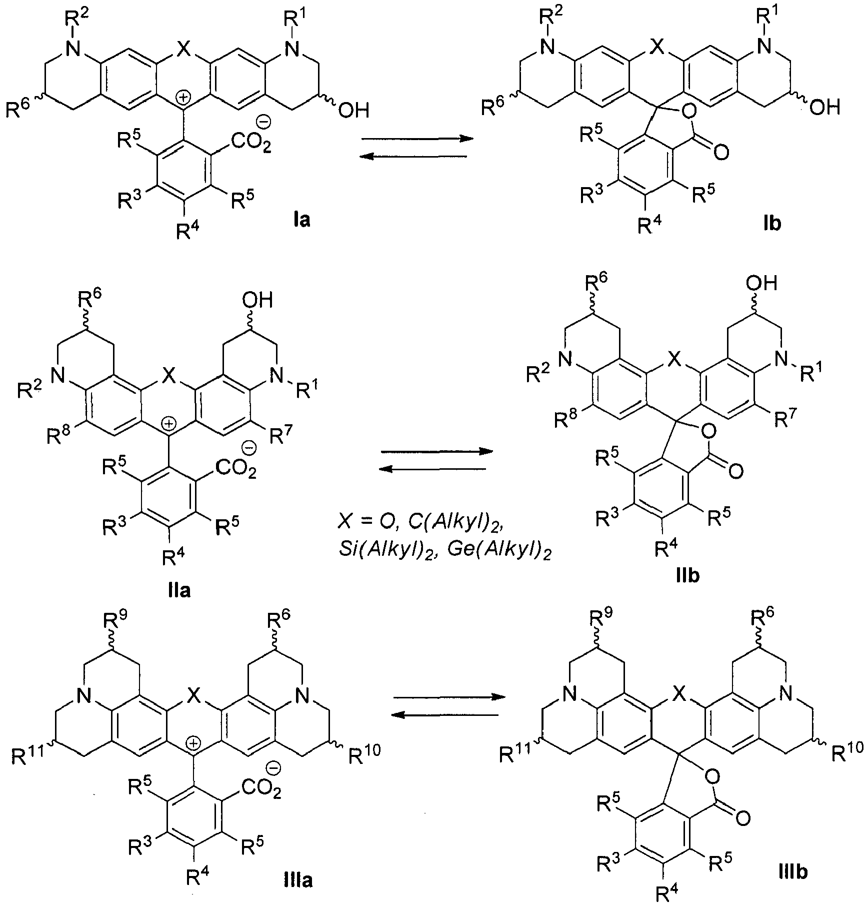 which exist in equilibrium between the open zwitterionic form (**a**) and the closed form (**b**),
wherein in formulae **I-III**
*X* = O C*(Alkyl)*₂*,* Si*(Alkyl)*₂ or Ge*(Alkyl)*₂ with *Alkyl* being a C₁-C₄ alkyl group, such as a methyl, ethyl, propyl, isopropyl, *n*-butyl, isobutyl or sec-butyl group;
R¹ and R² in structures **I** and **II** are independently CF₃CH₂, a C₁-C₆ alkyl group, or a substituted C₁-C₆ alkyl group;
R³ and R⁴ in structures **I-III** are independently
hydrogen; a C₁-C₆ alkyl group or substituted C₁-C₆ alkyl group; a carboxyl group or a derivative of the carboxyl group, e.g. a lower alkyl, aryl or hetaryl ester, in which the alkyl, aryl or hetaryl part is optionally substituted with any functional group; a primary or secondary alkyl, aryl or hetaryl carboxamide (CONHR', CONR'R''), in which the alkyl, aryl or hetaryl part(s) (R', R'') is (are) optionally substituted with any functional group(s); a *N*-hydroxysuccinimidyl ester or another reactive ester of formula CO₂X where X is a good leaving group, such as F, N₃, SR', OR' with R' = aryl or hetaryl; CONHNH₂ or CONR' NR''R''', with R' , R'', R''' being independently H, lower alkyl, preferably C₁-C₆ alkyl, aryl or hetaryl, in which the alkyl, aryl or hetaryl part is optionally substituted with any functional group; CONHOH or ONR'OR'', with R', R'' being independently H, lower alkyl, aryl or hetaryl, in which the alkyl, aryl or hetaryl part is optionally substituted with any functional group; COCHN₂ or COCR'N₂ with R' = lower alkyl, aryl or hetaryl, in which the alkyl, aryl or hetaryl part is optionally substituted with any functional group; an amino group, lower alkylamino group or dialkylamino group, the alkyl part(s) of which may be optionally substituted with any functional group, in particular a carboxyl group or a derivative of the carboxyl group, e.g. a lower alkyl, aryl or hetaryl ester, in which the alkyl, aryl or hetaryl part is optionally substituted with any functional group; azido group; *N*-maleimidoalkyl group, iodoacetamide or any other reactive group suitable for conjugation to biomolecules; an arylthio or alkylthio group, the aryl or alkyl part of which may be optionally substituted with any uncharged group, including an azide group -N₃, carboxyl group, a derivative of the carboxyl group, e.g. a lower alkyl, aryl or hetaryl ester, in which the alkyl, aryl or hetaryl part is optionally substituted with any functional group; an alkyl, aryl or hetaryl amide (NHCOR', NHCOR'R''), in which the alkyl, aryl or hetaryl part(s) (R', R'') is (are) optionally substituted with any functional group;
optionally, the carbon chains in R¹, R², R³ and R⁴ may contain double or triple bonds and/or cycles, and/or uncharged groups, e.g. hydroxyl, as well as one, two, three or four heteroatoms;
R⁵ = H, F or Cl;
R⁶, R⁹, R¹⁰, R¹¹ = H or OH and the wavy bonds (∼∼∼) in structures **I-III** above denote all possible isomers if one or more substituents from the set R⁶, R⁹, R¹⁰, R¹¹ is/are OH;
R⁷, R⁸ are independently H, a C₁-C₄ alkyl group, F, Cl, Br or I; provided (1) that all dyes represented by the formulae **I, II** and **III** contain at least one substituted 3-hydroxy-1,2,3,4-tetrahydroquinoline fragment (with an element -CH₂CH(OH)CH₂-) incorporated into the structure of the fluorophore, as shown in formulae **I, II** and **III** and in the structural formula below, 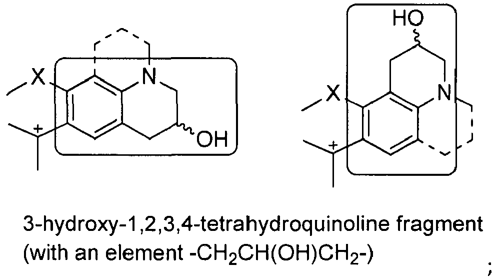
with an additional provision (2) for structure **III,** that at least one of R⁶, R⁹, R¹⁰ or R¹¹ is a hydroxyl group; 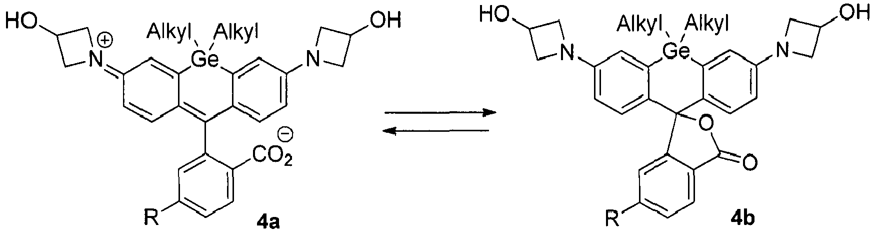 Wherein
*Alkyl* in structure **4** is a C₁-C₆ alkyl group, such as methyl, ethyl, propyl, isopropyl, n-butyl, isobutyl, sec-butyl, pentyl, isopentyl, 2-methylbutyl, 2,2-dimethylpropyl, hexyl, isohexyl, 2-methylpentyl, 3-methylpentyl, 2,2-dimethylbutyl;
R in structure **4** may be hydrogen, a C₁-C₆ alkyl group or substituted C₁-C₆ alkyl group, a carboxyl group or a derivative of the carboxyl group, e.g. a lower alkyl, aryl or hetaryl ester, in which the alkyl, aryl or hetaryl part is optionally substituted with any functional group; a primary or secondary alkyl, aryl or hetaryl carboxamide (CONHR', CONR'R''), in which the alkyl, aryl or hetaryl part(s) (R', R'') is (are) optionally substituted with any functional group(s); a *N-*hydroxysuccinimidyl ester or another reactive ester of formula CO₂X where X is a good leaving group, such as F, N₃, SR', OR' with R' = aryl or hetaryl; CONHNH₂ or CONR'NR'' R''' with R', R'', R''' being independently H, C₁-C₆ alkyl, aryl or hetaryl, in which the alkyl, aryl or hetaryl part(s) is (are) optionally substituted with any functional group(s)); CONHOH or CONR'OR'' with R', R'' being independently H, lower alkyl, aryl or hetaryl, in which the alkyl, aryl or hetaryl part(s) is (are) optionally substituted with any functional group(s)); COCHN₂ or COCR'N₂ with R' = lower alkyl, aryl or hetaryl, in which the alkyl, aryl or hetaryl part is optionally substituted with any functional group; an amino group, lower alkylamino group or dialkylamino group, the alkyl part(s) of which may be optionally substituted with any functional group, in particular with a carboxyl group or a derivative of the carboxyl group, e.g., lower alkyl, aryl or hetaryl ester, in which the alkyl, aryl or hetaryl part is optionally substituted with any functional group; a primary or secondary alkyl, aryl or hetaryl amide (NHCOR', NHCOR'R''), in which the alkyl, aryl or hetaryl part(s) (R', R'') is (are) optionally substituted with any functional group(s); azido group; *N-*maleimidoalkyl group, iodoacetamide or any other reactive group suitable for conjugation to biomolecules;
optionally, the carbon chains in the group R may contain double or triple bonds and/or cycles, and/or uncharged groups as well as one, two, three or four heteroatoms.

2. A compound of structure **III** according to claim 1 which exhibits two hydroxyl groups and wherein either R⁶ = R⁹ = OH and R¹⁰ = R¹¹ = H, or R⁶ = R⁹ = H and R¹⁰ = R¹¹ = OH.

3. A compound of structure **III** according to claim 1 which exhibits one hydroxyl group and wherein either R⁶ = OH and R⁹ = R¹⁰ = R¹¹ = H, or R¹⁰ = OH and R⁶ = R⁹ = R¹¹ = H.

4. A fluorescent dye according to claim 1 representing one of the compounds **5**-R³, wherein R¹ = R² = CH₂CF₃, R⁶ = OH and R³ = CO₂H its reactive ester, preferably a N-succinimidyloxycarbonyl, 2,3,5,6-tetrafluorophenoxycarbonyl or pentafluorophenoxycarbonyl group, or amide with a linker bearing one terminal amino or carboxylic acid group as indicated in the structural formulae below

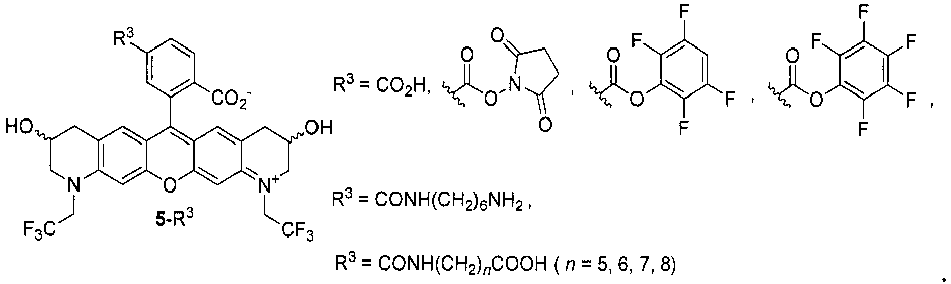

5. A fluorescent dye according to claim 1 representing one of the compounds **14a,b-**R³**,** wherein either R¹ = R² = OH, or R¹ = OH and R² = H, and wherein R³ = CO₂H, its reactive ester, preferably a *N*-succinimidyloxycarbonyl, 2,3,5,6-tetrafluorophenoxycarbonyl or pentafluorophenoxycarbonyl group, or amide with a linker bearing one terminal amino or carboxylic acid group.

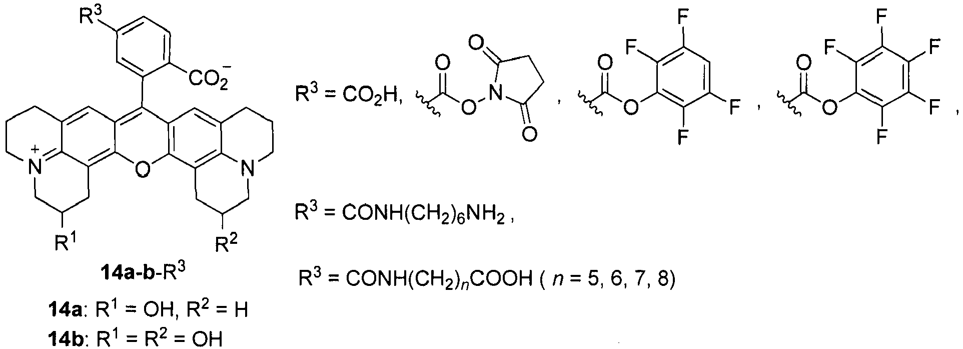

6. A fluorescent dye of structure **4** according to claim 1 where *Alkyl* is a straight or branched C₁-C₆ alkyl chain, preferably C₁-C₄ alkyl, most preferably methyl; R is CO₂H or a reactive ester, preferably a N-succinimidyloxycarbonyl, 2,3,5,6-tetrafluorophenoxycarbonyl or pentafluorophenoxycarbonyl group, or a functional group capable of participating in a ligation reaction such as, for example, an azide, alkyne, strained alkene or tetrazine reactive group, which functional group is connected with the phenyl group through a linker, or R is any ligand forming a covalent bond with a biological target of interest, or a noncovalent ligand binding to a biological target of interest, such as a docetaxel or jasplakinolide derivative.

7. A fluorescent dye according to claim 6 representing the compounds **4**-R, wherein R = CO₂H or its reactive ester, preferably a N-succinimidyloxycarbonyl, 2,3,5,6-tetrafluorophenoxycarbonyl or pentafluorophenoxycarbonyl group, as indicated in the structural formulae below

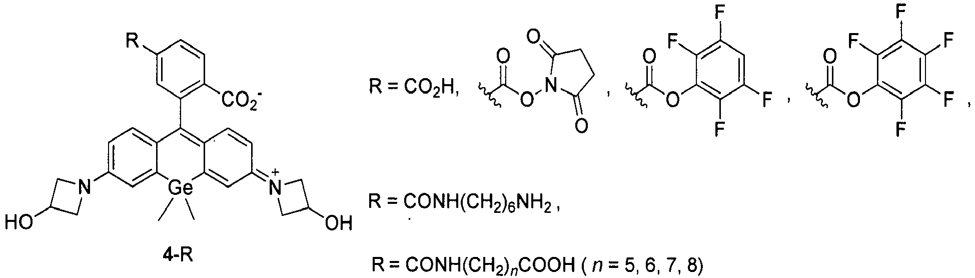

8. A fluorescent dye according to claim 6 representing one of the compounds **33**, **33**-NHS, **33**-Halo, **33**-tubulin and **34** as indicated in the structural formulae below

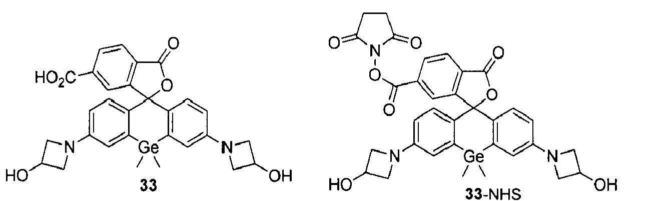

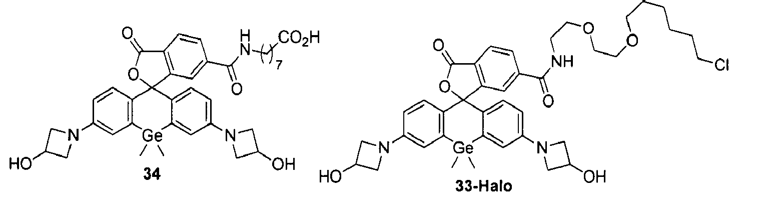

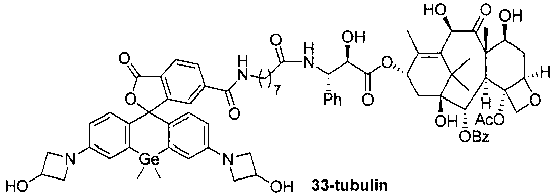

9. A method for preparing germa- and silaxanthones comprising the following steps:
a) a regioselective bromination of di-*O*-TIPS-protected bis (3-hydroxyphenyl)silanes or -germanes to the corresponding di-*O*-TIPS-protected bis(2-bromo-5-hydroxyphenyl)silanes or -germanes using an electrophilic brominating reagent, preferably *N*-bromosuccinimide;
b) double metal-halogen exchange on the dibromide resulting from the step a) followed by reaction with a carbamoyl chloride, preferably *N,N*-dimethylcarbamoyl chloride;
c) optionally further deprotection/reprotection steps.

10. The method according to claim 9 for preparing the compounds **24, 37-OH** below or their *O*-protected analogs, such as compounds **25** in Scheme 4 and **37** in Scheme 5:

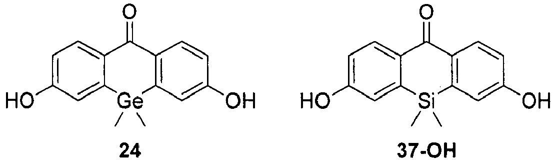

11. Use of the compounds according to claims 1-8 as reagents for conjugation or bioconjugation.

12. The use according to claim 11, wherein the conjugation or bioconjugation comprises formation of at least one covalent chemical bond or at least one molecular complex with a chemical entity or substance, such as amine, thiol, carboxylic acid, aldehyde, alcohol, aromatic compound, heterocycle, e.g. tetrazine, alkyne, alkene including strained and bicyclic alkenes, e. g. *trans*-cyclooctene*,* cyclopropene and norbornene derivatives, organic azide, dye, amino acid, amino acid residue coupled to any chemical entity, peptide, protein, in particular enzymes and immunoglobulins, antibody, single-domain antibody, carbohydrate including a carbohydrate residue attached to a protein, nucleic acid, toxin, lipid, virus, virus-like particle.

13. A conjugate or bioconjugate comprising a dye according to any one of claims 1-8 coupled via at least one covalent chemical bond or at least one molecular complex to a chemical entity or substance, such as amine, thiol, carboxylic acid, aldehyde, alcohol, aromatic compound, heterocycle, e.g. tetrazine, alkyne, alkene including strained and bicyclic alkenes, e. g. trans-cyclooctene, cyclopropene and norbornene derivatives, organic azide, dye, amino acid, amino acid residue coupled to any chemical entity, peptide, protein, in particular enzymes and immunoglobulins, antibody, single-domain antibody, carbohydrate including a carbohydrate residue attached to a protein, nucleic acid, toxin, lipid, virus, virus-like particle.

14. A conjugate or bioconjugate comprising a dye according to any one of claims 1-8 coupled via a covalent bond to Pepstatin A, Jasplakinolide derivatives, Docetaxel derivative, and Aminophalloidin depicted below

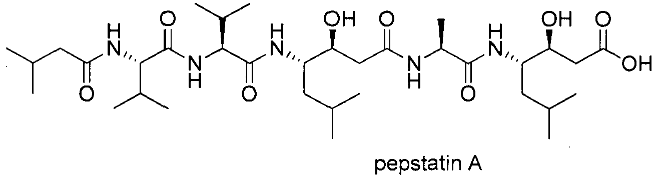

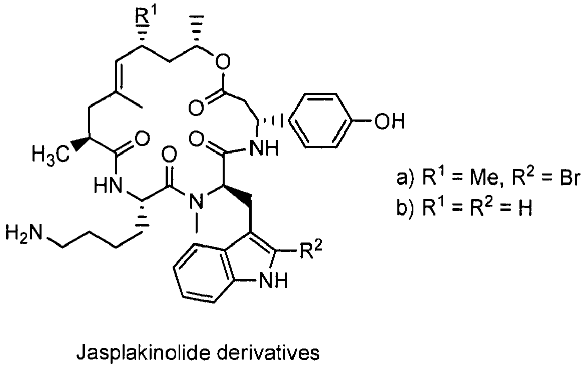

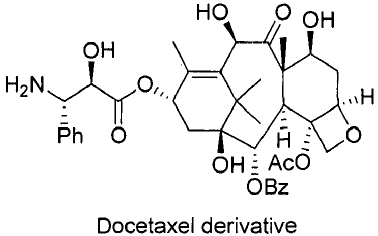

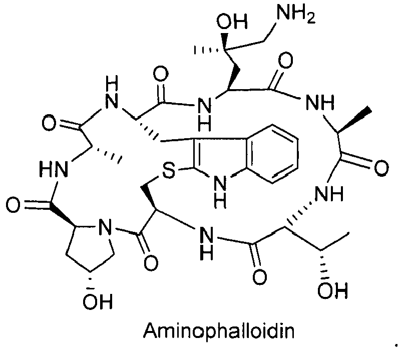

15. Use of the compounds according to any one of claims 1-8 or of their conjugates according to claims 13 and 14 as cell permeant substances penetrating through membranes of living and fixed cells.

16. Use of the compounds according to any one of claims 1-8 or of their conjugates according to claims 13 and 14 for tracking and monitoring dynamic processes in a sample or in an object.

17. Use of the compounds according to any one of claims 1-8 or of their conjugates according to claims 13 and 14 as fluorescent tags, analytical reagents and labels in optical microscopy, imaging techniques, protein tracking, nucleic acid labelling and flow cytometry.

18. The use according to claim 17 wherein the optical microscopy and imaging techniques comprise: confocal microscopy, structured illumination microscopy, stimulated emission depletion microscopy [STED], fluorescence correlation spectroscopy [FCS], combination of STED and FCS (STED-FCS), fluorescence recovery after photobleaching [FRAP], fluorescence lifetime imaging [FLIM], ground state depletion with individual molecular return [GSD or GSDIM], RESOLFT microscopy, fluorescence resonant energy transfer [FRET], single molecule switching techniques, such as single molecule localization microscopy [SMLM], photoactivation localization microscopy [PALM, PALMIRA, fPALM], and stochastic optical reconstruction microscopy [STORM].
